(19) Europäisches Patentamt
European Patent Office
Office européen des brevets

(11) EP 2 128 622 A1

# (12) EUROPEAN PATENT APPLICATION

(43) Date of publication:
**02.12.2009 Bulletin 2009/49**

(51) Int Cl.:
***G01N 33/574** (2006.01)* ***C12N 15/10** (2006.01)*

(21) Application number: **08157191.1**

(22) Date of filing: **29.05.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicants:
- **DKFZ Deutsches Krebsforschungszentrum 69120 Heidelberg (DE)**
- **Ruprecht-Karls-Universität Heidelberg 69117 Heidelberg (DE)**

(72) Inventors:
- **Angel, Peter 68535 Edingen-Neckarhausen (DE)**
- **Busch, Hauke 64625 Bensheim (DE)**
- **Eils, Roland 69198 Schriesheim (DE)**
- **Szabowski, Axel 69221 Dossenheim (DE)**
- **Camacho-Trujillo, David 69221 Heidelberg (DE)**

(74) Representative: **Dick, Alexander**
**Isenbruck Bösl Hörschler Wichmann LLP**
**Eastsite One**
**Seckenheimer Landstrasse 4**
**68163 Mannheim (DE)**

## (54) Means and methods for diagnosing metastasising potentials of tumor cells

(57) The present invention is concerned with diagnostic means and methods for determining the metastasising potential of solid tumors. More specifically, the present invention relates to a method of diagnosing whether a solid tumor in a subject has metastasising potential comprising the steps of determining in a sample of a subject suspected to suffer from a solid tumor the expression levels of the marker genes GPR109B, DHRS9, and ERRFI-1 and of a reference gene, calculating at least one variable from one or more of said marker genes and a reference gene and comparing the variable to a reference variable whereby it is to be diagnosed whether the solid tumor has metastasising potential. Moreover, the present invention relates to a method of determining whether a therapy against a solid tumor having metastasising potential is successful. Further contemplated are a solid support, diagnostic device and a kit for carrying out the aforementioned methods.

EP 2 128 622 A1

SEQUENCE LISTING

<110> DKFZ Deutsches Krebsforschungszentrum, Stiftung des öffentlichen Rechts

<120> Means and methods for diagnosing metastasising potentials of tumor cells

<130> DK65194EP

<160> 16

<170> PatentIn version 3.4

<210> 1
<211> 2152
<212> DNA
<213> Homo sapiens

<400> 1
ttcgtagttt cctgataacc attcagtcat ctatttcaac accctgacat gacataaagg 60

caggcacgga accacacgtt caccatacag acacacgcca ctttgctgga gcattcacta 120

ggcgaggcgc tccatcggac tcactagctg cactcatgaa tcggcaccat ctgcaggatc 180

actttctgga aatagacaag aagaactgct gtgtgttccg agatgacttc attgccaagg 240

tgttgccgcc ggtgttgggg ctggagttta tctttgggct tctgggcaat ggccttgccc 300

tgtggatttt ctgtttccac ctcaagtcct ggaaatccag ccggattttc ctgttcaacc 360

tggcagtagc tgactttcta ctgatcatct gcctgccgtt cgtgatggac tactatgtgc 420

ggcgttcaga ctggaagttt ggggacatcc cttgccggct ggtgctcttc atgtttgcca 480

tgaaccgcca gggcagcatc atattcctca cggtggtggc ggtagacagg tatttccggg 540

tggtccatcc ccaccacgcc ctgaacaaga tctccaattg gacagcagcc atcatctctt 600

gccttctgtg gggcatcact gttggcctaa cagtccacct cctgaagaag aagttgctga 660

tccagaatgg cactgcaaat gtgtgcatca gcttcagcat ctgccatacc ttccggtggc 720

acgaagctat gttcctcctg gagttcttcc tgcccctggg catcatcctg ttctgctcag 780

ccagaattat ctggagcctg cggcagagac aaatggaccg gcatgccaag atcaagagag 840

ccatcacctt catcatggtg gtggccatcg tctttgtcat ctgcttcctt cccagcgtgg 900

ttgtgcggat ccacatcttc tggctcctgc acacttcggg cacgcagaat tgtgaagtgt 960

accgctcggt ggacctggcg ttctttatca ctctcagctt cacctacatg aacagcatgc 1020

tggaccccgt ggtgtactac ttctccagcc catcctttcc caacttcttc tccactttga 1080

tcaaccgctg cctccagagg aagataacag gtgagccaga taataaccgc agcacgagcg 1140

tcgagctcac aggggacccc aacaaaacca gaggcgctcc agaggcgtta atcgccaact 1200

ccggtgagcc atggagcccc tcttatctgg gcccaacctc aaataaccat tccaagaagg 1260

gacattgtca ccaagaacca gcatctctgg agaaacagtt gggctgttgc atcgagtaat 1320

gtcactggac tcggcctaag atttcctgga acttccagat tcagagaatc tgatttaggg 1380

aaactgtggc agatgagtgg gagactggtt gcaaggtgtg accgcaggaa tcctggagga 1440

acagagagta aagcttctag gcatctgaaa cttgcttcat ctctgacgct cgcaggactg 1500

aagatgggca aattgtaggc gtttctgctg agcagagttg gagccagaga tctacttgtg 1560
acttgttggc cttcttccca catctgcctc agactggagg gggctcagct cctggggtga 1620
tatctagcct gcttgtgagc tctagcaggg ataaggagag ctgagattgg agggaattgt 1680
gttgttcctg gaggaagccc aggcatcatt aaacaagcca gtaggtcacc tggcttccgt 1740
ggaccaattc atctttcaga caatctttag cagaaatgga ctcagggaag agactcacat 1800
gctttggtta gtatctgtgt ttccggtggg tgtaataggg gattagcccc agaagggact 1860
gagctaaaca gtgttattat gggaaaggaa atggcattgc tgctttcaac cagcgactaa 1920
tgcaatccat tcctctcttg tttatagtaa tctaagggtt gaacagttaa aacggcttca 1980
ggatagaaag ctgtttccca cctgtttgct tttaccatta aaagggaaac gtgcctctgc 2040
cccacgggta gaggggtgca cgttcctcct ggttccttcg cttgtgtttc tgtacttacc 2100
aaaaatctac catttcaata aattttgata ggagacaaaa aaaaaaaaaa aa 2152

<210> 2
<211> 387
<212> PRT
<213> Homo sapiens

<400> 2

Met Asn Arg His His Leu Gln Asp His Phe Leu Glu Ile Asp Lys Lys
1 5 10 15

Asn Cys Cys Val Phe Arg Asp Asp Phe Ile Ala Lys Val Leu Pro Pro
20 25 30

Val Leu Gly Leu Glu Phe Ile Phe Gly Leu Leu Gly Asn Gly Leu Ala
35 40 45

Leu Trp Ile Phe Cys Phe His Leu Lys Ser Trp Lys Ser Ser Arg Ile
50 55 60

Phe Leu Phe Asn Leu Ala Val Ala Asp Phe Leu Leu Ile Ile Cys Leu
65 70 75 80

Pro Phe Val Met Asp Tyr Tyr Val Arg Arg Ser Asp Trp Lys Phe Gly
85 90 95

Asp Ile Pro Cys Arg Leu Val Leu Phe Met Phe Ala Met Asn Arg Gln
100 105 110

Gly Ser Ile Ile Phe Leu Thr Val Val Ala Val Asp Arg Tyr Phe Arg
115 120 125

Val Val His Pro His His Ala Leu Asn Lys Ile Ser Asn Trp Thr Ala
130 135 140

Ala Ile Ile Ser Cys Leu Leu Trp Gly Ile Thr Val Gly Leu Thr Val
145 150 155 160

His Leu Leu Lys Lys Lys Leu Leu Ile Gln Asn Gly Thr Ala Asn Val
165 170 175

Cys Ile Ser Phe Ser Ile Cys His Thr Phe Arg Trp His Glu Ala Met
180 185 190

Phe Leu Leu Glu Phe Phe Leu Pro Leu Gly Ile Ile Leu Phe Cys Ser
195 200 205

Ala Arg Ile Ile Trp Ser Leu Arg Gln Arg Gln Met Asp Arg His Ala
210 215 220

Lys Ile Lys Arg Ala Ile Thr Phe Ile Met Val Val Ala Ile Val Phe
225 230 235 240

Val Ile Cys Phe Leu Pro Ser Val Val Val Arg Ile His Ile Phe Trp
245 250 255

Leu Leu His Thr Ser Gly Thr Gln Asn Cys Glu Val Tyr Arg Ser Val
260 265 270

Asp Leu Ala Phe Phe Ile Thr Leu Ser Phe Thr Tyr Met Asn Ser Met
275 280 285

Leu Asp Pro Val Val Tyr Tyr Phe Ser Ser Pro Ser Phe Pro Asn Phe
290 295 300

Phe Ser Thr Leu Ile Asn Arg Cys Leu Gln Arg Lys Ile Thr Gly Glu
305 310 315 320

Pro Asp Asn Asn Arg Ser Thr Ser Val Glu Leu Thr Gly Asp Pro Asn
325 330 335

Lys Thr Arg Gly Ala Pro Glu Ala Leu Ile Ala Asn Ser Gly Glu Pro
340 345 350

Trp Ser Pro Ser Tyr Leu Gly Pro Thr Ser Asn Asn His Ser Lys Lys
355 360 365

Gly His Cys His Gln Glu Pro Ala Ser Leu Glu Lys Gln Leu Gly Cys
370 375 380

Cys Ile Glu
385

<210> 3
<211> 2939
<212> DNA
<213> Homo sapiens

<400> 3
atctgaaaaa ttaataattc cttaattatc aaatatccat tatttaaatt tataattgtg 60

```
tcataaatat tgtcataaat agatttgctg ttttaaagct tgttccttca ttttctctgt    120
tttgttttag ataaacattg tcataaatag atttgttgtt ttaaagcttg ttccttcatt    180
ttctctgatt gttttgtttt agattcagag gttacttatg cttgtttgtt acatggatgt    240
tacatgtgta atgggggata ttggacttct agtgtactca tcacccatat actgaacact    300
gtactcaaaa gggattgaaa gaaactagga aacttggcag gaagatcatt cttaagccag    360
gaaaaaaatt tttaatgctc acatgtgaac atgtgatggt cataccagaa ggagcaccca    420
cctccctccc tctgtgacag acacattttc ttagccttca cctttccttc tttcaagttg    480
ctgaaaatcc acagtgtttc tgttcatttg ttactttcat tctcacctat cttctctctt    540
gctccatcta ccagaacaat aattccccat ataatacttc tcacttcact tttcaacgca    600
ggacctcttg ttggtctgat ctgtttgtct gtccgcttta tcaatattat cagatgtaag    660
tttacatgaa tacacacaca tattcactaa actgagggga aaaaatgcct tgtaggtcat    720
aaaaaagcag ggaaattccc aacaattcat atttgatccc tggatccagg ggtggcagca    780
ataagcctgc tttagatatt tactccccat tttatgatcc ggtggtttgg tttttcaaat    840
gatgatatgg ctcctttcgc aatgacttga tgtttaggag gtgtgcttca ataaatacat    900
tttaaaatca acaatcaagt tagagttgta caaatggctc tgaaatgtcc cactacactg    960
ttagaccaag ggcacagatt gtgcttctgt actatttatc ctagtatccc tcggcatata   1020
ttaactgctc taaaaatctc cttggctaca cgctgcatca aatcaaagtt aaatgttata   1080
ccacctttct attctatttt taatattcaa agagggtgct cagattttag aacaaatttc   1140
aatgtttaag tacacacaaa aaaatcatta actcatatat ttcaagagta ggaaatggga   1200
actggtgtta aaactcttat aacaaatgtc actgtcttaa gggacagtgt ttaaaaacgc   1260
atacctggcc gggcgcggtg gctcatgcct gtaatcccag cactttggga ggccgaggcc   1320
ggcggatcac aaggaaaaca aactcaggaa gaaaaaggaa agcagaagtg atcaaggaga   1380
gcgctcgagt tgcaatattt tcctttggct gctgacaggc agttactata aagcattgtg   1440
catggacacc atcttcttgt attatacaag aaaggagtgt acctatcaca cacaggggga   1500
aaaatgctct tttgggtgct aggcctccta atcctctgtg gttttctgtg gactcgtaaa   1560
ggaaaactaa agattgaaga catcactgat aagtacattt ttatcactgg atgtgactcg   1620
ggctttggaa acttggcagc cagaactttt gataaaaagg gatttcatgt aatcgctgcc   1680
tgtctgactg aatcaggatc aacagcttta aaggcagaaa cctcagagag acttcgtact   1740
gtgcttctgg atgtgaccga cccagagaat gtcaagagga ctgcccagtg ggtgaagaac   1800
caagttgggg agaaaggtct ctggggtctg atcaataatg ctggtgttcc cggcgtgctg   1860
gctcccactg actggctgac actagaggac tacagagaac ctattgaagt gaacctgttt   1920
ggactcatca gtgtgacact aaatatgctt cctttggtca agaaagctca agggagagtt   1980
attaatgtct ccagtgttgg aggtcgcctt gcaatcgttg gagggggcta tactccatcc   2040
aaatatgcag tggaaggttt caatgacagc ttaagacggg acatgaaagc ttttggtgtg   2100
```

cacgtctcat gcattgaacc aggattgttc aaaacaaact tggcagatcc agtaaaggta 2160
attgaaaaaa aactcgccat ttgggagcag ctgtctccag acatcaaaca acaatatgga 2220
gaaggttaca ttgaaaaaag tctagacaaa ctgaaaggca ataaatccta tgtgaacatg 2280
gacctctctc cggtggtaga gtgcatggac cacgctctaa caagtctctt ccctaagact 2340
cattatgccg ctggaaaaga tgccaaaatt ttctggatac ctctgtctca catgccagca 2400
gctttgcaag actttttatt gttgaaacag aaagcagagc tggctaatcc caaggcagtg 2460
tgactcagct aaccacaaat gtctcctcca ggctatgaaa ttggccgatt tcaagaacac 2520
atctcctttt caaccccatt ccttatctgc tccaacctgg actcatttag atcgtgctta 2580
tttggattgc aaaagggagt cccaccatcg ctggtggtat cccagggtcc ctgctcaagt 2640
tttctttgaa aaggagggct ggaatggtac atcacatagg caagtcctgc cctgtattta 2700
ggctttgcct gcttggtgtg atgtaaggga aattgaaaga cttgcccatt caaaatgatc 2760
tttaccgtgg cctgccccat gcttatggtc cccagcattt acagtaactt gtgaatgtta 2820
agtatcatct cttatctaaa tattaaaaga taagtcaaac attaaaaaaa aaaaaaaaaa 2880
aaaaaaaaaa aaaaaaaaaa aaaaaaaaaa aaaaaaaaaa aaaaaaaaaa aaaaaaaaa 2939

<210> 4
<211> 319
<212> PRT
<213> Homo sapiens

<400> 4

Met Leu Phe Trp Val Leu Gly Leu Leu Ile Leu Cys Gly Phe Leu Trp
1 5 10 15

Thr Arg Lys Gly Lys Leu Lys Ile Glu Asp Ile Thr Asp Lys Tyr Ile
20 25 30

Phe Ile Thr Gly Cys Asp Ser Gly Phe Gly Asn Leu Ala Ala Arg Thr
35 40 45

Phe Asp Lys Lys Gly Phe His Val Ile Ala Ala Cys Leu Thr Glu Ser
50 55 60

Gly Ser Thr Ala Leu Lys Ala Glu Thr Ser Glu Arg Leu Arg Thr Val
65 70 75 80

Leu Leu Asp Val Thr Asp Pro Glu Asn Val Lys Arg Thr Ala Gln Trp
85 90 95

Val Lys Asn Gln Val Gly Glu Lys Gly Leu Trp Gly Leu Ile Asn Asn
100 105 110

Ala Gly Val Pro Gly Val Leu Ala Pro Thr Asp Trp Leu Thr Leu Glu
115 120 125

Asp Tyr Arg Glu Pro Ile Glu Val Asn Leu Phe Gly Leu Ile Ser Val

```
        130                 135                 140

Thr Leu Asn Met Leu Pro Leu Val Lys Lys Ala Gln Gly Arg Val Ile
145                 150                 155                 160

Asn Val Ser Ser Val Gly Gly Arg Leu Ala Ile Val Gly Gly Gly Tyr
                165                 170                 175

Thr Pro Ser Lys Tyr Ala Val Glu Gly Phe Asn Asp Ser Leu Arg Arg
            180                 185                 190

Asp Met Lys Ala Phe Gly Val His Val Ser Cys Ile Glu Pro Gly Leu
        195                 200                 205

Phe Lys Thr Asn Leu Ala Asp Pro Val Lys Val Ile Glu Lys Lys Leu
    210                 215                 220

Ala Ile Trp Glu Gln Leu Ser Pro Asp Ile Lys Gln Gln Tyr Gly Glu
225                 230                 235                 240

Gly Tyr Ile Glu Lys Ser Leu Asp Lys Leu Lys Gly Asn Lys Ser Tyr
                245                 250                 255

Val Asn Met Asp Leu Ser Pro Val Val Glu Cys Met Asp His Ala Leu
            260                 265                 270

Thr Ser Leu Phe Pro Lys Thr His Tyr Ala Ala Gly Lys Asp Ala Lys
        275                 280                 285

Ile Phe Trp Ile Pro Leu Ser His Met Pro Ala Ala Leu Gln Asp Phe
    290                 295                 300

Leu Leu Leu Lys Gln Lys Ala Glu Leu Ala Asn Pro Lys Ala Val
305                 310                 315

<210>  5
<211>  3099
<212>  DNA
<213>  Homo sapiens

<400>  5
tgcgaggcag agtgctagcg ggagcgcgag ccagcaagag gcgcctgcgc gatgtccggg      60

cccctgagcc cgcggcgctg agccagccgg gacggacatg cgcgggaggg cgccgcgggg     120

cagccgccgc tcctccgggg gaatgaaagc tactggttga ttttaaagtg cctgggcctc     180

acaggtttgg agatgtccca gaataaggca caatgtcaat agcaggagtt gctgctcagg     240

agatcagagt cccattaaaa actggatttc tacataatgg ccgagccatg gggaatatga     300

ggaagaccta ctggagcagt cgcagtgagt ttaaaaacaa ctttttaaat attgacccga     360

taaccatggc ctacagtctg aactcttctg ctcaggagcg cctaatacca cttgggcatg     420

cttccaaatc tgctccgatg aatggccact gctttgcaga aaatggtcca tctcaaaagt     480
```

```
ccagcttgcc ccctcttctt attcccccaa gtgaaaactt gggaccacat gaagaggatc     540
aagttgtatg tggttttaag aaactcacag tgaatggggt ttgtgcttcc acccctccac     600
tgacacccat aaaaaactcc ccttcccttt tcccctgtgc ccctctttgt gaacggggtt     660
ctaggcctct tccaccgttg ccaatctctg aagccctctc tctggatgac acagactgtg     720
aggtggaatt cctaactagc tcagatacag acttcctttt agaagactct acactttctg     780
atttcaaata tgatgttcct ggcaggcgaa gcttccgtgg gtgtggacaa atcaactatg     840
catattttga taccccagct gtttctgcag cagatctcag ctatgtgtct gaccaaaatg     900
gaggtgtccc agatccaaat cctcctccac ctcagaccca ccgaagatta agaaggtctc     960
attcgggacc agctggctcc tttaacaagc cagccataag gatatccaac tgttgtatac    1020
acagagcttc tcctaactcc gatgaagaca aacctgaggt tccccccaga gttcccatac    1080
ctcctagacc agtaaagcca gattatagaa gatggtcagc agaagttact tcgagcacct    1140
atagtgatga agacaggcct cccaaagtac cgccaagaga acctttgtca ccgagtaact    1200
cgcgcacacc gagtcccaaa agccttccgt cttacctcaa tggggtcatg cccccgacac    1260
agagctttgc ccctgatccc aagtatgtca gcagcaaagc actgcaaaga cagaacagcg    1320
aaggatctgc cagtaaggtt ccttgcattc tgcccattat tgaaaatggg aagaaggtta    1380
gttcaacaca ttattaccta ctacctgaac gaccaccata cctggacaaa tatgaaaaat    1440
tttttaggga agcagaagaa acaaatggag gcgcccaaat ccagccatta cctgctgact    1500
gcggtatatc ttcagccaca gaaaagccag actcaaaaac aaaaatggat ctgggtggcc    1560
acgtgaagcg taaacattta tcctatgtgg tttctcctta gaccttgggg tcatggttca    1620
gcagaggtta cataggagca aatggttctc aattttccag tttgattgaa gtgcagagaa    1680
aaatccctta gattgcaaaa taaaatagtt gaactctctg tcttcatgtg gaaggtttag    1740
agcagttgtg agatgctgtt atgctgagaa accctgactt tgttagtgtt ggaaaaaagt    1800
cttacaagtc tataatttaa agatgtgatg gtggggaggg gaggatgggg aagcttttta    1860
tatatgcata cattacatac ctatatataa acttgtggta taaccataga ccatagctgc    1920
aggttaacca attagttact atcgtagagt aatatatatt cagaataata aactcaagct    1980
ggagaaatga gtcctgatag actgaaaatt gagcaaatgg aagaagatac agtattgttt    2040
agatcagaat cattaaaaaa tatttttgtt tagtaagttt gaagatttct ggcttttagg    2100
ccttttctat tttgttccat ttatttttgc aggcaatctt ttccatggag ggcagggtat    2160
ccattcttta ccatgggtgt acctgcttag gttaaaaatc ataccaaggc ctcatacttc    2220
caggtttcat gttgcgtctt gttgagggag ggagagcagg ttacttggca accatattgt    2280
cacctgtacc tgtcacacat cttgaaaaat aaaacgataa tagaactagt gactaatttt    2340
cccttacagt tcctgcttgg tcccacccac tgaagtagct catcgtagtg cgggccgtat    2400
tagaagcagt ggggtacgtt agactcagat ggaaaagtat tctaggtgcc agtgttagga    2460
tgtcagtttt acaaaataat gaagcaatta gctatgtgat tgagagttat tgtttgggga    2520
tgtgtgttgt ggttttgctt tttttttttt agactgtatt aataaacata caacacaagc    2580
```

tggccttgtg ttgctggttc ctattcagta tttcctgggg attgtttgct ttttaagtaa 2640

aacacttctg acccatagct cagtatgtct gaattccaga ggtcacatca gcatctttct 2700

gctttgaaaa ctctcacagc tgtggctgct tcacttagat gcagtgagac acatagttgg 2760

tgttccgatt ttcacatcct tccatgtatt tatcttgaag agataagcac agaagagaag 2820

gtgctcacta acagaggtac attactgcaa tgttctctta acagttaaac aagctgttta 2880

cagtttaaac tgctgaatat tatttgagct atttaaagct tattatattt tagtatgaac 2940

taaatgaagg ttaaaacatg cttaagaaaa atgcactgat ttctgcatta tgtgtacagt 3000

attggacaaa ggattttatt cattttgttg cattattttg aatattgtct tttcatttta 3060

ataaagttat aatacttaaa aaaaaaaaaa aaaaaaaaa 3099

<210> 6
<211> 462
<212> PRT
<213> Homo sapiens

<400> 6

Met Ser Ile Ala Gly Val Ala Ala Gln Glu Ile Arg Val Pro Leu Lys
1 5 10 15

Thr Gly Phe Leu His Asn Gly Arg Ala Met Gly Asn Met Arg Lys Thr
20 25 30

Tyr Trp Ser Ser Arg Ser Glu Phe Lys Asn Asn Phe Leu Asn Ile Asp
35 40 45

Pro Ile Thr Met Ala Tyr Ser Leu Asn Ser Ser Ala Gln Glu Arg Leu
50 55 60

Ile Pro Leu Gly His Ala Ser Lys Ser Ala Pro Met Asn Gly His Cys
65 70 75 80

Phe Ala Glu Asn Gly Pro Ser Gln Lys Ser Ser Leu Pro Pro Leu Leu
85 90 95

Ile Pro Pro Ser Glu Asn Leu Gly Pro His Glu Glu Asp Gln Val Val
100 105 110

Cys Gly Phe Lys Lys Leu Thr Val Asn Gly Val Cys Ala Ser Thr Pro
115 120 125

Pro Leu Thr Pro Ile Lys Asn Ser Pro Ser Leu Phe Pro Cys Ala Pro
130 135 140

Leu Cys Glu Arg Gly Ser Arg Pro Leu Pro Pro Leu Pro Ile Ser Glu
145 150 155 160

Ala Leu Ser Leu Asp Asp Thr Asp Cys Glu Val Glu Phe Leu Thr Ser
165 170 175

Ser Asp Thr Asp Phe Leu Leu Glu Asp Ser Thr Leu Ser Asp Phe Lys
180 185 190

Tyr Asp Val Pro Gly Arg Arg Ser Phe Arg Gly Cys Gly Gln Ile Asn
195 200 205

Tyr Ala Tyr Phe Asp Thr Pro Ala Val Ser Ala Ala Asp Leu Ser Tyr
210 215 220

Val Ser Asp Gln Asn Gly Gly Val Pro Asp Pro Asn Pro Pro Pro Pro
225 230 235 240

Gln Thr His Arg Arg Leu Arg Arg Ser His Ser Gly Pro Ala Gly Ser
245 250 255

Phe Asn Lys Pro Ala Ile Arg Ile Ser Asn Cys Cys Ile His Arg Ala
260 265 270

Ser Pro Asn Ser Asp Glu Asp Lys Pro Glu Val Pro Pro Arg Val Pro
275 280 285

Ile Pro Pro Arg Pro Val Lys Pro Asp Tyr Arg Arg Trp Ser Ala Glu
290 295 300

Val Thr Ser Ser Thr Tyr Ser Asp Glu Asp Arg Pro Pro Lys Val Pro
305 310 315 320

Pro Arg Glu Pro Leu Ser Pro Ser Asn Ser Arg Thr Pro Ser Pro Lys
325 330 335

Ser Leu Pro Ser Tyr Leu Asn Gly Val Met Pro Pro Thr Gln Ser Phe
340 345 350

Ala Pro Asp Pro Lys Tyr Val Ser Ser Lys Ala Leu Gln Arg Gln Asn
355 360 365

Ser Glu Gly Ser Ala Ser Lys Val Pro Cys Ile Leu Pro Ile Ile Glu
370 375 380

Asn Gly Lys Lys Val Ser Ser Thr His Tyr Tyr Leu Leu Pro Glu Arg
385 390 395 400

Pro Pro Tyr Leu Asp Lys Tyr Glu Lys Phe Phe Arg Glu Ala Glu Glu
405 410 415

Thr Asn Gly Gly Ala Gln Ile Gln Pro Leu Pro Ala Asp Cys Gly Ile
420 425 430

Ser Ser Ala Thr Glu Lys Pro Asp Ser Lys Thr Lys Met Asp Leu Gly
435 440 445

```
Gly His Val Lys Arg Lys His Leu Ser Tyr Val Val Ser Pro
    450                 455                 460
```

<210> 7
<211> 4465
<212> DNA
<213> Homo sapiens

<400> 7

```
caattgtcat acgacttgca gtgagcgtca ggagcacgtc caggaactcc tcagcagcgc      60
ctccttcagc tccacagcca gacgccctca gacagcaaag cctacccccg cgccgcgccc     120
tgcccgccgc tcggatgctc gcccgcgccc tgctgctgtg cgcggtcctg gcgctcagcc     180
atacagcaaa tccttgctgt tcccacccat gtcaaaaccg aggtgtatgt atgagtgtgg     240
gatttgacca gtataagtgc gattgtaccc ggacaggatt ctatggagaa aactgctcaa     300
caccggaatt tttgacaaga ataaaattat ttctgaaacc cactccaaac acagtgcact     360
acatacttac ccacttcaag ggattttgga acgttgtgaa taacattccc ttccttcgaa     420
atgcaattat gagttatgtc ttgacatcca gatcacattt gattgacagt ccaccaactt     480
acaatgctga ctatggctac aaaagctggg aagccttctc taacctctcc tattatacta     540
gagcccttcc tcctgtgcct gatgattgcc cgactccctt gggtgtcaaa ggtaaaaagc     600
agcttcctga ttcaaatgag attgtggaaa aattgcttct aagaagaaag ttcatccctg     660
atccccaggg ctcaaacatg atgtttgcat tctttgccca gcacttcacg catcagtttt     720
tcaagacaga tcataagcga gggccagctt tcaccaacgg gctgggccat ggggtggact     780
taaatcatat ttacggtgaa actctggcta gacagcgtaa actgcgcctt ttcaaggatg     840
gaaaaatgaa atatcagata attgatggag agatgtatcc tcccacagtc aaagatactc     900
aggcagagat gatctaccct cctcaagtcc ctgagcatct acggtttgct gtggggcagg     960
aggtctttgg tctggtgcct ggtctgatga tgtatgccac aatctggctg cgggaacaca    1020
acagagtatg cgatgtgctt aaacaggagc atcctgaatg gggtgatgag cagttgttcc    1080
agacaagcag gctaatactg ataggagaga ctattaagat tgtgattgaa gattatgtgc    1140
aacacttgag tggctatcac ttcaaactga aatttgaccc agaactactt ttcaacaaac    1200
aattccagta ccaaaatcgt attgctgctg aatttaacac cctctatcac tggcatcccc    1260
ttctgcctga cacctttcaa attcatgacc agaaatacaa ctatcaacag tttatctaca    1320
acaactctat attgctggaa catggaatta cccagtttgt tgaatcattc accaggcaaa    1380
ttgctggcag ggttgctggt ggtaggaatg ttccacccgc agtacagaaa gtatcacagg    1440
cttccattga ccagagcagg cagatgaaat accagtcttt taatgagtac cgcaaacgct    1500
ttatgctgaa gccctatgaa tcatttgaag aacttacagg agaaaaggaa atgtctgcag    1560
agttggaagc actctatggt gacatcgatg ctgtggagct gtatcctgcc cttctggtag    1620
aaaagcctcg gccagatgcc atctttggtg aaaccatggt agaagttgga gcaccattct    1680
ccttgaaagg acttatgggt aatgttatat gttctcctgc ctactggaag ccaagcactt    1740
```

ttggtggaga agtgggtttt caaatcatca acactgcctc aattcagtct ctcatctgca 1800
ataacgtgaa gggctgtccc tttacttcat tcagtgttcc agatccagag ctcattaaaa 1860
cagtcaccat caatgcaagt tcttcccgct ccggactaga tgatatcaat cccacagtac 1920
tactaaaaga acgttcgact gaactgtaga agtctaatga tcatatttat ttatttatat 1980
gaaccatgtc tattaattta attatttaat aatatttata ttaaactcct tatgttactt 2040
aacatcttct gtaacagaag tcagtactcc tgttgcggag aaaggagtca tacttgtgaa 2100
gacttttatg tcactactct aaagattttg ctgttgctgt taagtttgga aaacagtttt 2160
tattctgttt tataaaccag agagaaatga gttttgacgt ctttttactt gaatttcaac 2220
ttatattata agaacgaaag taaagatgtt tgaatactta aacactatca caagatggca 2280
aaatgctgaa agtttttaca ctgtcgatgt ttccaatgca tcttccatga tgcattagaa 2340
gtaactaatg tttgaaattt taaagtactt ttggttattt ttctgtcatc aaacaaaaac 2400
aggtatcagt gcattattaa atgaatattt aaattagaca ttaccagtaa tttcatgtct 2460
actttttaaa atcagcaatg aaacaataat ttgaaatttc taaattcata gggtagaatc 2520
acctgtaaaa gcttgtttga tttcttaaag ttattaaact tgtacatata ccaaaaagaa 2580
gctgtcttgg atttaaatct gtaaaatcag atgaaatttt actacaattg cttgttaaaa 2640
tattttataa gtgatgttcc tttttcacca agagtataaa cctttttagt gtgactgtta 2700
aaacttcctt ttaaatcaaa atgccaaatt tattaaggtg gtggagccac tgcagtgtta 2760
tctcaaaata agaatatttt gttgagatat tccagaattt gtttatatgg ctggtaacat 2820
gtaaaatcta tatcagcaaa agggtctacc tttaaaataa gcaataacaa agaagaaaac 2880
caaattattg ttcaaattta ggtttaaact tttgaagcaa actttttttt atccttgtgc 2940
actgcaggcc tggtactcag attttgctat gaggttaatg aagtaccaag ctgtgcttga 3000
ataacgatat gttttctcag attttctgtt gtacagttta atttagcagt ccatatcaca 3060
ttgcaaaagt agcaatgacc tcataaaata cctcttcaaa atgcttaaat tcatttcaca 3120
cattaatttt atctcagtct tgaagccaat tcagtaggtg cattggaatc aagcctggct 3180
acctgcatgc tgttcctttt cttttcttct tttagccatt ttgctaagag acacagtctt 3240
ctcatcactt cgtttctcct attttgtttt actagtttta agatcagagt tcactttctt 3300
tggactctgc ctatattttc ttacctgaac ttttgcaagt tttcaggtaa acctcagctc 3360
aggactgcta tttagctcct cttaagaaga ttaaaagaga aaaaaaaagg ccccttttaaa 3420
aatagtatac acttatttta agtgaaaagc agagaatttt atttatagct aattttagct 3480
atctgtaacc aagatggatg caaagaggct agtgcctcag agagaactgt acggggtttg 3540
tgactggaaa aagttacgtt cccattctaa ttaatgccct ttcttattta aaaacaaaac 3600
caaatgatat ctaagtagtt ctcagcaata ataataatga cgataatact tcttttccac 3660
atctcattgt cactgacatt taatggtact gtatattact taatttattg aagattatta 3720
tttatgtctt attaggacac tatggttata aactgtgttt aagcctacaa tcattgattt 3780
ttttttgtta tgtcacaatc agtatatttt ctttggggtt acctctctga atattatgta 3840

aacaatccaa agaaatgatt gtattaagat ttgtgaataa atttttagaa atctgattgg 3900
catattgaga tatttaaggt tgaatgtttg tccttaggat aggcctatgt gctagcccac 3960
aaagaatatt gtctcattag cctgaatgtg ccataagact gaccttttaa aatgttttga 4020
gggatctgtg gatgcttcgt taatttgttc agccacaatt tattgagaaa atattctgtg 4080
tcaagcactg tgggttttaa tatttttaaa tcaaacgctg attacagata atagtattta 4140
tataaataat tgaaaaaaat tttcttttgg gaagagggag aaaatgaaat aaatatcatt 4200
aaagataact caggagaatc ttctttacaa ttttacgttt agaatgttta aggttaagaa 4260
agaaatagtc aatatgcttg tataaaacac tgttcactgt ttttttaaa aaaaaaactt 4320
gatttgttat taacattgat ctgctgacaa aacctgggaa tttgggttgt gtatgcgaat 4380
gtttcagtgc ctcagacaaa tgtgtattta acttatgtaa aagataagtc tggaaataaa 4440
tgtctgttta tttttgtact attta 4465

<210> 8
<211> 604
<212> PRT
<213> Homo sapiens

<400> 8

Met Leu Ala Arg Ala Leu Leu Leu Cys Ala Val Leu Ala Leu Ser His
1 5 10 15

Thr Ala Asn Pro Cys Cys Ser His Pro Cys Gln Asn Arg Gly Val Cys
20 25 30

Met Ser Val Gly Phe Asp Gln Tyr Lys Cys Asp Cys Thr Arg Thr Gly
35 40 45

Phe Tyr Gly Glu Asn Cys Ser Thr Pro Glu Phe Leu Thr Arg Ile Lys
50 55 60

Leu Phe Leu Lys Pro Thr Pro Asn Thr Val His Tyr Ile Leu Thr His
65 70 75 80

Phe Lys Gly Phe Trp Asn Val Val Asn Asn Ile Pro Phe Leu Arg Asn
85 90 95

Ala Ile Met Ser Tyr Val Leu Thr Ser Arg Ser His Leu Ile Asp Ser
100 105 110

Pro Pro Thr Tyr Asn Ala Asp Tyr Gly Tyr Lys Ser Trp Glu Ala Phe
115 120 125

Ser Asn Leu Ser Tyr Tyr Thr Arg Ala Leu Pro Pro Val Pro Asp Asp
130 135 140

Cys Pro Thr Pro Leu Gly Val Lys Gly Lys Lys Gln Leu Pro Asp Ser
145 150 155 160

```
Asn Glu Ile Val Glu Lys Leu Leu Leu Arg Arg Lys Phe Ile Pro Asp
                165                 170                 175

Pro Gln Gly Ser Asn Met Met Phe Ala Phe Phe Ala Gln His Phe Thr
            180                 185                 190

His Gln Phe Phe Lys Thr Asp His Lys Arg Gly Pro Ala Phe Thr Asn
        195                 200                 205

Gly Leu Gly His Gly Val Asp Leu Asn His Ile Tyr Gly Glu Thr Leu
    210                 215                 220

Ala Arg Gln Arg Lys Leu Arg Leu Phe Lys Asp Gly Lys Met Lys Tyr
225                 230                 235                 240

Gln Ile Ile Asp Gly Glu Met Tyr Pro Pro Thr Val Lys Asp Thr Gln
                245                 250                 255

Ala Glu Met Ile Tyr Pro Pro Gln Val Pro Glu His Leu Arg Phe Ala
            260                 265                 270

Val Gly Gln Glu Val Phe Gly Leu Val Pro Gly Leu Met Met Tyr Ala
        275                 280                 285

Thr Ile Trp Leu Arg Glu His Asn Arg Val Cys Asp Val Leu Lys Gln
    290                 295                 300

Glu His Pro Glu Trp Gly Asp Glu Gln Leu Phe Gln Thr Ser Arg Leu
305                 310                 315                 320

Ile Leu Ile Gly Glu Thr Ile Lys Ile Val Ile Glu Asp Tyr Val Gln
                325                 330                 335

His Leu Ser Gly Tyr His Phe Lys Leu Lys Phe Asp Pro Glu Leu Leu
            340                 345                 350

Phe Asn Lys Gln Phe Gln Tyr Gln Asn Arg Ile Ala Ala Glu Phe Asn
        355                 360                 365

Thr Leu Tyr His Trp His Pro Leu Leu Pro Asp Thr Phe Gln Ile His
    370                 375                 380

Asp Gln Lys Tyr Asn Tyr Gln Gln Phe Ile Tyr Asn Asn Ser Ile Leu
385                 390                 395                 400

Leu Glu His Gly Ile Thr Gln Phe Val Glu Ser Phe Thr Arg Gln Ile
                405                 410                 415

Ala Gly Arg Val Ala Gly Gly Arg Asn Val Pro Pro Ala Val Gln Lys
            420                 425                 430
```

```
Val Ser Gln Ala Ser Ile Asp Gln Ser Arg Gln Met Lys Tyr Gln Ser
        435                 440                 445

Phe Asn Glu Tyr Arg Lys Arg Phe Met Leu Lys Pro Tyr Glu Ser Phe
    450                 455                 460

Glu Glu Leu Thr Gly Glu Lys Glu Met Ser Ala Glu Leu Glu Ala Leu
465                 470                 475                 480

Tyr Gly Asp Ile Asp Ala Val Glu Leu Tyr Pro Ala Leu Leu Val Glu
                485                 490                 495

Lys Pro Arg Pro Asp Ala Ile Phe Gly Glu Thr Met Val Glu Val Gly
            500                 505                 510

Ala Pro Phe Ser Leu Lys Gly Leu Met Gly Asn Val Ile Cys Ser Pro
        515                 520                 525

Ala Tyr Trp Lys Pro Ser Thr Phe Gly Gly Glu Val Gly Phe Gln Ile
    530                 535                 540

Ile Asn Thr Ala Ser Ile Gln Ser Leu Ile Cys Asn Asn Val Lys Gly
545                 550                 555                 560

Cys Pro Phe Thr Ser Phe Ser Val Pro Asp Pro Glu Leu Ile Lys Thr
                565                 570                 575

Val Thr Ile Asn Ala Ser Ser Ser Arg Ser Gly Leu Asp Asp Ile Asn
            580                 585                 590

Pro Thr Val Leu Leu Lys Glu Arg Ser Thr Glu Leu
        595                 600


<210>  9
<211>  4342
<212>  DNA
<213>  Homo sapiens

<400>  9
gatcaatact gaggccgcgt ccgacccccct tgagccgaga tcccccccca gcccagcccc      60
caccccaccc cccgcacacg ccccaccccc cccacgaccc agcctcatac cgcaccagct     120
gaggcaccca agaggattac cccctggggc cctctcccgc cccccaaaaa agagaagatc     180
ccctctcctg gcccatccct tcccttcttc cctccccccct cccccgaac tttccctctc     240
gcatgctttt cccctgcacc acggatcgcc tctcggatgc cgcttgcctg gaagctgcgt     300
taggagcgag cggcggcggt ggcggcggtg gcggcggcgg cggcagctcg ggagtgctat     360
gaccggcaaa ctcgccgaga agctgccggt gaccatgagc agtttgctaa accaactgcc     420
tgacaatctg taccccgagg agatccccag cgcgctcaac ctcttctccg gcagcagcga     480
ctcggtagtc cattacaatc agatggctac agagaatgta atggacatcg gtctgaccaa     540
```

```
cgagaagccc aacccggaac tctcttactc cggctccttc cagccagccc ccggcaacaa     600
gaccgtgacc tacttgggaa agttcgcctt cgactcccct tccaactggt gccaggacaa     660
catcattagc ctcatgagcg ccggcatctt gggggtgccc ccggcttcag gggcgctcag     720
cacgcagacg tccacggcca gcatggtgca gccaccgcag ggtgacgtgg aggccatgta     780
tcccgcgcta ccccccctact ccaactgcgg cgacctctac tcagagcccg tgtctttcca     840
cgaccccccag ggcaatcccg ggctcgccta ttcccccccag gattaccaat cggccaagcc     900
ggcgttggac agcaatctct tccccatgat tcctgactac aacctctacc accaccccaa     960
cgacatgggc tccattccgg agcacaagcc cttccagggc atggaccccca tccgggtcaa    1020
cccgcccccct attacccctc tggagaccat caaggcattc aaagacaagc agatccaccc    1080
gggctttggc agcctgcccc agccgccgct caccctcaag cccatccggc cccgcaagta    1140
ccccaaccgg cctagcaaga caccgctcca cgaacggccc cacgcgtgcc cggccgaggg    1200
ctgcgaccgc cgtttcagcc gttcggacga gctgacccgg cacctgcgca tccacacggg    1260
ccacaagccc ttccagtgcc ggatctgcat gcggagcttc agccgcagcg accacctcac    1320
cactcacatc cgcactcata cgggcgagaa gccctttgcc tgcgagttct gcgggcgcaa    1380
gtttgcgcgc agcgacgagc gcaagcgcca cgccaagatc cacctcaagc aaaaggagaa    1440
gaaggcggag aagggcggtg caccctctgc atcctcggcg ccccccgtgt cgctggcccc    1500
cgtggtcacc acctgcgcct gaggatcggg cccccagatc cccacttttc ccctccagtg    1560
cctcccggct gctagcctga aagcagcggg aaagccagcc acggaggcgt aggggccgcg    1620
ccctggcctc tccatggacg tgcggcccct tgcttcccct tcgatgcccc cggttcccaa    1680
cctttcacgc cggccagcgg tcaggggcca gggctggagg cgccttcccc tcgcggtccc    1740
ccacttagcc aaggcgtggg ggcggaaagg tggcgtctag cccgctttgt tcagttcgga    1800
tcgccttgat ccaggggccg ccgggccgcg ccaaggacct gcaagggact gaaggcggag    1860
cccatccaac cctcgcccga cccaaacacc tcattgtttc cccccacgtct ccctctatac    1920
cccctcgaag actcgagagg gggagggggt aaggagcgca ccaaagcgca gagcttgctg    1980
cccgccgcac gcacgcgcgc ctgcgtgcgg ggatgcgcgc gagtgtgtgc gtgctcgcgt    2040
gtgtgtgtat gtgtgtgtgt gagtgtgtgt gtgtgcgcgc gcgcaagcgt gtgtgtttaa    2100
gactcttgag ctgaactggg ctgtgtttac cccaaactct tccccacctc gggtccccaa    2160
gccgctggga gatgtcccat gctgggggtc cgcacgtggc tggaggaggt ggtcttccat    2220
ccgctctgaa atcatgtttc ttagagaaat gcctcggatg ccgccgacgc ggtgctgctg    2280
ccgccgcttc gggtttggcc cctcagaacc cctccttttc tgagcgcttc cctcttaggc    2340
ctcagggcag tttgatctgt ggggagaaag agcagccatc gctgagcctg cctttttaaaa    2400
tatatgtgta tttccttagc cccactctaa gaaatctatg ttcctgagtt tgccccctgc    2460
cctcccactc cttccccttc tcccctctaa accttctccc atctctttca aaatcttttc    2520
ccagaaaggc aggcttcaac cagccactcc agctttgtgt cttctctcaa ttacatagca    2580
atttctcctt cccaccatca tggggaagct ggctctgctt ttgccctttg tcatcaccaa    2640
```

cacaacagat agaatttaaa tataagtata tggtgtgcgt gtgtatgtat gtgtatgtat 2700
atgcatgcat gtgtataaag atgcacatgc gtacatatac ataacataca cacaatatgt 2760
attcctagca aaataaaatc tctaaggtac ttggttatcc agtgcagtgc accggaataa 2820
agagaatttg taggcgtata cagctttaaa tgatttattt tttatgaaaa tgttaactga 2880
tgagattata tctacatacg tgtgttatgt gtgtggatat agatgcacac atatctgtac 2940
atatacatgt gagtgtacat atatacacat atacacatct atgtatggat atgtgtgtat 3000
acacatatac acatctctgt ccaaaatttc ctcaaagata tggggatttt ttcatgaatc 3060
catgtggatg aatgaggtgt ctcctttcca tacccagtct caccttctcc ccaccctacc 3120
tcacctcttc tcaggcaccc ctcttcccca gctgtcctgc cagcccttct cgtacagggt 3180
ggctcctttg aagtggagta atagggaagg ttgctctctg ccacagcttg cagcatggtc 3240
ttgactgaat gtactgttcc tgttagcgtt acttctcctg tggtcagtaa gttgcccaga 3300
gagaaggaac aaaggtctgg agtttacaga atgtctgttt ttaaagtcac tttatgcgtt 3360
ttccactttt ttcttttta agaaaaagaa gtaccatttt tgttttgttt tgttttgatt 3420
tttggtggtg gataaataat actaaaagga ctctagtgga aaggggggat atcgaagagc 3480
agggggttgt gaatttccag gtacttggac tttttgtaga aggagagaga agaagatgaa 3540
gtttgccagg agggcccata ttttttcagc tgaagggtaa aatctttctt tgcagagaca 3600
gtattttgct gaatactttt tataatgtga tgattattaa aaaagcaaaa attttggtca 3660
cttccaagct agaaggagga atcagatacc ctttaatatt ttttcctcgc tccttctggt 3720
atatgcatgt cactgcatga taattgagtt ttcctttgtt ttaataaaac tgttctcaga 3780
cattaagcta aactaagaga aaaataactt tgttgccaaa aggttgtgct atccagattt 3840
tttatatgtc tgcatgttta aaaaaaaaaa gcaacaaaag aaaatgcact ctaacttatg 3900
tgaactgaga gaaaaaaatc aggttttaaa caggaaaacc tatggggaat gatatttttt 3960
gaaagacttt tgtataaagt tgagtactta gaaaaaagac aaaccagatg taatatattt 4020
tgtggatgtt tttatttctt ggatttatag taccttatac taaggttaaa aaaatatgct 4080
tgatattgtg aaaaggtgaa attcttcacc aacatttcat ttgctccttt gtcatattgt 4140
aatgccaata taatatagtt aatgaaaaca gcatttttaa aaaccgaaat attgaaatgg 4200
tgtaatgttg taccatttgc actgtgagca aatgctaata cagtaaatat attgtgtttg 4260
ctgacaatca gccggcctat aaatctcctt attttatttc ttgtttttat agcataaagc 4320
tttagtttgg cctgaaaaaa aa 4342

<210> 10
<211> 387
<212> PRT
<213> Homo sapiens

<400> 10

Met Thr Gly Lys Leu Ala Glu Lys Leu Pro Val Thr Met Ser Ser Leu
1 5 10 15

```
Leu Asn Gln Leu Pro Asp Asn Leu Tyr Pro Glu Glu Ile Pro Ser Ala
            20                  25                  30

Leu Asn Leu Phe Ser Gly Ser Ser Asp Ser Val Val His Tyr Asn Gln
        35                  40                  45

Met Ala Thr Glu Asn Val Met Asp Ile Gly Leu Thr Asn Glu Lys Pro
    50                  55                  60

Asn Pro Glu Leu Ser Tyr Ser Gly Ser Phe Gln Pro Ala Pro Gly Asn
65                  70                  75                  80

Lys Thr Val Thr Tyr Leu Gly Lys Phe Ala Phe Asp Ser Pro Ser Asn
                85                  90                  95

Trp Cys Gln Asp Asn Ile Ile Ser Leu Met Ser Ala Gly Ile Leu Gly
            100                 105                 110

Val Pro Pro Ala Ser Gly Ala Leu Ser Thr Gln Thr Ser Thr Ala Ser
        115                 120                 125

Met Val Gln Pro Pro Gln Gly Asp Val Glu Ala Met Tyr Pro Ala Leu
    130                 135                 140

Pro Pro Tyr Ser Asn Cys Gly Asp Leu Tyr Ser Glu Pro Val Ser Phe
145                 150                 155                 160

His Asp Pro Gln Gly Asn Pro Gly Leu Ala Tyr Ser Pro Gln Asp Tyr
                165                 170                 175

Gln Ser Ala Lys Pro Ala Leu Asp Ser Asn Leu Phe Pro Met Ile Pro
            180                 185                 190

Asp Tyr Asn Leu Tyr His His Pro Asn Asp Met Gly Ser Ile Pro Glu
        195                 200                 205

His Lys Pro Phe Gln Gly Met Asp Pro Ile Arg Val Asn Pro Pro Pro
    210                 215                 220

Ile Thr Pro Leu Glu Thr Ile Lys Ala Phe Lys Asp Lys Gln Ile His
225                 230                 235                 240

Pro Gly Phe Gly Ser Leu Pro Gln Pro Pro Leu Thr Leu Lys Pro Ile
                245                 250                 255

Arg Pro Arg Lys Tyr Pro Asn Arg Pro Ser Lys Thr Pro Leu His Glu
            260                 265                 270

Arg Pro His Ala Cys Pro Ala Glu Gly Cys Asp Arg Arg Phe Ser Arg
        275                 280                 285
```

```
Ser Asp Glu Leu Thr Arg His Leu Arg Ile His Thr Gly His Lys Pro
    290                 295                 300

Phe Gln Cys Arg Ile Cys Met Arg Ser Phe Ser Arg Ser Asp His Leu
305                 310                 315                 320

Thr Thr His Ile Arg Thr His Thr Gly Glu Lys Pro Phe Ala Cys Glu
                325                 330                 335

Phe Cys Gly Arg Lys Phe Ala Arg Ser Asp Glu Arg Lys Arg His Ala
            340                 345                 350

Lys Ile His Leu Lys Gln Lys Glu Lys Lys Ala Glu Lys Gly Gly Ala
        355                 360                 365

Pro Ser Ala Ser Ser Ala Pro Pro Val Ser Leu Ala Pro Val Val Thr
    370                 375                 380

Thr Cys Ala
385

<210>  11
<211>  3497
<212>  DNA
<213>  Homo sapiens

<400>  11
aggctcagca cagagagtgg aaaacagcag aggtgacaga gcagccgtgc tcgaagcgtt      60
cctggagccc aagctctcct ccacaggtga agacagggcc agcaggagac accatggggc     120
acctctcagc cccacttcac agagtgcgtg taccctggca ggggcttctg ctcacagcct     180
cacttctaac cttctggaac ccgcccacca ctgcccagct cactactgaa tccatgccat     240
tcaatgttgc agaggggaag gaggttcttc tccttgtcca caatctgccc cagcaacttt     300
ttggctacag ctggtacaaa ggggaaagag tggatggcaa ccgtcaaatt gtaggatatg     360
caataggaac tcaacaagct accccagggc ccgcaaacag cggtcgagag acaatatacc     420
ccaatgcatc cctgctgatc cagaacgtca cccagaatga cacaggattc tacaccctac     480
aagtcataaa gtcagatctt gtgaatgaag aagcaactgg acagttccat gtatacccgg     540
agctgcccaa gccctccatc tccagcaaca actccaaccc tgtggaggac aaggatgctg     600
tggccttcac ctgtgaacct gagactcagg acacaaccta cctgtggtgg ataaacaatc     660
agagcctccc ggtcagtccc aggctgcagc tgtccaatgg caacaggacc ctcactctac     720
tcagtgtcac aaggaatgac acaggaccct atgagtgtga aatacagaac ccagtgagtg     780
cgaaccgcag tgacccagtc accttgaatg tcacctatgg cccggacacc cccaccattt     840
ccccttcaga cacctattac cgtccagggg caaacctcag cctctcctgc tatgcagcct     900
ctaacccacc tgcacagtac tcctggctta tcaatggaac attccagcaa agcacacaag     960
agctctttat ccctaacatc actgtgaata atagtggatc ctatacctgc cacgccaata    1020
```

```
actcagtcac tggctgcaac aggaccacag tcaagacgat catagtcact gagctaagtc   1080
cagtagtagc aaagccccaa atcaaagcca gcaagaccac agtcacagga gataaggact   1140
ctgtgaacct gacctgctcc acaaatgaca ctggaatctc catccgttgg ttcttcaaaa   1200
accagagtct cccgtcctcg gagaggatga agctgtccca gggcaacacc accctcagca   1260
taaaccctgt caagagggag gatgctggga cgtattggtg tgaggtcttc aacccaatca   1320
gtaagaacca aagcgacccc atcatgctga acgtaaacta taatgctcta ccacaagaaa   1380
atggcctctc acctggggcc attgctggca ttgtgattgg agtagtggcc ctggttgctc   1440
tgatagcagt agccctggca tgttttctgc atttcgggaa gaccggcagg gcaagcgacc   1500
agcgtgatct cacagagcac aaaccctcag tctccaacca cactcaggac cactccaatg   1560
acccacctaa caagatgaat gaagttactt attctaccct gaactttgaa gcccagcaac   1620
ccacacaacc aacttcagcc tccccatccc taacagccac agaaataatt tattcagaag   1680
taaaaaagca gtaatgaaac ctgtcctgct cactgcagtg ctgatgtatt tcaagtctct   1740
caccctcatc actaggagat tcctttcccc tgtaggggta gaggggtggg gacagaaaca   1800
actttctcct actcttcctt cctaataggc atctccaggc tgcctggtca ctgcccctct   1860
ctcagtgtca atagatgaaa gtacattggg agtctgtagg aaacccaacc ttcttgtcat   1920
tgaaatttgg caaagctgac tttgggaaag agggaccaga acttcccctc ccttcccctt   1980
ttcccaacct ggacttgttt taaacttgcc tgttcagagc actcattcct tcccaccccc   2040
agtcctgtcc tatcactcta attcggattt gccatagcct tgaggttatg tccttttcca   2100
ttaagtacat gtgccaggaa acaagagaga gagaaagtaa aggcagtaat gccttctcct   2160
atttctccaa agccttgtgt gaactcacca aacacaagaa aatcaaatat ataaccaata   2220
gtgaaatgcc acacctttgt ccactgtcag ggttgtctac ctgtaggatc agggtctaag   2280
caccttggtg cttagctaga ataccaccta atccttctgg caagcctgtc ttcagagaac   2340
ccactagaag caactaggaa aatcacttgc caaaatccaa ggcaattcct gatggaaaat   2400
gcaaaagcac atatatgttt taatatcttt atgggctctg ttcaaggcag tgctgagagg   2460
gaggggttat agcttcagga gggaaccagc ttctgataaa cacaatctgc taggaacttg   2520
ggaaaggaat cagagagctg cccttcagcg attatttaaa ttattgttaa agaatacaca   2580
atttggggta ttgggatttt tctccttttc tctgagacat tccaccattt taatttttgt   2640
aactgcttat ttatgtgaaa agggttattt ttacttagct tagctatgtc agccaatccg   2700
attgccttag gtgaaagaaa ccaccgaaat ccctcaggtc ccttggtcag gagcctctca   2760
agattttttt tgtcagaggc tccaaataga aaataagaaa aggttttctt cattcatggc   2820
tagagctaga tttaactcag tttctaggca cctcagacca atcatcaact accattctat   2880
tccatgtttg cacctgtgca ttttctgttt gcccccattc actttgtcag gaaaccttgg   2940
cctctgctaa ggtgtatttg gtccttgaga agtgggagca ccctacaggg acactatcac   3000
tcatgctggt ggcattgttt acagctagaa agctgcactg gtgctaatgc cccttgggga   3060
aatggggctg tgaggaggag gattataact taggcctagc ctcttttaac agcctctgaa   3120
```

```
atttatcttt tcttctatgg ggtctataaa tgtatcttat aataaaaagg aaggacagga   3180
ggaagacagg caaatgtact tctcacccag tcttctacac agatggaatc tctttggggc   3240
taagagaaag gttttattct atattgctta cctgatctca tgttaggcct aagaggcttt   3300
ctccaggagg attagcttgg agttctctat actcaggtac ctctttcagg gttttctaac   3360
cctgacacgg actgtgcata ctttccctca tccatgctgt gctgtgttat ttaatttttc   3420
ctggctaaga tcatgtctga attatgtatg aaaattattc tatgttttta taataaaaat   3480
aatatatcag acatcga                                                  3497
```

```
<210>  12
<211>  526
<212>  PRT
<213>  Homo sapiens

<400>  12

Met Gly His Leu Ser Ala Pro Leu His Arg Val Arg Val Pro Trp Gln
1               5                   10                  15

Gly Leu Leu Leu Thr Ala Ser Leu Leu Thr Phe Trp Asn Pro Pro Thr
            20                  25                  30

Thr Ala Gln Leu Thr Thr Glu Ser Met Pro Phe Asn Val Ala Glu Gly
        35                  40                  45

Lys Glu Val Leu Leu Leu Val His Asn Leu Pro Gln Gln Leu Phe Gly
    50                  55                  60

Tyr Ser Trp Tyr Lys Gly Glu Arg Val Asp Gly Asn Arg Gln Ile Val
65                  70                  75                  80

Gly Tyr Ala Ile Gly Thr Gln Gln Ala Thr Pro Gly Pro Ala Asn Ser
                85                  90                  95

Gly Arg Glu Thr Ile Tyr Pro Asn Ala Ser Leu Leu Ile Gln Asn Val
            100                 105                 110

Thr Gln Asn Asp Thr Gly Phe Tyr Thr Leu Gln Val Ile Lys Ser Asp
        115                 120                 125

Leu Val Asn Glu Glu Ala Thr Gly Gln Phe His Val Tyr Pro Glu Leu
    130                 135                 140

Pro Lys Pro Ser Ile Ser Ser Asn Asn Ser Asn Pro Val Glu Asp Lys
145                 150                 155                 160

Asp Ala Val Ala Phe Thr Cys Glu Pro Glu Thr Gln Asp Thr Thr Tyr
                165                 170                 175

Leu Trp Trp Ile Asn Asn Gln Ser Leu Pro Val Ser Pro Arg Leu Gln
            180                 185                 190
```

```
Leu Ser Asn Gly Asn Arg Thr Leu Thr Leu Leu Ser Val Thr Arg Asn
        195                 200                 205

Asp Thr Gly Pro Tyr Glu Cys Glu Ile Gln Asn Pro Val Ser Ala Asn
    210                 215                 220

Arg Ser Asp Pro Val Thr Leu Asn Val Thr Tyr Gly Pro Asp Thr Pro
225                 230                 235                 240

Thr Ile Ser Pro Ser Asp Thr Tyr Tyr Arg Pro Gly Ala Asn Leu Ser
                245                 250                 255

Leu Ser Cys Tyr Ala Ala Ser Asn Pro Pro Ala Gln Tyr Ser Trp Leu
            260                 265                 270

Ile Asn Gly Thr Phe Gln Gln Ser Thr Gln Glu Leu Phe Ile Pro Asn
        275                 280                 285

Ile Thr Val Asn Asn Ser Gly Ser Tyr Thr Cys His Ala Asn Asn Ser
    290                 295                 300

Val Thr Gly Cys Asn Arg Thr Thr Val Lys Thr Ile Ile Val Thr Glu
305                 310                 315                 320

Leu Ser Pro Val Val Ala Lys Pro Gln Ile Lys Ala Ser Lys Thr Thr
                325                 330                 335

Val Thr Gly Asp Lys Asp Ser Val Asn Leu Thr Cys Ser Thr Asn Asp
            340                 345                 350

Thr Gly Ile Ser Ile Arg Trp Phe Phe Lys Asn Gln Ser Leu Pro Ser
        355                 360                 365

Ser Glu Arg Met Lys Leu Ser Gln Gly Asn Thr Thr Leu Ser Ile Asn
    370                 375                 380

Pro Val Lys Arg Glu Asp Ala Gly Thr Tyr Trp Cys Glu Val Phe Asn
385                 390                 395                 400

Pro Ile Ser Lys Asn Gln Ser Asp Pro Ile Met Leu Asn Val Asn Tyr
                405                 410                 415

Asn Ala Leu Pro Gln Glu Asn Gly Leu Ser Pro Gly Ala Ile Ala Gly
            420                 425                 430

Ile Val Ile Gly Val Val Ala Leu Val Ala Leu Ile Ala Val Ala Leu
        435                 440                 445

Ala Cys Phe Leu His Phe Gly Lys Thr Gly Arg Ala Ser Asp Gln Arg
    450                 455                 460
```

Asp Leu Thr Glu His Lys Pro Ser Val Ser Asn His Thr Gln Asp His
465 470 475 480

Ser Asn Asp Pro Pro Asn Lys Met Asn Glu Val Thr Tyr Ser Thr Leu
485 490 495

Asn Phe Glu Ala Gln Gln Pro Thr Gln Pro Thr Ser Ala Ser Pro Ser
500 505 510

Leu Thr Ala Thr Glu Ile Ile Tyr Ser Glu Val Lys Lys Gln
515 520 525

<210> 13
<211> 3444
<212> DNA
<213> Homo sapiens

<400> 13
aggctcagca cagagagtgg aaaacagcag aggtgacaga gcagccgtgc tcgaagcgtt 60
cctggagccc aagctctcct ccacaggtga agacagggcc agcaggagac accatggggc 120
acctctcagc cccacttcac agagtgcgtg taccctggca ggggcttctg ctcacagcct 180
cacttctaac cttctggaac ccgcccacca ctgcccagct cactactgaa tccatgccat 240
tcaatgttgc agaggggaag gaggttcttc tccttgtcca caatctgccc cagcaacttt 300
ttggctacag ctggtacaaa ggggaaagag tggatggcaa ccgtcaaatt gtaggatatg 360
caataggaac tcaacaagct accccagggc ccgcaaacag cggtcgagag acaatatacc 420
ccaatgcatc cctgctgatc cagaacgtca cccagaatga cacaggattc tacaccctac 480
aagtcataaa gtcagatctt gtgaatgaag aagcaactgg acagttccat gtatacccgg 540
agctgcccaa gccctccatc tccagcaaca actccaaccc tgtggaggac aaggatgctg 600
tggccttcac ctgtgaacct gagactcagg acacaaccta cctgtggtgg ataaacaatc 660
agagcctccc ggtcagtccc aggctgcagc tgtccaatgg caacaggacc ctcactctac 720
tcagtgtcac aaggaatgac acaggaccct atgagtgtga aatacagaac ccagtgagtg 780
cgaaccgcag tgacccagtc accttgaatg tcacctatgg cccggacacc cccaccattt 840
ccccttcaga cacctattac cgtccagggg caaacctcag cctctcctgc tatgcagcct 900
ctaacccacc tgcacagtac tcctggctta tcaatggaac attccagcaa agcacacaag 960
agctctttat ccctaacatc actgtgaata atagtggatc ctatacctgc cacgccaata 1020
actcagtcac tggctgcaac aggaccacag tcaagacgat catagtcact gagctaagtc 1080
cagtagtagc aaagccccaa atcaaagcca gcaagaccac agtcacagga gataaggact 1140
ctgtgaacct gacctgctcc acaaatgaca ctggaatctc catccgttgg ttcttcaaaa 1200
accagagtct cccgtcctcg gagaggatga agctgtccca gggcaacacc accctcagca 1260
taaaccctgt caagagggag gatgctggga cgtattggtg tgaggtcttc aacccaatca 1320
gtaagaacca aagcgacccc atcatgctga acgtaaacta taatgctcta ccacaagaaa 1380

```
atggcctctc acctggggcc attgctggca ttgtgattgg agtagtggcc ctggttgctc   1440
tgatagcagt agccctggca tgttttctgc atttcgggaa gaccggcagc tcaggaccac   1500
tccaatgacc cacctaacaa gatgaatgaa gttacttatt ctaccctgaa ctttgaagcc   1560
cagcaaccca cacaaccaac ttcagcctcc ccatccctaa cagccacaga aataatttat   1620
tcagaagtaa aaaagcagta atgaaacctg tcctgctcac tgcagtgctg atgtatttca   1680
agtctctcac cctcatcact aggagattcc tttcccctgt aggggtagag gggtggggac   1740
agaaacaact ttctcctact cttccttcct aataggcatc tccaggctgc ctggtcactg   1800
cccctctctc agtgtcaata gatgaaagta cattgggagt ctgtaggaaa cccaaccttc   1860
ttgtcattga aatttggcaa agctgacttt gggaaagagg gaccagaact tcccctccct   1920
tccccttttc ccaacctgga cttgttttaa acttgcctgt tcagagcact cattccttcc   1980
cacccccagt cctgtcctat cactctaatt cggatttgcc atagccttga ggttatgtcc   2040
ttttccatta agtacatgtg ccaggaaaca agagagagag aaagtaaagg cagtaatgcc   2100
ttctcctatt tctccaaagc cttgtgtgaa ctcaccaaac acaagaaaat caaatatata   2160
accaatagtg aaatgccaca cctttgtcca ctgtcagggt tgtctacctg taggatcagg   2220
gtctaagcac cttggtgctt agctagaata ccacctaatc cttctggcaa gcctgtcttc   2280
agagaaccca ctagaagcaa ctaggaaaat cacttgccaa aatccaaggc aattcctgat   2340
ggaaaatgca aaagcacata tatgttttaa tatctttatg ggctctgttc aaggcagtgc   2400
tgagagggag gggttatagc ttcaggaggg aaccagcttc tgataaacac aatctgctag   2460
gaacttggga aaggaatcag agagctgccc ttcagcgatt atttaaatta ttgttaaaga   2520
atacacaatt tggggtattg ggatttttct ccttttctct gagacattcc accattttaa   2580
tttttgtaac tgcttattta tgtgaaaagg gttatttttta cttagcttag ctatgtcagc   2640
caatccgatt gccttaggtg aaagaaacca ccgaaatccc tcaggtccct tggtcaggag   2700
cctctcaaga tttttttgt cagaggctcc aaatagaaaa taagaaaagg ttttcttcat   2760
tcatggctag agctagattt aactcagttt ctaggcacct cagaccaatc atcaactacc   2820
attctattcc atgtttgcac ctgtgcattt tctgtttgcc cccattcact ttgtcaggaa   2880
accttggcct ctgctaaggt gtatttggtc cttgagaagt gggagcaccc tacagggaca   2940
ctatcactca tgctggtggc attgtttaca gctagaaagc tgcactggtg ctaatgcccc   3000
ttggggaaat ggggctgtga ggaggaggat tataacttag gcctagcctc ttttaacagc   3060
ctctgaaatt tatcttttct tctatggggt ctataaatgt atcttataat aaaaaggaag   3120
gacaggagga agacaggcaa atgtacttct cacccagtct tctacacaga tggaatctct   3180
ttggggctaa gagaaaggtt ttattctata ttgcttacct gatctcatgt taggcctaag   3240
aggctttctc caggaggatt agcttggagt tctctatact caggtacctc tttcagggtt   3300
ttctaaccct gacacggact gtgcatactt tccctcatcc atgctgtgct gtgttattta   3360
atttttcctg gctaagatca tgtctgaatt atgtatgaaa attattctat gtttttataa   3420
taaaaataat atatcagaca tcga                                          3444
```

<210> 14
<211> 464
<212> PRT
<213> Homo sapiens

<400> 14

```
Met Gly His Leu Ser Ala Pro Leu His Arg Val Arg Val Pro Trp Gln
1               5                   10                  15

Gly Leu Leu Leu Thr Ala Ser Leu Leu Thr Phe Trp Asn Pro Pro Thr
            20                  25                  30

Thr Ala Gln Leu Thr Thr Glu Ser Met Pro Phe Asn Val Ala Glu Gly
        35                  40                  45

Lys Glu Val Leu Leu Leu Val His Asn Leu Pro Gln Gln Leu Phe Gly
    50                  55                  60

Tyr Ser Trp Tyr Lys Gly Glu Arg Val Asp Gly Asn Arg Gln Ile Val
65                  70                  75                  80

Gly Tyr Ala Ile Gly Thr Gln Gln Ala Thr Pro Gly Pro Ala Asn Ser
                85                  90                  95

Gly Arg Glu Thr Ile Tyr Pro Asn Ala Ser Leu Leu Ile Gln Asn Val
            100                 105                 110

Thr Gln Asn Asp Thr Gly Phe Tyr Thr Leu Gln Val Ile Lys Ser Asp
        115                 120                 125

Leu Val Asn Glu Glu Ala Thr Gly Gln Phe His Val Tyr Pro Glu Leu
    130                 135                 140

Pro Lys Pro Ser Ile Ser Ser Asn Asn Ser Asn Pro Val Glu Asp Lys
145                 150                 155                 160

Asp Ala Val Ala Phe Thr Cys Glu Pro Glu Thr Gln Asp Thr Thr Tyr
                165                 170                 175

Leu Trp Trp Ile Asn Asn Gln Ser Leu Pro Val Ser Pro Arg Leu Gln
            180                 185                 190

Leu Ser Asn Gly Asn Arg Thr Leu Thr Leu Leu Ser Val Thr Arg Asn
        195                 200                 205

Asp Thr Gly Pro Tyr Glu Cys Glu Ile Gln Asn Pro Val Ser Ala Asn
    210                 215                 220

Arg Ser Asp Pro Val Thr Leu Asn Val Thr Tyr Gly Pro Asp Thr Pro
225                 230                 235                 240
```

```
Thr Ile Ser Pro Ser Asp Thr Tyr Tyr Arg Pro Gly Ala Asn Leu Ser
                245                 250                 255

Leu Ser Cys Tyr Ala Ala Ser Asn Pro Pro Ala Gln Tyr Ser Trp Leu
            260                 265                 270

Ile Asn Gly Thr Phe Gln Gln Ser Thr Gln Glu Leu Phe Ile Pro Asn
        275                 280                 285

Ile Thr Val Asn Asn Ser Gly Ser Tyr Thr Cys His Ala Asn Asn Ser
    290                 295                 300

Val Thr Gly Cys Asn Arg Thr Thr Val Lys Thr Ile Ile Val Thr Glu
305                 310                 315                 320

Leu Ser Pro Val Val Ala Lys Pro Gln Ile Lys Ala Ser Lys Thr Thr
                325                 330                 335

Val Thr Gly Asp Lys Asp Ser Val Asn Leu Thr Cys Ser Thr Asn Asp
            340                 345                 350

Thr Gly Ile Ser Ile Arg Trp Phe Phe Lys Asn Gln Ser Leu Pro Ser
        355                 360                 365

Ser Glu Arg Met Lys Leu Ser Gln Gly Asn Thr Thr Leu Ser Ile Asn
    370                 375                 380

Pro Val Lys Arg Glu Asp Ala Gly Thr Tyr Trp Cys Glu Val Phe Asn
385                 390                 395                 400

Pro Ile Ser Lys Asn Gln Ser Asp Pro Ile Met Leu Asn Val Asn Tyr
                405                 410                 415

Asn Ala Leu Pro Gln Glu Asn Gly Leu Ser Pro Gly Ala Ile Ala Gly
            420                 425                 430

Ile Val Ile Gly Val Val Ala Leu Val Ala Leu Ile Ala Val Ala Leu
        435                 440                 445

Ala Cys Phe Leu His Phe Gly Lys Thr Gly Ser Ser Gly Pro Leu Gln
    450                 455                 460

<210>  15
<211>  3888
<212>  DNA
<213>  Homo sapiens

<400>  15
aaaactcagg gaagcccagg gcccgtgttg tgcttttggc ccaggtaggt ggacagacat      60

gtcccaggcg ggggacgtag aaggccccag cacaggagac cctgtgctca gtccccaaca     120

caactgtgag cttttacaga acatggaagg agccagctcc atgccaggcc tgtcaccaga     180

tgggccggga gcaagctctg ggcccggagt cagggctggc agcagaagga agatccccag     240
```

```
gaaggaggcc cttcgaggtg gcagctcccg ggctgcaggt gctgctgagg tccggccagg     300
ggtcttggag ctgctagctg tggtacagag ccggggctcg atgctggctc ctgggctcca     360
catgcagctg ccctcggtgc ctactcaggg gagagctctg acctccaaga ggctccaggt     420
ttctctgtgt gacatcttag atgacagttg ccccaggaaa ctttgtagca ggtctgctgg     480
cctcccagag agagctctgg cctgcaggga gaggcttgca ggagtggagg aggtgagctg     540
cctcaggccc agggaggcca gagacggtgg aatgagttct ccagggtgtg acagaagaag     600
ccccacactc agcaaagagg agccccctgg aaggcccctg acatcctcac cagacccagt     660
ccctgtgagg gtaagaaaga aatggaggag gcaaggggct cattcagagt gtgaggaagg     720
ggctggtgac ttcctgtggc ttgatcagag ccctcgtggg gacaacctcc tgtctgtggg     780
agaccctccc caagttgctg acctggagtc cttgggaggc ccttgcagac ctccctctcc     840
aaaagacact gggtctgggc ctggagagcc aggtggaagt ggggcaggat gtgcctcagg     900
gactgagaaa tttggatatt tgcccgctac aggggatggg ccccagccag gcagcccctg     960
tggccctgtc gggttcccag tgcccagtgg aggggagtcc ctcagttcag ctgcacaggc    1020
tcctccacag agcgcagcac tgtgcctggg ggcgtcagca caggcctctg cagagcagca    1080
agaagctgtg tgtgtcgtgc ggactggcag cgatgaaggc caggctccag cacaggacca    1140
ggaggagctg gaggccaagg ctcagccagc ttccagggga aggctggagc aaggactcgc    1200
tgcccccgct gacacctgtg ccagctcccg ggagcccttg ggcggcctca gctcctccct    1260
ggatactgaa gccagcaggg cctgctcagg cccattcatg gagcagagaa gatccaaggg    1320
cactaagaac ctgaagaaag gtccagtgcc ctgtgcccaa gaccggggca cagacagaag    1380
ctcagacaac tcccaccagg acaggccaga ggaacccagc ccaggaggct gccccagact    1440
ggaggaagtg aaaatacccc atggagtgaa gcttgtgtgc tacctgggtt ccgggccagt    1500
gatccagctc ctgggggcca tcagccacgg ccaggcaggg gggcagctgc caccaaagct    1560
ggaggttcta gaggacttga tggaggtcag ctcaccctca cctgcccaga ggctcagaag    1620
gaagaaaagg cccatggtgc agggccctgc tgggtgccag gttttccagc cttctccttc    1680
aggaggcaca gcaggggacc ctggtggcct ctctgacccc ttctaccctc caagaagcgg    1740
ttccctggcc cttggcgacc ccagctcgga ccctgcatgt tcccagagtg gcccaatgga    1800
ggctgaagag gattctcttc cggagcagcc agaggactca gctcagctcc aacaggagaa    1860
gccatccctg tatattgggg tgcggggcac tgttgtccgt tccatgcagg aggtactatg    1920
gactcgcctt cgggagctcc cagacccagt gctgagtgag gaggtggtgg agggcattgc    1980
tgctggcatt gaggcagccc tctgggacct gacacaaggc accaatggcc ggtacaagac    2040
caagtatcgc agcctgctgt tcaacctgcg ggaccccagg aacctggact tgtttctcaa    2100
agtggttcat ggagatgtca ccccctacga cctggtgcgg atgagctcga tgcagctggc    2160
cccccaggag ctggcccgct ggcgggacca ggaggagaaa aggggcctga atatcattga    2220
gcagcaacag aaggagccgt gcagacttcc agcctccaaa atgacccaca agggcgaagt    2280
```

ggagattcag cgggacatgg accagacact gaccctggag gatctggtgg gaccgcagat 2340

gttcatggac tgcagcccac aggccctgcc catcgcatca gaggacacca cggggcagca 2400

tgaccaccac ttcttagacc ccaactgcca catctgcaag gactgggagc cctcgaatga 2460

gctgctaggc tccttcgaag ccgccaagag ctgcggggac aatatcttcc agaaagccct 2520

aagccaaact cctatgcctg ctccagagat gcccaaaacc agggagttgt ctcccacgga 2580

accacaggac agggtccctc catctgggct ccatgtgcct gctgcaccca caaaggccct 2640

gccctgcctg ccaccctggg aaggtgttct ggacatgttc tccatcaagc ggttccgggc 2700

cagggcccag ctggtctcgg gacacagctg tcggcttgtc caggctctgc ccaccgtgat 2760

ccgctcggca ggctgcatcc cctccaacat tgtctgggac cttctggcca gcatctgccc 2820

agccaaggcc aaggacgtct gcgtggtcag actgtgccca catggggccc gggacaccca 2880

gaactgccgc ctgctctact catacctcaa tgataggcag cgccacgggc tggcctctgt 2940

ggagcacatg gggatggtcc tgctgcccct gcctgccttc cagcccctgc ccaccaggct 3000

gcgccctttg gggggcccag gcctttgggc tcttcctgtc tcccctctcc tttccccagg 3060

tctggaggtc actcactcaa gtctgttgct ggctgtgctg ctccccaagg aagggcttcc 3120

agacacagca gggtccagcc cctggttggg gaaggttcaa aagatggtct ccttcaacag 3180

taaggtggag aagagatact atcagccaga tgacaggagg ccgaatgtgc ccctgaaggg 3240

cacccctccc ccaggaggtg cctggcagca gagccagggc aggggcagta tagctccaag 3300

gggaatctct gcttggcaga ggccccccag aggcaggggg aggctctggc cagagcctga 3360

aaactggcag catcctgggc gagggcagtg gcccccagag ccaggcttgc gccagtccca 3420

gcatccctat tcagtagcac cagctggtca tggctttggc cgtggccagc acttccacag 3480

ggactcctgt ccccaccaag ccctgctccg gcacctcgaa tccctggcga ccatgagtca 3540

ccagctccaa gccttactgt gcccccagac caagagctcc atcccccgcc ctctgcagcg 3600

tttgtctagc gcccttgcag ctccagagcc ccctggccca gcccgtgact cctctttggg 3660

gcctacagat gaagctggct ctgagtgtcc cttccctaga aaggcctgac cctccttacc 3720

caccagaaca ggggttttga tgccctcact agtgttgaag cctgttccag agagaggtgg 3780

gactgcaagg agaggatggt cagccctacc cacctgccct gtttgagctt cctgtttgac 3840

aatgtttgct gttgattttt tgttcaataa agaatttggt aaaattgg 3888

<210> 16
<211> 1216
<212> PRT
<213> Homo sapiens

<400> 16

Met Ser Gln Ala Gly Asp Val Glu Gly Pro Ser Thr Gly Asp Pro Val
1 5 10 15

Leu Ser Pro Gln His Asn Cys Glu Leu Leu Gln Asn Met Glu Gly Ala
20 25 30

```
Ser Ser Met Pro Gly Leu Ser Pro Asp Gly Pro Gly Ala Ser Ser Gly
        35                  40                  45

Pro Gly Val Arg Ala Gly Ser Arg Arg Lys Ile Pro Arg Lys Glu Ala
    50                  55                  60

Leu Arg Gly Gly Ser Ser Arg Ala Ala Gly Ala Ala Glu Val Arg Pro
65                  70                  75                  80

Gly Val Leu Glu Leu Leu Ala Val Val Gln Ser Arg Gly Ser Met Leu
                85                  90                  95

Ala Pro Gly Leu His Met Gln Leu Pro Ser Val Pro Thr Gln Gly Arg
            100                 105                 110

Ala Leu Thr Ser Lys Arg Leu Gln Val Ser Leu Cys Asp Ile Leu Asp
        115                 120                 125

Asp Ser Cys Pro Arg Lys Leu Cys Ser Arg Ser Ala Gly Leu Pro Glu
    130                 135                 140

Arg Ala Leu Ala Cys Arg Glu Arg Leu Ala Gly Val Glu Glu Val Ser
145                 150                 155                 160

Cys Leu Arg Pro Arg Glu Ala Arg Asp Gly Gly Met Ser Ser Pro Gly
                165                 170                 175

Cys Asp Arg Arg Ser Pro Thr Leu Ser Lys Glu Glu Pro Pro Gly Arg
            180                 185                 190

Pro Leu Thr Ser Ser Pro Asp Pro Val Pro Val Arg Val Arg Lys Lys
        195                 200                 205

Trp Arg Arg Gln Gly Ala His Ser Glu Cys Glu Glu Gly Ala Gly Asp
    210                 215                 220

Phe Leu Trp Leu Asp Gln Ser Pro Arg Gly Asp Asn Leu Leu Ser Val
225                 230                 235                 240

Gly Asp Pro Pro Gln Val Ala Asp Leu Glu Ser Leu Gly Gly Pro Cys
                245                 250                 255

Arg Pro Pro Ser Pro Lys Asp Thr Gly Ser Gly Pro Gly Glu Pro Gly
            260                 265                 270

Gly Ser Gly Ala Gly Cys Ala Ser Gly Thr Glu Lys Phe Gly Tyr Leu
        275                 280                 285

Pro Ala Thr Gly Asp Gly Pro Gln Pro Gly Ser Pro Cys Gly Pro Val
    290                 295                 300
```

Gly Phe Pro Val Pro Ser Gly Gly Glu Ser Leu Ser Ser Ala Ala Gln
305                 310                 315                 320

Ala Pro Pro Gln Ser Ala Ala Leu Cys Leu Gly Ala Ser Ala Gln Ala
                325                 330                 335

Ser Ala Glu Gln Gln Glu Ala Val Cys Val Val Arg Thr Gly Ser Asp
            340                 345                 350

Glu Gly Gln Ala Pro Ala Gln Asp Gln Glu Glu Leu Glu Ala Lys Ala
        355                 360                 365

Gln Pro Ala Ser Arg Gly Arg Leu Glu Gln Gly Leu Ala Ala Pro Ala
    370                 375                 380

Asp Thr Cys Ala Ser Ser Arg Glu Pro Leu Gly Gly Leu Ser Ser Ser
385                 390                 395                 400

Leu Asp Thr Glu Ala Ser Arg Ala Cys Ser Gly Pro Phe Met Glu Gln
                405                 410                 415

Arg Arg Ser Lys Gly Thr Lys Asn Leu Lys Lys Gly Pro Val Pro Cys
            420                 425                 430

Ala Gln Asp Arg Gly Thr Asp Arg Ser Ser Asp Asn Ser His Gln Asp
        435                 440                 445

Arg Pro Glu Glu Pro Ser Pro Gly Gly Cys Pro Arg Leu Glu Glu Val
    450                 455                 460

Lys Ile Pro His Gly Val Lys Leu Val Cys Tyr Leu Gly Ser Gly Pro
465                 470                 475                 480

Val Ile Gln Leu Leu Gly Ala Ile Ser His Gly Gln Ala Gly Gly Gln
                485                 490                 495

Leu Pro Pro Lys Leu Glu Val Leu Glu Asp Leu Met Glu Val Ser Ser
            500                 505                 510

Pro Ser Pro Ala Gln Arg Leu Arg Arg Lys Lys Arg Pro Met Val Gln
        515                 520                 525

Gly Pro Ala Gly Cys Gln Val Phe Gln Pro Ser Pro Ser Gly Gly Thr
    530                 535                 540

Ala Gly Asp Pro Gly Gly Leu Ser Asp Pro Phe Tyr Pro Pro Arg Ser
545                 550                 555                 560

Gly Ser Leu Ala Leu Gly Asp Pro Ser Ser Asp Pro Ala Cys Ser Gln
                565                 570                 575

Ser Gly Pro Met Glu Ala Glu Glu Asp Ser Leu Pro Glu Gln Pro Glu

```
            580                 585                 590

Asp Ser Ala Gln Leu Gln Gln Glu Lys Pro Ser Leu Tyr Ile Gly Val
        595                 600                 605

Arg Gly Thr Val Val Arg Ser Met Gln Glu Val Leu Trp Thr Arg Leu
    610                 615                 620

Arg Glu Leu Pro Asp Pro Val Leu Ser Glu Glu Val Val Glu Gly Ile
625                 630                 635                 640

Ala Ala Gly Ile Glu Ala Ala Leu Trp Asp Leu Thr Gln Gly Thr Asn
                645                 650                 655

Gly Arg Tyr Lys Thr Lys Tyr Arg Ser Leu Leu Phe Asn Leu Arg Asp
            660                 665                 670

Pro Arg Asn Leu Asp Leu Phe Leu Lys Val Val His Gly Asp Val Thr
        675                 680                 685

Pro Tyr Asp Leu Val Arg Met Ser Ser Met Gln Leu Ala Pro Gln Glu
    690                 695                 700

Leu Ala Arg Trp Arg Asp Gln Glu Glu Lys Arg Gly Leu Asn Ile Ile
705                 710                 715                 720

Glu Gln Gln Gln Lys Glu Pro Cys Arg Leu Pro Ala Ser Lys Met Thr
                725                 730                 735

His Lys Gly Glu Val Glu Ile Gln Arg Asp Met Asp Gln Thr Leu Thr
            740                 745                 750

Leu Glu Asp Leu Val Gly Pro Gln Met Phe Met Asp Cys Ser Pro Gln
        755                 760                 765

Ala Leu Pro Ile Ala Ser Glu Asp Thr Thr Gly Gln His Asp His His
    770                 775                 780

Phe Leu Asp Pro Asn Cys His Ile Cys Lys Asp Trp Glu Pro Ser Asn
785                 790                 795                 800

Glu Leu Leu Gly Ser Phe Glu Ala Ala Lys Ser Cys Gly Asp Asn Ile
                805                 810                 815

Phe Gln Lys Ala Leu Ser Gln Thr Pro Met Pro Ala Pro Glu Met Pro
            820                 825                 830

Lys Thr Arg Glu Leu Ser Pro Thr Glu Pro Gln Asp Arg Val Pro Pro
        835                 840                 845

Ser Gly Leu His Val Pro Ala Ala Pro Thr Lys Ala Leu Pro Cys Leu
    850                 855                 860
```

```
Pro Pro Trp Glu Gly Val Leu Asp Met Phe Ser Ile Lys Arg Phe Arg
865                 870                 875                 880

Ala Arg Ala Gln Leu Val Ser Gly His Ser Cys Arg Leu Val Gln Ala
                885                 890                 895

Leu Pro Thr Val Ile Arg Ser Ala Gly Cys Ile Pro Ser Asn Ile Val
            900                 905                 910

Trp Asp Leu Leu Ala Ser Ile Cys Pro Ala Lys Ala Lys Asp Val Cys
        915                 920                 925

Val Val Arg Leu Cys Pro His Gly Ala Arg Asp Thr Gln Asn Cys Arg
    930                 935                 940

Leu Leu Tyr Ser Tyr Leu Asn Asp Arg Gln Arg His Gly Leu Ala Ser
945                 950                 955                 960

Val Glu His Met Gly Met Val Leu Leu Pro Leu Pro Ala Phe Gln Pro
                965                 970                 975

Leu Pro Thr Arg Leu Arg Pro Leu Gly Gly Pro Gly Leu Trp Ala Leu
            980                 985                 990

Pro Val Ser Pro Leu Leu Ser Pro  Gly Leu Glu Val Thr  His Ser Ser
        995                 1000                 1005

Leu Leu  Leu Ala Val Leu Leu  Pro Lys Glu Gly Leu  Pro Asp Thr
    1010                 1015                 1020

Ala Gly  Ser Ser Pro Trp Leu  Gly Lys Val Gln Lys  Met Val Ser
    1025                 1030                 1035

Phe Asn  Ser Lys Val Glu Lys  Arg Tyr Tyr Gln Pro  Asp Asp Arg
    1040                 1045                 1050

Arg Pro  Asn Val Pro Leu Lys  Gly Thr Pro Pro Pro  Gly Gly Ala
    1055                 1060                 1065

Trp Gln  Gln Ser Gln Gly Arg  Gly Ser Ile Ala Pro  Arg Gly Ile
    1070                 1075                 1080

Ser Ala  Trp Gln Arg Pro Pro  Arg Gly Arg Gly Arg  Leu Trp Pro
    1085                 1090                 1095

Glu Pro  Glu Asn Trp Gln His  Pro Gly Arg Gly Gln  Trp Pro Pro
    1100                 1105                 1110

Glu Pro  Gly Leu Arg Gln Ser  Gln His Pro Tyr Ser  Val Ala Pro
    1115                 1120                 1125
```

```
Ala Gly  His Gly Phe Gly Arg  Gly Gln His Phe His  Arg Asp Ser
    1130                 1135                 1140

Cys Pro  His Gln Ala Leu Leu  Arg His Leu Glu Ser  Leu Ala Thr
    1145                 1150                 1155

Met Ser  His Gln Leu Gln Ala  Leu Leu Cys Pro Gln  Thr Lys Ser
    1160                 1165                 1170

Ser Ile  Pro Arg Pro Leu Gln  Arg Leu Ser Ser Ala  Leu Ala Ala
    1175                 1180                 1185

Pro Glu  Pro Pro Gly Pro Ala  Arg Asp Ser Ser Leu  Gly Pro Thr
    1190                 1195                 1200

Asp Glu  Ala Gly Ser Glu Cys  Pro Phe Pro Arg Lys  Ala
    1205                 1210                 1215
```

## Description

The present invention is concerned with diagnostic means and methods for determining the metastasising potential of solid tumors. More specifically, the present invention relates to a method of diagnosing whether a solid tumor in a subject has metastasising potential comprising the steps of determining in a sample of a subject suspected to suffer from a solid tumor the expression levels of the marker genes GPR109B, DHRS9, and ERRFI-1 and the expression level of a reference gene, calculating at least one variable from the reference gene and one or more of said marker genes and comparing variable to a reference variable, whereby it is to be diagnosed whether the solid tumor has metastasising potential. Moreover, the present invention relates to a method of determining whether a therapy against a solid tumor having metastasising potential is successful. Further contemplated are a solid support, diagnostic device and a kit for carrying out the aforementioned methods.

Metastasis is a hallmark of malignancy of tumors. It refers to a process wherein tumor cells migrate from the primary tumor tissue into other tissues where they settle down and form secondary tumors. Most solid tumors, at later stages, develop the capability of generating metastases.

The secondary tumors which arise from the metastasising cells of the primary tumor most often are even more life-threatening than the primary tumor itself. Moreover, primary solid tumors usually can be treated very well by local therapeutic approaches such as surgery or local irradiation. Such local therapies are less harmful for the organism than systemic therapies such as chemotherapy.

The current method of assessing the risk of a tumor for formation of metastases is usually the assessment of said tumor with an internationally unified tumor staging system (TMN). Based on features of the individual tumor such as its size or the invasion of regional lymph nodes each tumor is assigned a stage within the system. The risk of the tumor for the formation of metastases is then estimated based on the stage it has reached. This method does not allow an individual prognosis for a patient because the TMN system cannot differentiate the metastasising potentials of tumors of the same stage. However, there is a clear need for determining the metastasising potential of individual tumors of the same stage.

The technical problem underlying the present invention can be seen as the provision of means and methods for complying with the aforementioned needs. The technical problem is solved by the embodiments characterized in the claims and herein below.

Accordingly, the present invention relates to a method of diagnosing whether a solid tumor in a subject has metastasising potential comprising the steps of:

(a) determining in a sample of a subject suspected to suffer from a solid tumor the expression levels of the marker genes GPR109B, DHRS9, and ERRFI-1 and the expression level of a reference gene, and
(b) calculating at least one variable from one or more of said marker genes and the reference gene; and
(c) comparing the variable calculated in step b) to a reference variable, whereby it is to be diagnosed whether the solid tumor has metastasising potential.

The method referred to above may comprise further steps such as sample pre-treatment steps or evaluation steps, e.g., steps concerning the recommendation of suitable therapies by including an automated, computer-based expert system. It is to be understood that the method is to be carried out in vitro, i.e. on a sample of a subject rather than on the subject itself. The steps of the method may be assisted by automation. For example, the determination of the expression levels may be assisted by microarrays and robotics. The step of calculating the variable and comparing said variable to a suitable reference could be assisted by a computer implemented algorithm for data comparison.

The term "diagnosing" as used herein refers to assessing the probability according to which a solid tumor in a subject has metastasising potential. As will be understood by those skilled in the art, such an assessment is usually not intended to be correct for 100% of the subjects to be diagnosed. The term, however, requires that a statistically significant portion of subjects can be correctly diagnosed. Whether a portion is statistically significant can be determined without further ado by the person skilled in the art using various well known statistic evaluation tools, e.g., determination of confidence intervals, p-value determination, Student's t-test, Mann-Whitney test etc.. Details are found in the standard text book on statistics Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001. Preferably, the probability envisaged by the present invention allows that the diagnosis will be correct for at least 60%, at least 70%, at least 80%, or at least 90% of the subjects of a given cohort or population.

The term "solid tumor" as used herein refers to a tumor which arises from or which develops in solid tissues. Such solid tumors, preferably, encompass sarcomas, germ cell tumors or blastic tumors. More preferably, a solid tumor as referred to herein is a carcinoma. Most preferably it is a carcinoma of the lung, pharynx, breast, stomach, pancreas or the colon.

The term "metastasising potential" refers to the capability of a tumor cell to migrate from the solid tumor into other tissues and to form metastases. The formation of metastases is a crucial process in tumor development since, as a result of the said process, the tumor will spread in other tissues of the organism and, thus, systemically affect the organism. Moreover, therapeutic approaches become limited to systemic approaches such as chemotherapy since the tumor can not be treated successfully any longer by local therapies such as surgery or local irradiation.

The term "subject" as used herein refers to an animal, preferably to a mammal, more preferably, to a primate and, most preferably, to a human.

The term "sample" refers to any tissue or body fluid sample comprising cells of the solid tumor. More preferably, the sample is a biopsy sample of the said solid tumor. Said biopsy sample, most preferably, essentially contains tumor cells only.

The term "GPR109B" refers to a gene encoding the G protein-coupled receptor 109B. This receptor is also called chemokine receptor HM74, HM74, PUMAG or PUMAG nicotinic acid receptor (Toulza 2007, Genome Biol. 8(6), R107; Soga 2003, Biochem Biophys Res Commun 303(1): 364-369; Wise 2003, J Biol Chem 278(11): 9869-9874; Tunaru 2003, Nat Med 9(3): 352-355; Irobi 2002, J Peripher Nerv Syt 7(2): 87-95; Zingoni 1997, Genomics 42(3): 519-523; Nomura 1993, Int Immunol 5(10): 1239-1249). The term encompasses the human GPR109B gene as well as variants thereof, preferably, homologs, orthologs and paralogs thereof. Transcripts of the human GPR109B gene are, preferably, polynucleotides having a nucleic acid sequence as shown in SEQ ID NO: 1 or polynucleotides encoding a GPR109B receptor polypeptide having an amino acid sequence as shown in SEQ ID NO: 2. Transcripts of variants of the aforementioned GPR109B gene as used herein, preferably, have nucleic acid sequences being at least 70%, at least 80%, at least 90%, at least 95%, at least 96%, at least 97%, at least 98% or at least 99% identical to the nucleic acid sequence shown in SEQ ID NO: 1 or encode polypeptides being at least 70%, at least 80%, at least 90%, at least 95%, at least 96%, at least 97%, at least 98% or at least 99% identical to the amino acid sequence shown in SEQ ID NO: 2. The degree of sequence identity between nucleic acid or amino acid sequences as referred to in this specification is, preferably calculated by the algorithms of Needleman and Wunsch or Smith and Waterman give particularly reliable results. To carry out the sequence alignments, the program PileUp (J. Mol. Evolution., 25, 351-360, 1987, Higgins et al., CABIOS, 5 1989: 151-153) or the programs Gap and BestFit [Needleman and Wunsch (J. Mol. Biol. 48; 443-453 (1970)) and Smith and Waterman (Adv. Appl. Math. 2; 482-489 (1981))], which are part of the GCG software packet [Genetics Computer Group, 575 Science Drive, Madison, Wisconsin, USA 53711 (1991)], are to be used. The sequence identity values recited above in percent (%) are to be determined, preferably, using the program GAP over the entire sequence region with the following settings: Gap Weight: 50, Length Weight: 3, Average Match: 10.000 and Average Mismatch: 0.000. Preferably, the variant genes referred to herein also encode GPR109B receptor polypeptides as specified above.

The term "DHRS9" refers to a gene encoding the dehydrogenase/reductase (SDR family) member 9, also called called retinol dehydrogenase L. (Jones 2007, J Biol Chem 282(11) 8317-24; Jette 2004, J Biol Chem 279(33): 34397-405; Clark 2003, Genome Res 13(10) 2265-2270; Markova 2003, Mol Genet Metab 78(2): 119-135; Soref 2001, J Biol Chem 276(26): 24194-202; Chetyrkin 2001, J Biol Chem 276(25): 22278-286; Haeseleer 2000, Meth Enzymol 316: 372-383). The term encompasses the human DHRS9 gene as well as variants thereof, preferably, homologs, orthologs and paralogs thereof. Transcripts of the human DHRS9 gene are, preferably, polynucleotides having a nucleic acid sequence as shown in SEQ ID NO: 3 or polynucleotides encoding a DHRS9 receptor polypeptide having an amino acid sequence as shown in SEQ ID NO: 4. Transcripts of variants of the aforementioned DHRS9 gene as used herein, preferably, have nucleic acid sequences being at least 70%, at least 80%, at least 90%, at least 95%, at least 96%, at least 97%, at least 98% or at least 99% identical to the nucleic acid sequence shown in SEQ ID NO: 3 or encode polypeptides being at least 70%, at least 80%, at least 90%, at least 95%, at least 96%, at least 97%, at least 98% or at least 99% identical to the amino acid sequence shown in SEQ ID NO: 4. Preferably, the variant genes referred to herein also encode DHRS9 polypeptides having dehydrogenase/reductase activity as specified above.

The term "ERRFI-1" refers to a gene encoding the ErbB receptor feedback inhibitor 1 polypeptide (Zhang 2007, Cell Cycle 6(5): 507-513; Gregory 2006, Nature 441/7091): 315-321; Anastasi 2005, Oncogene 24(28): 4540-4548; Hackel 2001, Biol Chem 382(12): 1649-62; Wick 1995, Exp. Cell Res 219(2): 527-535). The term encompasses the human ERRF-1 gene as well as variants thereof, preferably, homologs, orthologs and paralogs thereof. Transcripts of the human ERRFI-1 gene are, preferably, polynucleotides having a nucleic acid sequence as shown in SEQ ID NO: 5 or polynucleotides encoding a ERRFI-1 polypeptide having an amino acid sequence as shown in SEQ ID NO: 6. Transcripts of variants of the aforementioned ERRFI-1 gene as used herein, preferably, have nucleic acid sequences being at least 70%, at least 80%, at least 90%, at least 95%, at least 96%, at least 97%, at least 98% or at least 99% identical to the nucleic acid sequence shown in SEQ ID NO: 5 or encode polypeptides being at least 70%, at least 80%, at least 90%, at least 95%, at least 96%, at least 97%, at least 98% or at least 99% identical to the amino acid sequence shown in SEQ ID NO: 6. Preferably, the variant genes referred to herein also encode ERRFI-1 polypeptides having ERRFI-1 inhibitory activity as specified above.

The term "expression level" as used herein relates to any quantitative parameter representing either the amount of transcripts, i.e. RNA polynucleotides, of the marker genes or the amount or biological activity of polypeptides encoded by the marker genes which are present in the cells of the solid tumor present in the sample.

Accordingly, the expression level is, preferably, determined by determining the amount of transcribed polynucleotides of the marker genes present in the said sample (i.e. transcripts of the marker genes). Alternatively but also preferred, the expression level is determined by determining the amount or activity of polypeptides encoded by the marker genes present in the said sample.

Techniques for determining the amounts of transcribed polynucleotides present in a sample are well known in the art. Preferably, such techniques are based on nucleic acid hybridization and include, preferably, PCR-based techniques, Northern blots, in situ hybridization or the use of microarrays, e.g., as described elsewhere in this specification. The amounts of the transcribed polynucleotides are, thus, preferably determined by polynucleotide probes being capable of specifically binding to the transcribed polynucleotides of the marker genes or oligonucleotide primers capable of amplifying a specific sequence of a marker gene. Nucleic acid sequences for the marker genes from which the said polynucleotide probes or oligonucleotide primers can be derived are disclosed elsewhere in this specification. A polynucleotide probe which is capable of specifically binding to the polynucleotide transcript of a marker gene, preferably, hybridizes under stringent conditions with the polynucleotide transcript in the sample (i.e. an RNA polynucleotide) or a reverse transcribed cDNA obtained by using the polynucleotide transcript as a template. In the case of cDNA arrays the polynucleotide probes, preferably, comprise at 300, at least 500, or at least 800 contiguous nucleotides of the specific sequences disclosed elsewhere in the specification. An oligonucleotide primer as referred to herein is capable of amplifying a specific nucleic acid sequence of a marker gene. Preferably, such oligonucleotide primers essentially consists of 15 to 30, more preferably, 18 to 25, contiguous nucleotides of the specific sequences recited elsewhere herein. It is preferable to use at least 2, at least 4, at least 6, at least 8, at least 10 different oligonucleotide probes for each expressed sequence.

The amounts of polypeptides encoded by the marker genes referred to herein are, preferably, determined by antibody based techniques including affinity chromatography, Western blots or ELISA. Means for determining the amounts of the polypeptides encoded by the marker genes are, preferably, antibodies including antibody fragments, such as Fab, Fv or scFv fragments or a chemically modified derivative of any of these fragments, as well as ligands which specifically bind to the said polypeptides. Antibodies or ligands which specifically bind to the polypeptides are those which do not significantly cross-react with other polypeptides but bind to the polypeptides in detectable amounts. It is to be understood that the methods such as mass spectrometry (MS) which allow for a quantitative and/or qualitative determination of molecular species in general can be also applied for the determination of the amounts of polynucleotides being transcripts of the marker genes or the amounts of the polypeptides encoded by the marker genes.

Dependent on the polypeptide to be determined, a specific biological activity of the said polypeptide can be measured in order to determine the expression level. It is to be understood that the amount of activity of the polypeptides encoded by the marker genes directly correlates with the amount of polypeptides and, thus, is also an indicator of the expression level. Preferred methods for the determination of the polypeptides encoded by the marker genes are quantitative or semi-quantitative Western-blot, all antibody based methods or mass spectrometry.

The term "calculating at least one variable" refers to a mathematical operation whereby the expression levels of a reference gene and of at least one of the marker genes are used to calculate a value that indicates whether the tumor has metastasising potential or not. Preferably, the at least one variable is the ratio of the expression level of at least one marker gene and the expression level of the reference gene in the same patient at the same time. Also preferably, the expression levels of at least two marker genes are added first a ratio is calcultated, subsequentially, from said added expression levels and the reference gene expression level. It is to be understood that for any ratio to be calculated according to the method of the invention the reciprocal value gives the same information and can thus be used as well.

The term "reference gene" refers to a gene that is known as a part of the network that regulates the formation of metastases and is permanently up regulated for the duration of the metastasising process.

A preferred reference gene is PTGS-2. This gene encodes for the Cyclooxygenase-2. The term encompasses the human PTGS-2 gene as well as variants thereof, preferably, homologs, orthologs and paralogs thereof. Transcripts of the human PTGS-2 gene are, preferably, polynucleotides having a nucleic acid sequence as shown in SEQ ID NO: 7 or polynucleotides encoding a PTGS-2 polypeptide having an amino acid sequence as shown in SEQ ID NO: 8. Transcripts of variants of the aforementioned PTGS-2 gene as used herein, preferably, have nucleic acid sequences being at least 70%, at least 80%, at least 90%, at least 95%, at least 96%, at least 97%, at least 98% or at least 99% identical to the nucleic acid sequence shown in SEQ ID NO: 7 or encode polypeptides being at least 70%, at least 80%, at least 90%, at least 95%, at least 96%, at least 97%, at least 98% or at least 99% identical to the amino acid sequence shown in SEQ ID NO: 8.

Preferably, the variant genes referred to herein also encode PTGS-2 polypeptides having PTGS-2 inhibitory activity as specified above.

Another preferred reference gene is human EGR-3 (Patwardhan 1991, Oncogene 6: 917-928), encoding a 387 amino acid protein containing three zinc fingers. This gene is known to be induced in various brain regions in response to stress or following focal brain injury. The term "EGR-3" encompasses the human EGR-3 gene as well as variants thereof, preferably, homologs, orthologs and paralogs thereof. Transcripts of the human EGR-3 gene are, preferably, polynucleotides having a nucleic acid sequence as shown in SEQ ID NO: 9 or polynucleotides encoding a EGR-3 polypeptide having an amino acid sequence as shown in SEQ ID NO: 10. Transcripts of variants of the aforementioned EGR-3 gene as used herein, preferably, have nucleic acid sequences being at least 70%, at least 80%, at least 90%, at least 95%, at least 96%, at least 97%, at least 98% or at least 99% identical to the nucleic acid sequence shown in SEQ ID NO: 9 or encode polypeptides being at least 70%, at least 80%, at least 90%, at least 95%, at least 96%, at least 97%, at least 98% or at least 99% identical to the amino acid sequence shown in SEQ ID NO: 10. Preferably, the variant genes referred to herein also encode EGR-3 polypeptides having PTGS-2 inhibitory activity as specified above.

The term "reference variable" as used herein is a variable which allows allocating the calculated variable to either the group of variables accompanying solid tumors having metastasizing potential or to the group of variables accompanying solid tumors lacking metastasizing potential. Accordingly, suitable reference variables are, preferably, the variables determined in a sample of a subject known to not suffer from a solid tumor having metastasising potential. More preferably, variables being different from the reference variables are indicative for a subject suffering from a solid tumor having metastasising potential. Alternatively but also preferred suitable reference variables are the variables calculated in a subject known to suffer from a solid tumor having metastasising potential. More preferably, variables being essentially identical to the reference variables are indicative for a subject suffering from a solid tumor having metastasising potential. Most preferably, a reference variable being at least 1.5, 2.0, 2.5, 3.0 fold the variable determined in a sample of a subject which does not suffer from a solid tumor or which suffers from a solid tumor lacking metastasizing potential is indicative for a subject suffering from a solid tumor having metastasizing potential. Alternatively but also preferred, reference variables for subjects suffering from tumors with different metastasising potentials are obtained by the simulation of Example 1. Alternatively but also preferred, reference variables for subjects suffering from tumors with different metastasising potentials are obtained by the simulation of Example 1.

The term "comparing" as used herein refers to a comparison of a variable calculated in the previous step to a suitable reference, i.e. a reference variable. Comparison of the variables calculated according to the method of the present invention requires that only those variables are compared that have been calculated with the same algorithm. The comparison referred to in step (b) of the method of the present invention may be carried out manually or computer assisted. A computer program may further evaluate the result of the comparison, i.e. automatically provide the desired assessment in a suitable output format. Based on the comparison of the aforementioned amounts, it is possible to assess whether a solid tumor has metastasising potential, or not, as specified above in detail.

It has been found in the studies underlying the present invention that a variable calculated from the expression level of at least one of the aforementioned marker genes in combination with the expression level of a reference gene, advantageously, allows for a reliable determination of the metastasizing potential of solid tumors. Specifically, since the absolute expression levels of the marker genes may vary between individuals, a relative expression levels provided in accordance with the present invention, i.e. the at least one variable. Accordingly, thanks to the present invention an efficient therapy can be selected for a solid tumor, in particular, the necessity of a supplementary systemic therapy such as chemotherapy can be assessed. The harmful systemic therapies can be avoided for patients suffering from solid tumors without metastasizing potential. On the other hand, systemic therapies may be used supplementary in cases where a solid tumor has metastasizing potential, even as a precautionary measure only.

The explanations and definitions of the terms made herein above apply mutatis mutandis to the other embodiments of the present invention except if stated otherwise.

In a preferred embodiment of the method of the present invention, the expression level of the marker gene Ceacam-1 is further determined and used for the calculation of a variable.

The term "Ceacam-1" refers to a gene encoding the *Homo sapiens* carcinoembryonic antigen-related cell adhesion molecule 1 (biliary glycoprotein), transcript variant 1 (Klaile 2007, J Biol Chem 282(36): 26629-26640; Lee 2007, Infect Immun 75(9): 4449-4455; Hokari 2007, Life Sci 81(4): 336-345; N'Guessan 2007, J Infect Dis 195(11): 1651-1660; Yu 2006, J Biol Chem 281(51): 39179-39193; Thompson 1992, Genomics 12(4): 761-772; Kuroki 1991, Biochem Biophys Res Commun 176(2): 578-585; Barnett 1989, J Cell Biol 108(2): 267-276; Hinoda 1988, Proc Natl Acad Sci USA 85(18): 6959-6963; Svenberg 1979, Clin Exp Immunol 36(3):436-441). Transcript variant 1 (SEQ ID NO: 11) represents the longer transcript and encodes the longer isoform of the peptide (SEQ ID NO: 12). It is also known as transmembrane carcinoembryonic antigen BGPa, TM1-CEA, and CEACAM1-4L.

Transcript variant 2 lacks an exon in the 3' coding region that results in a frameshift and an early stop codon, compared to variant 1. The resulting protein (isoform 2, SEQ ID NO: 14) has a distinct C-terminus and is shorter than isoform 1 (SEQ ID NO: 12). This variant has been referred to by multiple names, including BGPc, transmembrane carcinoembryonic antigen 3, TM3-CEA, and CEACAM1-4S (Klaile 2007, J Biol Chem 282(36): 26629-26640; Lee 2007, Infect Immun 75(9): 4449-4455; Hokari 2007, Life Sci 81(4): 336-345; N'Guessan 2007, J Infect Dis 195(11): 1651-1660; Yu 2006, J Biol Chem 281(51): 39179-39193; Thompson 1992, Genomics 12(4): 761-772; Kuroki 1991, Biochem Biophys Res Commun 176(2): 578-585; Barnett 1989, J Cell Biol 108(2): 267-276; Hinoda 1988, Proc Natl Acad Sci USA 85(18): 6959-6963; Svenberg 1979, Clin Exp Immunol 36(3):436-441).

The term CEACAM-1 encompasses both transcripts of the human Cecam-1 gene as well as variants thereof, preferably, homologs, orthologs and paralogs thereof. Transcripts of the human Cecam-1 gene are, preferably, polynucleotides having a nucleic acid sequence as shown in SEQ ID NO: 11 or SEQ ID NO: 13 or polynucleotides encoding a Cecam-1 polypeptide having an amino acid sequence as shown in SEQ ID NO: 12 or SEQ ID NO: 14. Transcripts of variants of the aforementioned Cecam-1 gene as used herein, preferably, have nucleic acid sequences being at least 70%, at least 80%, at least 90%, at least 95%, at least 96%, at least 97%, at least 98% or at least 99% identical to the nucleic acid sequence shown in SEQ ID NO: 11 or SEQ ID NO: 13 or encode polypeptides being at least 70%, at least 80%, at least 90%, at least 95%, at least 96%, at least 97%, at least 98% or at least 99% identical to the amino acid sequence shown in SEQ ID NO: 12 or SEQ ID NO 14. Preferably, the variant genes referred to herein also encode Cecam-1 polypeptides having Cecam-1 activity as specified above. It has been found that the reliability of the method can be further improved by taken into account also Cecam-1 as a marker.

In another preferred embodiment of the method of the present invention, the expression level of the marker gene SPOCD1 is further determined and used for the calculation of a variable.
The term "SPOCD1" (SPEN paralog ond ortholog C-terminal domain) refers to a domain occurring in genes of the SPEN family of proteins. The SPEN family of proteins s a group of large proteins characterized by N-terminal RNA recognition motifs. The term encompasses the human SPOCD1 gene as well as variants thereof, preferably, homologs, orthologs and paralogs thereof. Transcripts of the human SPOCD1 gene are, preferably, polynucleotides having a nucleic acid sequence as shown in SEQ ID NO: 15 or polynucleotides encoding a SPOCD1 polypeptide having an amino acid sequence as shown in SEQ ID NO: 16. Transcripts of variants of the aforementioned SPOCD1 gene as used herein, preferably, have nucleic acid sequences being at least 70%, at least 80%, at least 90%, at least 95%, at least 96%, at least 97%, at least 98% or at least 99% identical to the nucleic acid sequence shown in SEQ ID NO: 15 or encode polypeptides being at least 70%, at least 80%, at least 90%, at least 95%, at least 96%, at least 97%, at least 98% or at least 99% identical to the amino acid sequence shown in SEQ ID NO: 16. Preferably, the variant genes referred to herein also encode SPOCD1 polypeptides having SPOCD1 activity as specified above. It has been found that the reliability of the method can be further improved by taken into account also SPOCD1 as a marker.

The present invention, furthermore, relates to a method of determining whether a therapy against a solid tumor having metastasising potential is successful comprising:

(a) determining in a first sample of a subject suffering from a solid tumor having metastasising potential obtained prior to the onset of the therapy the expression levels of GPR109B, DHRS9, ERRFI-1 and the reference gene,
(b) calculating a variable from one or more of said marker genes and the reference gene; and
(c) determining in a second sample of the said subject obtained after the onset of the therapy the variable of b); and
(d) comparing the variable calculated in step b) to the variable calculated in step c), wherein a difference of the variables is indicative for a successful therapy against a solid tumor having metastasising potential.

In accordance with the present invention, a therapy against a solid tumor is to be deemed "successful" if the tumor loses its metastasising potential. This is preferably achieved if the solid tumor does not develop metastases within a predictive window of at least 2, 3, 4, 5, 6, 7, 8, 9 or 10 years after the onset of the therapy. Moreover, tumor size, growth and/or progression after the therapy may also become significantly reduced compared to the tumor size, growth and/or progression prior to the therapy. Whether a reduction is statistically significant can be determined by techniques well known in the art and, e.g., described elsewhere in this specification. It is to be understood that a successful therapy could also result in removal of the solid tumor. Preferably, the solid tumor is removed to at least 80%, at least 90%, at least 95%, at least 98%, at least 99% or at least 100%.

The term "therapy against a solid tumor" refers to any drug-, irradiation- or surgery- based therapies applied against solid tumors. The term also includes therapeutic measures for prevention of metastasis for solid tumors known to be at risk of metastasis.

The term "difference" as used herein refers to a statistically significant difference between the variable in the first sample and the second sample. It is to be understood that the variable calculated in the first sample will be characteristic for a solid tumor having metastasising potential. Upon applying a successful therapy, the solid tumor will disappear or at least lose its metastasizing potential. Accordingly, the variables calculated from samples of successfully treated subjects shall at least reflect a solid tumor lacking metastasizing potential. Preferably, a decreasing difference between the variable calculated from the sample of a subject undergoing anti-tumor therapy and from a sample of a healthy subject indicates a successful therapy. Also preferably, a decreasing difference between the variable calculated from the sample of a subject undergoing anti-tumor therapy and from a sample of a subject with a tumor with a lower metastasizing potential indicates a successful therapy.

Advantageously, the aforementioned method allows for monitoring and evaluation of therapies applied in order to prevent formation of metastases. The method allows a rapid and reliable identification of therapies which are successful in this respect.

In a preferred embodiment of the aforementioned method, the expression level of the marker gene Ceacam-1 is further determined.

In another preferred embodiment of the aforementioned method, the expression level of the marker gene SPOCD1 is further determined.

The marker genes referred to above or means for determining their expression levels in a sample are, in principle, useful for diagnosing a solid tumor having metastasising potential in a subject or for the manufacture of a diagnostic composition for diagnosing a solid tumor having metastasising potential in a subject. In addition, they can be used for monitoring whether a therapy against a solid tumor having metastasising potential is successful or for the manufacture of a diagnostic composition for diagnosing whether a therapy against a solid tumor having metastasising potential is successful.

The term "means for determining expression levels" refers to a polynucleotide probe which is capable of specifically hybridizing to the polynucleotide transcript of a marker gene, oligonucleotide primers which are capable of amplifying a specific nucleic acid sequence of a marker gene, an antibody which specifically recognizes a polypeptide encoded by a marker gene or a ligand which specifically recognizes a polypeptide encoded by a marker gene as defined elsewhere in this specification.

The present invention also relates to a solid support comprising polynucleotides or polypeptides of the marker genes of the present invention or means for the determination of such polynucleotides or polypeptides.

A solid support as referred to herein is, preferably, a small membrane, e.g., a nylon membrane, or glass slide, containing samples of various immobilized polynucleotides or polypeptides or means for their determination (such as antibodies specifically recognizing the polypeptides) arranged in a regular pattern (i.e. a microarray or biochip). The solid support of the present invention can be used to determine the expression levels of the marker genes. This is achieved by contacting the solid support with the sample of the solid tumor. Polynucleotide transcripts comprised by the sample can be determined by a solid support comprising polynucleotides of the marker genes due to hybridization of the polynucleotide transcripts of the sample to the immobilized polynucleotides on the solid support. Successful hybridization can be detected by techniques well known in the art. Polypeptides encoded by the marker genes can be determined in a sample mutatis mutandis by a solid support comprising means for the detection of polypeptides such as antibodies or specific ligands.

The present invention also relates to a device adapted for carrying out the method of the present invention comprising

(a) means for determining the expression levels of the marker genes and the reference gene in a sample; and
(b) means for calculating a variable from one or more of said marker genes and the reference gene; and
(c) means for comparing the variable calculated by the means of b) with a reference variable.

The term "device" as used herein relates to a system comprising at least the aforementioned means operatively linked to each other as to allow carrying out the methods of the present invention. Preferred means for determining the expression levels as well as means for carrying out the calculation and comparison are disclosed above in connection with the method of the invention. How to link the means in an operating manner will depend on the type of means included into the device. For example, where means for automatically determining the expression levels are applied, the data obtained by said automatically operating means can be processed by, e.g., a computer program in order to obtain the desired results. Preferably, the means are comprised by a single device in such a case. Said device may accordingly include an analyzing unit for the measurement of the amount of the polypeptides in an applied sample and a computer unit for processing the resulting data for the evaluation. The computer unit, preferably, comprises a database including the stored references recited elsewhere in this specification as well as a computer-implemented algorithm for carrying out a comparison of the determined expression levels with the stored references of the database. Computer-implemented as used herein refers to a computer-readable program code tangibly embedded into the computer unit. The person skilled in the art will realize how to link the means without further ado. Preferred devices are those which can be applied without the particular knowledge of a specialized clinician, e.g., electronic devices which merely require loading with a sample. The results may be given as output of raw data which need interpretation by the clinician. Preferably, the output of the device is, however, processed, i.e. evaluated, raw data the interpretation of which does not require a clinician.

The present invention also relates to a kit comprising means for determining the expression levels of the maker genes or the solid support of the present invention.

The term "kit" as used herein refers to a collection of the aforementioned means or solid support, preferably, provided separately or within a single container. The components of the kit may be comprised by separate vials (i.e. as a kit of separate parts) or provided in a single vial. Moreover, it is to be understood that the kit of the present invention is to be used for practising the methods referred to herein above. It is, preferably, envisaged that all components are provided in a ready-to-use manner for practising the methods referred to above. Further, the kit preferably contains instructions for carrying out the said methods. The instructions can be provided by a user's manual in paper- or electronic form. For example, the manual may comprise instructions for interpreting the results obtained when carrying out the aforementioned methods using the kit of the present invention.

Further, the present invention also contemplates a method for the identification of a compound capable of inhibiting metastasis comprising the steps of:

(a) contacting a compound suspected to be capable of inhibiting metastasis to a cell of a solid tumor having meastasising potential;
(b) determining the expression levels of a reference gene and the marker genes GPR109B, DHRS9, and ERRFI-1 in the said cell; and
(c) calculating a variable from the epression levels of the reference gene and the marker genes, and
(d) comparing the variable calculated in step c) to references whereby a compound capable of inhibiting metastasis is identified.

Preferably, reference variables are expression levels of solid tumor cells lacking metastasising potential or non-tumor cells of the tissue from which the tumor originated. In said case, a variable calculated in step c) being essentially identical to the reference is indicative for a compound being capable of inhibiting metastasis. Alternatively, reference variables are variables determined in solid tumor cells having metastasising potential. In such a case, a variable calculated in step c) which significantly differs from the reference is indicative for a compound being capable of inhibiting metastasis. In a preferred embodiment of the aforementioned method, the expression level of the marker gene Ceacam-1 and/or SPOCD1 are further determined and in combination of a reference gene used for the calculation of a variable.

Also contemplated in the present invention is a method for the modeling of a biological process in a system comprising the steps of

a) measuring the amount of at least two suitable biological markers of the biological process of interest over a first time span;
b) fitting of equations to the measured amounts of a) and fitness evaluation;
c) simulation of the equations over second time span larger than the first time span;
d) elimination of equations that differ from a predetermined behavior over the second time span;
e) performing a robustness evaluation; and
f) performing a parameter evaluation.

The method of the present invention allows for the simulation of complex biological processes, because it reduces the complexity of the system for modeling purposes. This reduction of the complexity is based on the adiabatic approximation of the behavior of faster elements of the system. Thus, it is not necessary to model every single event in the biological process. The long term behavior of a system can rather be expressed in terms of the slowly evolving variables.

Such modeling approaches are well known in theoretical physics. The present invention relates to a method for the selection of biological markers suitable for such a modeling approach and the implementation of this approach in the context of biological processes.

The term "system" refers to a biological entity, preferably an ecosystem, an organism, subcellular system or a population of cells, most preferably a single cell.

The term "biological process", preferably, refers to the interactions of the components of a prokaryotic or eukaryotic cell, a group of cells, a subcellular system or a multicellular organism or of an ecosystem which leads to observable changes in the behavior of the studied system. It is to be understood that the biological processes to be studied by the method of the invention are the result of the interaction of dozens ore even hundreds of components, which influence each other in a non-linear fashion. In the case of cells preferred biological processes are those that lead to migration, differentiation, the production of signaling molecules, the production of metabolites, the degradation of chemical compounds, proliferation or death. In the case of groups of cells preferred biological processes are proliferation, preferably the growth of a tumor or the formation of metastases, differentiation processes, the development of tissues or organs or the production of signaling molecules. A biological process in a subcellular system is, preferably a signaling cascade that transduces an intra- or extracellular signal. In the case of multicellular organisms preferred biological processes are development, cognitive processes or metabolism. In the case of ecosystems preferred biological functions are changes of abundance of species, particularly elimination, migration of individuals to other habitats or changes in the flows of matter or energy in the system. The term "species" has to be understood in a broad sense and not in the restricted sense of the biological species concept. It refers to any group of individual organisms that is characterized by at least one unique feature.

A "biological marker" is a biological entity, preferably a biological molecule or an organism associated with a biological process. Preferred biological molecules encompass polynucleotides, polypeptides, peptides or small molecules such as metabolites.

The proteins responsible for the process in question, particularly their activities, are preferred biological mediators if a cell, a group of cells or an organism is to be studied. However, for the purpose of a high throughput screening it may be preferable not to measure protein activities directly but rather to use protein amounts or mRNA levels as a proxy. The determination of mRNA levels is especially preferred because it is amenable to high throughput screening. Also preferred is the determination of protein levels with high resolution 2-dimensional gel electrophoresis or with mass spectometry. If the system to be studied is a group of cells or an organism, cell numbers or the metabolic activity of the cells are preferred markers. The metabolic activity of cells is preferably determined by the consumption of nutrients, by the formation of metabolic products or by the uptake of labeled compounds. If the system to be studied is an ecosystem, the numbers of individuals of a taxon or their activity are preferred markers. The person skilled in the art knows which methods to use for the counting of individuals of a certain species. The activity of organisms is, preferably, determined by the amount of their metabolic products by their nutrient consumption or by the uptake of labeled substances into their biomass.

The term "measuring the amount" in the context of the present method for modeling the behavior of a complex biological system, preferably, refers to determining the absolute amount of a biological marker. However, it is also preferred to determine changes of the amount of one marker in a sample relative to the amount of said marker in other samples. Furthermore, it is especially preferred to determine the amount of at least one biological marker relative the amount of at least one other marker in a sample. For these operations it is not necessary to determine absolute amounts of the biological markers. It suffices to measure a signal that is correlated to the amount of the biological marker in question.

A preferred method for deciding which biological markers are suitable for the modeling of a system according to the present invention is a ranking of biological markers based on their peak and mean fold abundances/activities. Those biological markers are selected whose abundance/activity shows high peaks, a high mean over the time span of the experiment or both. Each marker can be assigned a unique position in a two-dimensional space based on these two features. The selection is not based on absolute abundances or activities but rather on the abundance/activity of a biological marker normalized to maximal peak fold and mean fold abundance/activity of all biological markers.

The term "fitting of equations to the measured amounts" refers to a process, wherein parameters for each equation are developed that mathematically describe the observed development of the biological markers. Each biological marker is represented by one differential equation that mathematically describes its behaviour. Said equation is solved by more than one set of parameters, i.e. more than one set of parameters can be used to describe the behaviour of the biological marker over the first time span. Thus, it is impossible to select one set of parameters based only on the fitting procedure alone, because this procedure produces several sets of parameters that solve the equation. Methods for fitting the equations to the measured amounts are known to the person skilled in the art. Especially preferred are those methods that are described in Example 1.

Methods for the evaluation of the fitness of a model are known to the person skilled in the art as well.

The term "simulation of the equations" refers to a process wherein it is tested which of the sets of parameters for the equation that were developed by fitting the equations to the measured amounts is selected for the modeling.

The term "predetermined behavior" refers to a behavior of a biological marker that is biologically plausible. Because there are several sets of parameters for each equation and each of these sets describes the behavior of the biological marker in question over the first time span equally well, it is necessary to select one of the sets of parameters and to eliminate the other solutions. For each marker it can be decided theoretically which kind of behavior is biologically plausible. The markers for cell migration described elsewhere in this application, for example, can be expected to return to a low basal level in the absence of a stimulus. Based on the behavior of the marker over the second time span as predicted by the simulation of the equations, for each equation is one set of parameters selected based on the conformity of the predicted behavior of the marker to the biologically plausible behavior. It is to be understood that for the biological markers of other biological processes other kinds of behavior are plausible.

The term "robustness evaluation" is well known to the person skilled in the art, as are methods to perform such a robustness evaluation. For the method of the present invention those methods are preferred that are described in example 1.

The term "parameter evaluation" is well known to the person skilled in the art, as are methods to perform such a robustness evaluation. For the method of the present invention those methods are preferred that are described in example 1.

All references cited in this specification are herewith incorporated by reference with respect to their entire disclosure content and the disclosure content specifically mentioned in this specification.

The figures show:

Figure 1: **Identification of genes mediating HGF-induced cellular transition to migration** (A) Workflow of data analysis, inverse modeling and experimental validation. (B) Histogram of rank scores for all 20188 measured genes after HGF stimulation (cf. Methods Section). The ranks numbered 1-20 correspond to the 20 highest ranked genes (cf. gene list in (C)). (C) Top: mean and peak fold expression of the 20 top-ranked genes upon HGF stimulation. Bottom: expression of the same genes upon FGF-7 stimulation. Data points were normalized to the maximal mean and peak fold expression of the respective experiments (cf. Materials and methods).

Figure 2. **A HGF pulse is able to stimulate a sustained migratory response.** (A) Hypothetical input functions to the gene regulatory network. Blue: pulse, black: constant, green: oscillatory, yellow: non-linear increase, red: exponential decay. (B) A short HGF input pulse is able to induce a sustained migratory response. HGF induced migration distance of HaCaT cells for untreated (control), prolonged incubation (30 h), prolonged incubation (30 h) with Actinomycin D inhibition (5 μg/ml) and short time (1.5 h) incubation with 10 ng/ml recombinant human HGF, respectively (from left to right). For short time treatment, cultures were washed extensively after stimulation and media was replaced. Error bars denote ± standard deviation of mean migration distance from three independent experiments. Note that migratory responses for a constant and pulsed stimulus are almost indistinguishable

Figure 3: **Gene regulatory network topology of genes mediating HGF-induced migration predicts multiple points of interference.** (A) Spline-interpolated gene fold expression time series of all genes considered for the *in silico* analysis of the nine node network. Error bars denote the standard deviation of the probesets' fold expression values for each gene. The minimum error is ±0.08 fold expression, calculated from the mean fold expression of all genes. (B) Interaction weights $W_{ij}$ for the CTRNNs obtained by inverse modeling for networks of size 3,5,7,9 genes (from top to bottom). (C) Offsets θ, delays Δτ, time constant τ and input amplitudes / for the nine gene network. The interaction weights (B) and the parameter values in (C) are color-coded. Note that parameter values are mostly robust with increasing network size. (D) Maximal interaction strengths $W_{ij}^{\max}$ (Eq. 6) for the nine gene network shown in (E). (E) Network diagram for a nine gene network with interaction weights taken from (B). Note that only the strongest interactions are drawn for better illustration. (F) Histogram of the linear Pearson Correlation coefficients of parameter estimates from a nine gene network using the original (pink), time randomized (green) and additionally normalized experimental data (blue), having the correlation mean and standard deviation values of 0.97/0.01, 0.25/0.24 and 0.13/0.20, respectively (cf. Methods section). (G) Modulation of HGF induced migration by PKA- or EGF-R activity, simultaneous to HGF pulse. To induce a migratory response, HaCaT cells were incubated (30 h) with or without 10 ng/ml recombinant human HGF. PKA activity was modulated by addition of 1 μM H-89 (Calbiochem) or 200 μM 8-(4-Chlorophenylthio)adenosine 3',5'-cyclic monophosphate sodium salt (Sigma). Inhibition of PKA by addition of 10 μM Myristoylated PKI (14-22) amide, cell-permeable PKA inhibitor (Biomol) gave rise to similar results (data not shown). EGF-R activity was blocked by addition of 1 μM GW 2974 (Sigma). Error bars denote ± standard deviation of mean migration distance from three independent experiments.

Figure 4: **Sustained migration depends on a second input mediated by epidermal growth factor receptor.** System response to varied input strengths and shapes. (A) System response to increasing HGF input strength. Input functions are depicted in the small insets. The learned maximal input amplitudes are scaled with the following factors {0.0, 0.7, 0.8, 0.9, 1.0, 1.5, 2.0, 3.0} from dark green to pink (B) System response to a combined initial HGF input and subsequent second periodic input. Simulations were performed with a nine gene network. The ratio of maximal input amplitudes between the first and the second input increases from 0.0 (black) via 0.04, 0.06, 0.07, 0.08, 0.1, to 0.12 (purple). Results in (A-B) are shown for *egr3* (left), *ptgs2* (middle) and *akap12* (right). (C) Blocking the second, periodic input at (I) T=7.5h, (II) T=22h, (III) T=42.5h. Red arrows denote the time point of blocking. A sample input function normalized to one is shown in the small insets. (D) Relative migration upon EGF-R blocking or PTGS-2 inhibition 22 hours after HGF stimulation. HaCaT cells were stimulated for 1.5h with 10 ng/ml recombinant human HGF. Cells were washed extensively and further incubated (30 h). Functional perturbation of PTGS-2- (or EGF-R) activity was introduced by addition of 50 μM Meloxicam (4-Hydroxy-2-methyl-N-(5-methyl-2-thiazolyl)-2H-1,2-benzothiazine-2-carbox-amide-1,1-dioxide1, 5 μM GW 2974 (Sigma) (or 150 nM Tyrphostin AG1473 (Biomol)) 20.5 h after HGF pulse stimulation to culture media. Migratory response was determined in the time window of 22-30 hours beginning with time point of perturbation. Pretreatment of cells with recombinant EGF for 2 h (1 ng/ ml) before HGF stimulation is not able to replace EGF-R activity directly blocked after HGF-stimulation. Error bars denote ± standard deviation of mean migration distance from three independent experiments.

Figure 5: **A group of modulator genes regulate the minimum threshold intensity of the second input for sustained migration.** (A) Steady state behavior of *ptgs2* as a function of increasing input amplitude of the second pulsed input. *Ptgs2* as the highest ranked gene has been shown to be important over the whole period of cell migration. Hence its expression level can be considered as a marker for migration. (B-G) Simulation of *ptgs2* expression under increasing second input amplitude with regulated modulator genes. Input amplitudes of the second input $I^1$ are measured in relation to the initial input strength of the learned amplitude $I^0$ of the first input. Ratios are increased from 0 to 0.2 in steps of 0.02. Each line corresponds to the simulated expression level of *ptgs2* for a given ratio $I^1/I^0$.

Figure 6: **Enhanced PKA-pathway activity is able to prevent onset of migration or to stop a developed migratory response at any time.** (A-B) Effect of *akap12* on simulated gene expression for the nine gene network. The insets depict the normalized input into the network. (A) A short upshot of *akap12* expression at T=5/20/40 hours (I/II/III) switches off gene expression and thus cell migration despite continued input signaling. (B) Inhibition of *akap12* rescues the system from switching off (I), and (II-III) slightly up-regulates *ptgs2* (top green line) and down-regulates *egr3, egr1* and *fos* expression (bottom, pink, blue, green lines). The strength of down-regulation correlates with the transient length for *ptgs2* (depicted by grey area) to reach the new steady state. (C) Activation of the PKA-pathway at different time points with respect to HGF pulse stimulation (10 ng/ml for 1.5h) by supplementing culture media with 200 μM 8-(4-Chlorophenylthio)adenosine 3',5'-cyclic monophosphate (Sigma) strongly decreases HGF-induced HaCaT migration. Inhibition of PKA activity by addition of 0.5 μM H-89 at the time of HGF stimulation slightly increases migration. Inhibition of PKA-activity by H-89 (0.5 μM) directly after HGF pulse stimulation (1.5 h) does not alter development of a migratory response. Inhibition of PKA activity after 22 h, when cells already developed a full migratory response, delays further migration. Error bars denote ± standard deviation of mean migration distance from three independent experiments.

Figure 7: **Time ordered sequential events control cellular decision of migration.** Schematic representation of events regulating the initiation, maintenance, modulation and stopping of cell migration. (A) The migratory response depends on a certain threshold of gene activity, as indicated by the dashed line, and proceeds as long as no context information is provided via a stop signal or the sustained activation is depleted. (B) Summary scheme of the model for migration. A first pulse-like HGF stimulus is required to transform the cell into a sensitive state for migration. To initiate and sustain migration a second input preferably through the EGF-R signaling pathway is required. This second input can be modulated by a number of genes. Context information is integrated through the PKA pathway, which can stop migration at any point in time.

The following Examples shall merely illustrate the invention. They shall not be construed, whatsoever, to limit the scope of the invention.

**Example 1: Gene Network Dynamics controlling Keratinocyte Migration**

To derive an integrated model of cell migration, a workflow for data analysis, inverse modeling, and experimental validation was established (Fig. 1A). In the first step, it was aimed at identifying genes governing migration. DNA microarray experiments were performed at several time points upon HGF stimulation to obtain the gene expression kinetics from heterologous co-cultures containing primary human keratinocytes and murine *cjun* deficient fibroblasts. The latter were chosen to discriminate between human and murine mRNA, based on species-specific sequences and to provide an HGF-free background. Keratinocytes were stimulated with hepatocyte growth factor, HGF, which induces both proliferation and migration (Birchmaier *et al*, 2003). Three control experiments using Keratinocyte Growth Factor (KGF, FGF-7), Granulocyte Macrophage Colony Stimulating Factor (GM-CSF) and Stromal Derived Factor-1 (SDF-1) as stimuli were conducted, all of which induce cell proliferation, but not migration (Werner *et al*, 1994; Braunstein *et al*, 1994; Florin *et al*, 2004).

The gene expression kinetics from each experiment was ranked according to the absolute value of their mean and peak fold expression (see Materials and Methods). The rank distributions show a long tail at high rank scores, likely indicating that only very few genes mediate the decision to and the onset of cell migration or proliferation, respectively (cf.

Fig. 1B). However, genes that are strongly regulated upon the migratory HGF stimulus show a weak or even a down-regulated response upon FGF-7, which mediates proliferation (cf. Fig. 1C). Using other proliferation-inducing stimuli led to the same qualitative results (data not shown). This specific up-regulation of genes in response to HGF supports the idea that the ranking identified a set of important genes mediating HGF-induced migration. Indeed, a gene ontology (GO) analysis of the top ranked genes upon HGF stimulation reveals - among other categories - an enrichment with respect to response to stress, wounding and external stimulus, extracellular region and development. Contrary to this, the GO enrichment for up-regulated genes upon the FGF-7 stimulation finds protein targeting, import, transport and localization categories enriched. As an additional control, the gene expression kinetics were ranked using MaSigPro (Conesa et al, 2006), a statistical procedure to identify genes having distinct temporal expression profiles across analytical groups. The results of this regression-based ranking method show a good agreement with respect to differentially expressed genes and the respective ranking.

In order to further decipher the cellular information flow in the context of cell migration, the 50 top ranked genes were grouped into four categories representing (I) transcriptional regulators, (II) genes regulating intra-cellular information flow (receptors, kinases, phosphatases and other genes involved in signal transduction, e.g. scaffold proteins), (III) genes, whose products are secreted by the cell and are involved in matrix remodeling and cell-cell communication or are components of the extracellular matrix, and (IV) genes coding for structural proteins in the broadest sense and genes with unknown or unclear functions. Genes from groups (I) and (II) were considered for modeling. Genes from group III producing secreted proteins acting in a paracrine manner or remodeling the extracellular matrix were excluded, as the time delay between initial regulation and feedback via double paracrine activity is beyond the experimental time window. Genes from group (IV) were excluded, because their unknown or unspecified function might lead to misleading model results.

**Network topology and dynamics of genes mediating migration**

The causal relationships of the gene expression data (cf. Fig. 3A) were inferred using a Continuous Time Recurrent Neural Network (CTRNN) as an abstract dynamic model for the gene regulatory network mediating the cellular decision to migrate upon an external stimulus. The model describes the mutual influence of genes and their stimulus response as dynamic elements, regardless of how such an interaction or stimulation is realized in concrete biological terms. The experimental data were repeatedly fitted using a genetic algorithm and the independently obtained network solutions were further ranked according to their robustness to yield biologically plausible parameter estimates (see Materials and Methods). In general, the exact functional relationship between the external cellular stimuli and their regulatory impact on the respective genes is unknown. Several possible effective input functions can be assumed for modeling (cf. Fig. 2A). A single pulse-like input provided the best fitting results of the model dynamics with the gene expression kinetics. Experimentally, the presence of HGF for 1.5 hours is sufficient to initiate a sustained migratory response up to 30 hours (Fig. 2B), indicating that HGF-induced Met signaling solely acts as an initial trigger. HGF activity, thus, either induces a series of events leading to a lasting, self-sustained migratory response, or 'preconditions' the cell to a responsive state, upon which different system inputs and/or signaling pathways sustain migration.

The ranking restricted to genes in the functional groups I and II (see above) provides a list of genes with respect to their putative role in mediating cell migration. Modeling of the top ranked gene interactions started with a small three gene core network, consisting of the transcription factor *egr3, akap12*, encoding for the A-Kinase Anchor Protein-12 and *ptgs2,* encoding for Prostaglandine-Endoperoxide Synthase 2, PTGS-2, also known as Cox-2. Growing the network in size from three to five, seven and nine genes and each time repeating the inverse modeling workflow qualitatively conserved the topology of this gene core network (cf. Fig. 3B top to bottom) with the signal-to-noise ratio of the parameters increasing with network size.

As a further model verification a bootstrapping test was performed by fitting the data (i) to time-randomized gene expression kinetics and additionally (ii) to amplitude normalized data. (cf. Materials and Methods). The interaction weights thus obtained showed a low correlation with the original model (cf. Fig. 3F), indicating that the solution to the CTRNN obtained from inverse modeling is indeed a result of the temporal order of the experimental gene expression kinetics.

Usually, it is difficult to decide on the optimal model network size that is large enough to comprise all necessary variables, yet admissible for numerical investigation. However, the maximal gene interaction strengths $W_{ij}^{\max}$ from the learned networks (cf. Materials and Methods) showed that all genes in a rank order below *fos* have a weak regulatory effect in the network (cf. Fig. 3D), i.e. their dynamics are controlled by the external input and the higher ranked genes and hence a network with about 10 genes suffices to include most of the genes having a regulatory effect on the cellular decision towards migration. In the following we thus continue to work with a nine gene network.

All further model verification was done experimentally on the protein level. This is justifiable under the presuppositions that (i) protein levels follow mRNA changes over time, assuming the lack of actively regulated protein decay and (ii) the validity of the adiabatic approximation of fast protein signaling events as described in the introduction. As a consequence, information on long-term cellular decisions should be determined - or at least reflected in - the dynamics of the cellular gene regulatory network justifying the translation of the genetic dynamics in our model simulation into protein concentration changes and pathway activity, respectively.

*Ptgs2* fold expression was used as an indicator for model-based analysis of cell migration (*ptgs2* fold expression > 2), because it represents the highest ranked gene and has been shown to be important for initiation and sustaining of cell migration both *in silico* and *in vitro*.

Using a human keratinocyte cell line (HaCaT) in a Scratch-Assay as a model for wound healing, different model-predicted points of interference were tested. First it was confirmed that HGF-induced migration predominantly depends on a transcriptional response. Blocking transcription by Actimocyin D treatment abolished any migratory response to HGF (Fig. 2B), while the cells were still viable as controlled by a MTT-viability assay (data not shown).

Blocking the PTGS-2 activity reduced HGF-induced migration. In fact, the potential to inhibit migration appears largest at the onset of cell migration (compare Fig 3G, early and late time points). The network model finds the gene *akap12* to be important to cell migration, having mainly an inhibitory effect. This gene's protein product, AKAP-12, regulates the activity of protein kinase A (PKA) by tethering the enzyme near its physiologic substrates. PKA-signaling could suppress or negatively regulate HGF-induced migration. Here, in agreement with model predictions, simultaneous stimulation by HGF and enhancement of PKA-activity prevent the onset of migration. Decreasing the activity of PKA enhances the early migratory response (6h time points Fig. 3G). Further, a decreased PKA-activity alone is not sufficient to induce a migratory response in the absence of HGF stimulation and also other inhibitors used reveal only an effect on migration in combination with HGF stimulation.

Further down in the gene ranking list, the genes *plau*/*plaur* as well as *errfi1* and *hbegf* are found. *Plaur* and *plau* encode for the plasminogen urokinase activator receptor (PLAU-R) and its ligand plasminogen urokinase activator (PLAU), respectively. It has been shown that the inhibition of the PLAU receptor decreases, but not completely abolishes the migratory response (Schnickmann *et al*, in preparation). HB-EGF is a ligand to the EGF receptor, while ErbB Feedback Inhibitor 1 (ERRFI-1) is a cytoplasmic protein (Wick *et al*, 1995) that can interact with Erbb-1 and -2 (two EGF-R isoforms) and modulate their activity. Gene ranking and the network topology (Fig. 3E) thus suggest that EGF-R signaling is necessary for HGF induced migration. As predicted, blocking EGF-R signaling through the application of a double specific inhibitor (GW2974) targeting both EGF-R isoform activities (ERBB1 and ERBB2) abolish HGF-induced migration (Fig. 3G).

The inferred network topologies provide limited information concerning the dynamics of the network. To address the question whether the functional interference is mediated via a crosstalk between the signaling pathways and, additionally, whether the cell integrates this information on the genetic network level, *in silico* simulations and perturbations followed by experimental validations were performed.

**Time sequential signaling triggers and sustains cellular migration**

As shown above, the HGF input is only required during the first ≈ 1.5 hours to induce cell migration. The questions remains, whether this initial trigger is sufficient to induce a sustained migratory response, or whether an additional input at a later time point is required. Model simulations predict that even a massively enhanced HGF stimulus is not able to induce a permanent system response (cf. Fig. 4A). All genes are up-regulated with increasing stimulus intensity in a switch-like fashion. Yet, the upper, active state is apparently weakly unstable, as the gene expression, returns to the lower off state after roughly 24 hours, indicating that cell migration likewise would be stopped. This result is contradictory to experimental findings on cell migration, because cells can migrate for days during the process of wound healing.

The discrepancy between model prediction and experimental results can be resolved by either assuming that migrating cells receive a repeated HGF input with a minimum period of approximately one day, or a different system input is missing in the model. The experimental finding that a transient HGF stimulus already causes a sustained cell migration excludes the former hypothesis (Fig. 2B). Different lines of evidence further support the latter interpretation. The time series of a number of genes encoding for receptors and their respective ligands show a three hour regulation period suggesting a second, time-lagged periodic input into the system. Further, the identified genes in the network and the network topology itself hint at the influence of autocrine feedback loops (Fig. 3E).

Therefore, a second CTRNN model variant was fitted to the gene kinetic data, this time with each gene receiving an additional weak, pulsed input setting in 2.5 hours after the first input (see Materials and Methods, Eq. 6). Under this assumption model analysis predicts that the cell remains in a responsive state for up to 7-8 hours during which the onset of a minimal, permanent, second input suffices to develop a full and sustained migratory response (see Fig. 4B). After that time the cell returns into its previous homeostatic state, requiring again the preconditioning HGF trigger to initiate a new migratory response.

Repeating the inverse modeling workflow with the complex input (insets in Fig. 4B and Methods section), essentially the same network topologies and parameters were found as compared to the HGF input-only model. Simulation of network dynamics under the chosen conditions led to a stabilized active system state (Fig. 4B) as long as the system received the second input. Blocking the periodic input at any time switched the system off (see Fig. 4C). Note that the second input was modeled as periodic due to the fact that it most probably constitutes an auto- or juxtacrine signaling loop with the corresponding ligand/receptor genes periodically expressed. However, model simulations show that the same results hold true for a constant input as well (data not shown).

Model analysis suggests that the migration-sustaining information is modulated via Errfi1, thereby changing the MAPK-pathway activity (Ferby *et al*, 2006). Also the kinetics of the EGF-R ligand HB-EGF, shows a basal cellular expression that is increased in response to HGF stimulation (data not shown). Taken together with the above *in silico* prediction, the model suggests that continued EGF-R activity starting at around two hours after the initial HGF stimulation is essential to develop and sustain a migratory system status (Fig. 4B). This prediction was tested experimentally by stimulating cells with HGF for 1.5 hours and subsequently blocking EGF-R afterwards. In full agreement with the model predictions (Fig. 4C), blocking the EGF-R activity stopped cell migration even 22 hours after HGF stimulation (Fig. 4D). Moreover, EGF-R activation two hours before HGF stimulation is not sufficient to replace EGF-R activity after HGF stimulation (Fig. 4D), further supporting the hypothesis that HGF and EGF-R, respectively, trigger and support migration. In addition, the time-delayed perturbation experiments suggest that the processing and the functional interference of HGF and EGF-R signaling converge on the genetic network level.

**A group of modulator genes regulates the second input for sustained migration**

To study the precise regulatory role of *dhrs9, gpr109b* or *errfi1* in the nine gene network, their expression levels *in silico* were changed to investigate their modulation effect on cell migration. As above, *ptgs2* was used as an indicator for migration. Figures 5B-G depict the corresponding temporal behavior of *ptgs2* with increasing amplitude of the second input upon up- or down-regulation of the above genes. Down-regulation of *dhrs9* reduces the switching threshold for migration (Fig. 5A: shift of corresponding dark blue curve to the left compared to the black control curve), as the model predicts this gene to be an inhibitor of *ptgs2.* Up-regulation of *gpr109b, errfi1* or *dhrs9* shifts the migration threshold to larger input amplitudes (Fig. 5A, shift of corresponding curves to the right compared to the black control curve). Lastly, up-regulating both *dhrs9* and *gpr109b* shows an additive effect of their actions: the second input amplitude has to be significantly increased to induce a migratory system response (Fig. 5A, shift of corresponding brown curve to the right compared to all other curves). Model analysis thus reveals a complex modulation of the second input.

**PKA-Signaling provides context information and is able to stop migration at any time**

The above model analysis has demonstrated that both the amplitude and the timing of external and/or internal stimuli are important regulatory factors. In this line of thought, the impact of the PKA pathway on the early onset and the later steady state behavior of cell migration was investigated. *In silico* simulations predict that a pulse-like up-regulation of PKA stops cell migration at any time (Fig. 6A). To prove this *in vitro*, cells were stimulated with an HGF pulse to trigger the responsive state and to initiate the migratory response. PKA activity was enhanced at different time points thereafter (Fig. 6C, red bars). In full agreement with model predictions, enhancing the PKA-activity not only prevents the onset of migration, but it also shuts down the system both during the transition phase to migration (enhanced PKA-activity after 1.5h HGF stimulation) and at later time points, when the system has already stably established the migratory system state (perturbation at 22h after triggering migration by HGF).

Most interestingly, the complementary approach of decreasing PKA activity and thereby enhancing migration gives rise to a more complex scenario. Model simulations show that a down-regulation of *akap12* modulates, but does not stop cell migration. The gene expression levels after *akap12* regulation are similar, regardless of the drop of *akap12* expression (cf. Fig. 6B). The transient time to reach the new steady state, however, drops with increasing strength of this regulation. Moreover, the regulation has antagonistic effects, because *akap12* negatively regulates *ptgs2* and positively regulates transcription factors, such as *egr3*, *fos* or *egr1*. Up-regulation of all these genes promotes cell migration *in silico.* Which of these regulatory effects dominate are a matter of timing and the presence of the initial trigger HGF, since inhibition of PKA activity without HGF stimulation is not able to induce keratinocyte migration. Indeed, down-regulation of PKA-activity *in vitro* during the immediate trigger stage of migration results in a slightly enhanced migration, because the then important factor *ptgs2* becomes more active (immediate response in Fig. 6C). Late PKA pathway inhibition slows down migration, which can be an effect of transcription factor down- regulation (late response in Fig. 6C).

These findings indicate a crosstalk between PKA- and HGF signaling during the trigger state of migration, while at a later stage, signaling via the EGF-R and the transcription factors seems to be dominating. This is again in line with the experimental findings: immediate blocking of the PKA pathway roughens the migration front. PKA pathway regulation apparently allows the system to integrate context information that is derived from its environment, namely from cell-cell interaction and from communication with surrounding tissue. Interestingly, the migration state of cells is not constant since average migration speed increases over time (cf. migration speed at immediate and late time points in Fig. 6C).

**Discussion**

The performed integrated theoretical and experimental analysis of gene expression kinetics deciphers the dynamics of a surprisingly small, but extremely complex gene regulatory model of migration by inverse modeling.

Many methods for the topological and dynamic reconstruction of gene regulatory networks have been developed in recent years (for reviews see Stolovitzky et al, 2007; Bansal et al, 2007). In particular, neural networks have been previously applied for modeling signaling pathways and gene networks (Reinitz and Sharp, 1995; D'haeseleer, 2000; Vohradsky, 2001). However, the predictive power of these models, and hence their impact on biology has been rather limited up to now, which is attributed to the disproportionately large number of unknown parameters with respect to the experimental data and to the indefiniteness of the systems under investigation (Krishnan et al. 2007). Here, it is shown for the first time that inverse modeling of a gene network can establish an abstract model of a complex biological system that captures well its dynamic properties and has predictive power. This success is most likely due to several reasons: the choice of experimental time window focusing on the decision to migration and measuring at a sufficiently high sampling rate to capture the gene network dynamics, the choice of gene candidates from ranking, biological function and the cross validation against proliferative stimuli, the limited number of genes dominating the network dynamics and lastly the combination of fitness and robustness criteria in the search for a biologically plausible gene interaction model.

Model simulations proved efficient to elucidate the dynamic functionality of genes. A schematic overview of the revealed dynamic picture of interactions as well as the time series of events and different possible point and times of interference is provided in Fig. 7 and is discussed in detail below. The model for migration further suggests that the initiation of migration acts in a two-step mechanism. The first input into the system is triggered via the cellular reception of HGF through its Met receptor and subsequent signaling via the MAPK-pathway, bringing the cell to a responsive state. Complete development of a migratory phenotype and sustained migration, however, depends on a second input, most prominently via the time-sequential activation of EGF-R, which again feeds into the MAPK pathway. Redundantly to EGF-R, plasminogen urokinase activator receptor (PLAU-R) and its ligand plasminogen urokinase activator (PLAU) can contribute to the complete development of a migratory response (Schnickmann *et al*, in preparation). Different EGF-R ligands (such as EGF and HB-EGF) are expressed by keratinocytes both at a basal level and as a result of HGF stimulation (data not shown). The EGF-R mediates cjun-dependent formation of the keratinocyte leading edge (Li *et al*, 2003; Zenz *et al*, 2003) and is also an important player during tumor development and progression (Burtness, 2007; Nakamura, 2007; Sharma *et al*, 2007). Likewise, PLAU-R has been shown in a mouse model to be positively correlated to tumor initiation and growth (Ploplis *et al*, 2007). A functional connection between HGF and EGF-R signaling for migration and invasion was reported recently (Xu and Yu, 2007; Zhou *et al*, 2007), but these studies did not consider the temporal order of events and only discussed potential crosstalk at the signaling level.

Model simulations based on an initial activation by HGF and subsequent EGF-R input show that the cells remain responsive for approx. 7-8 hours, thus sensitizing the system to initiate migration even under adverse environmental conditions. Interestingly, model analysis demonstrates an active modulation of the second input through at least three different gene products, namely the G-protein coupled receptor 109B (GPR109B), DHRS9 and erbb feedback inhibitor 1 (ERRF11). Gpr109b is able to regulate cAMP levels and through that PKA activity (Tunaru *et al*, 2003), co-regulation DHRS9 and the Adenomatous Polyposis of the Colon (APC) gene in colon carcinomas (Jette *et al*, 2004). An *in silico* knockdown of *dhrs9* increases *ptgs2* expression (Fig. 5C) and therefore lowers the threshold for initiation of migration. This is in line with the biological observation that prostaglandin, which is synthesized by *ptgs2*, synergistically enhances tyrosine kinase-dependent signaling in colon cancer cells (Shao *et al*, 2004), and thus regulates cell motility by modulating EGF-R activity (Banu *et al*, 2007). The dominant roles of the PKA pathway and PTGS-2 activity with respect to migration are generally accepted in a variety of cell types and tissues (Howe, 2004; Rüegg *et al*, 2004; Liao *et al*, 2007). Our results suggest that strongly and persistently up-regulated genes, such as *akap12* or *ptgs2,* regulate and determine the global dynamics of the system and the simulated expression levels are indeed suitable indicators of a cellular migration state.

Because the initial Met and the following EGF-R signals converge on the MAPK pathway there must be features specific for each trigger, which might stem from dynamic regulation dependent on the history of a cell. In fact, it was shown that temporal information encoding can be realized for MAPK signaling via negative feedback regulation (Amit et al, 2007), and that ligand specificity can be achieved through mutual inhibition of distinct compounds in the same pathway (McClean *et al*, 2007). Comparing the gene expression profiles from this study with those obtained from EGF-only induced migration of a breast cancer cell line (Amit *et al*, 2007), one finds similar kinetics for the genes critically involved in the decision towards migration. However, the kinetics of some responding genes differs in the first three to four hours after stimulation. These alterations are especially prominent for genes modulating MAPK activity (DUSPs) as well as for DHRS9 and GPR109B, which were postulated to modulate EGF-R signaling intensity. Interestingly, while the initial conditions of each epithelial cell type (MCF10A and primary human keratinocytes) are different, they eventually converge to the same gene expression dynamics over time.

A variety of cell types and tissues are known to express secreted soluble factors or cytokines and their respective receptors. Still, a single factor is generally not sufficient to induce a complex cellular response like migration. Cells typically integrate a variety of internal signals together with environmental stimuli to compute a cellular decision. This mechanism prevents unwanted migration at the wrong time and place. Indeed, neither the transient HGF nor the constant EGF-R input alone can induce or sustain a lasting system response in the model simulation (Figs. 4A and 6A). During wound healing the cellular migration is restricted to the wound edge closely followed by a zone of enhanced proliferation. However, the balance between the proliferative and migratory zones is modulated dynamically depending on the wound type, bacterial infiltration or the inflammatory response (Martin, 1997; Bolotin and Fuchs, 2003; Stramer and Martin, 2005; Eming *et al*, 2007). Therefore, it is critical for a cell to integrate such diverse situations into the decision to migration, even under circumstances, when both HGF and EGF are present. Our data suggest that this kind of context is mediated through the PKA pathway activity (Fig. 6), onto which many receptors for paracrine signaling converge. Interestingly, the irregular shaped migration front induced by decreased PKA activity is reminiscent of the surface structure of a growing carcinoma. Importantly, it is not the activity of a single factor alone, but a sequence of time-ordered events with respect to HGF stimulation that determines the functional role of PKA signaling to prevent, enhance, decrease or stop migration. It should be noted that an involvement of the main signaling pathways (namely MAPK- and PKA-pathway) are in agreement with diverse published literature for different epithelial cell types, even if they where not always explicitly shown for keratinocytes. This study contributes to an enhanced understanding of migration by unraveling the dynamic interplay of genes mediating the time dependent order of their activities.

While biological systems are usually complex, it was surprising to see so few genes establishing a simple, yet robust decision making switch towards cell migration. Whether this finding of a few genes mediating cell fate decisions holds true in general in cellular processes such as proliferation, differentiation or cell death, or whether this is particularly true for cell migration cannot be decided at present. It should be noted, however, that the system reduction approach of the present study was only possible because the onset and thus the core function for the decision towards cell migration was considered. The complexity of all control or safety elements that guarantee a robust and simple behavior (Lauffenburger, 2000) as well as the actual migration processes was neglected in this study study.

The dependency on only a few genes for determining the cell transition towards migration shows a striking resemblance to principles in self-organizing systems (Haken, 2004). At the transition to the emergence of a new global system organization, often a drastic lowering in the degrees of freedom occurs. These degrees of freedom are then determined by a few ordering parameters that rule the emerging macroscopic pattern. If these parameters for system ordering guide one or more subsystems, they are said to enslave the subsystems, representing the key to understand how complex systems can self-organize under far-from-equilibrium conditions. Such phenomena of self-organization have been observed in many physical, chemical or biological systems undergoing phase transitions, e.g. in lasers (Haken, 2004), morphogenesis (Meinhardt and Gierer, 2000), spatiotemporal genome organization (Misteli, 2007) or hematopoietic stem cell organization (Roeder *et al*, 2005). While the realization of order parameters is specific to each system, e.g. the amplitude modes of a laser cavity or the spatial distribution of morphogens in development, their abstract description on the systems level is the same. In this case, it is the gene expression amplitudes that constitute the order parameters of the cell. Interestingly, if only a few genes act as the ordering parameters to control a cell state transition, then it suffices to determine these genes to gain an understanding of the emergence of new cellular phenotypes.

As a consequence, the slaving principle is simultaneously a curse and a chance for understanding biological processes. On the one hand, it claims that only a cell-wide rebalancing of functional components is able to lead to a state transition, which is very hard to be understood and controlled in all details, e.g. for reverting a tumor to a normal cell. On the other hand, system complexity is greatly reduced during the process of transition. If system control is the goal, then it will suffice to focus on the identification and perturbations of the dynamics of the few functional elements mediating the transition. From this point of view the present data analysis and modeling approach might be generally applicable to understand other cellular processes that involve decision making and may well prove instrumental in the attempt to decode and translate the language of cells.

## Materials and Methods

### *Cell Culture*

Normal human skin keratinocytes (NEK) and dermal fibroblasts (HDF) were derived from adult skin (Stark *et al*, 1999). HDF obtained from outgrowth of explant cultures were grown in DMEM (Dulbecco's modified Eagle's medium; Bio Whittaker) supplemented with 10% fetal calf serum (FCS), and cells from passages 4 to 8 were used. Mouse wild type and *cjun-/-* fibroblasts were isolated from mouse embryos and immortalized according to the 3T3 protocol (Schreiber *et al*, 1999) and used together with HDF as feeder cells. NEK were plated on X-irradiated feeder cells (HDFi, 70 Gy; MEFi, 20 Gy) in FAD medium (DMEM:Hams F12 / 3:1) with 100 U/ml penicillin, 50 μg/ml streptomycin and supplemented with 5% FCS, 5 μg/ml insulin, 0.1 ng/ml recombinant human EGF, 0.1 nM cholera toxin, 0.1 nM adenine, and 0.4 μg/ml hydrocortisone (Sigma). For expression profiling total-RNA of human keratinocytes was isolated 1, 2, 3, 4, 6, and 8 hours after stimulation with recombinant human cytokines (10 ng/ml HGF, 10 ng/ml GM-CSF, 10 ng/ml FGF-7 or 10 ng/ml SDF-1; all obtained from R&D Systems). Selective trypsination and harvesting of human keratinocytes have been described elsewhere (Maas-Szabowski *et al*, 1999).

### *Migration assay*

Immortalized human keratinocytes (HaCaT cells) were cultured in monoculture with DMEM (10% FCS, 100 U/ml penicillin, 100 μg/ml streptomycin). Subsequently, the cell monolayer was damaged with a 'scratch' using a pipette tip and cells were treated with 5 μg/ml mitomycin c (Sigma-Aldrich) for three hours before stimulation. Cells were stimulated at indicated time points and periods with cytokines and/or inhibitors: 10 ng/ml HGF, (R&D Systems), 1 ng/ml EGF (R&D Systems), 150 nM Tyrphostin AG1473 (Biomol) (EGF-R inhibitor) or 1 μM GW 2974 (Sigma) (EGF-R inhibitor), 50 mM Meloxicam (Biomol) (PTGS-2 inhibitor), 0.5 μM H-89 (Calbiochem) (PKA inhibitor) or 10 μM Myristoylated PKI (14-22) amide, cell-permeable PKA inhibitor (Biomol), 200 μM 8-(4-Chlorophenylthio)adenosine 3',5'-cyclic monophosphate sodium salt (Sigma) (PKA activator) and incubated for further 30 hours. Relative migratory activity was determined by measuring migration distance during culture using standard protocols.

*Microarray Data Acquisition and Analysis*

Microarray measurements were recorded for four different stimuli from cocultures, namely HGF, FGF-7, SDF, GM-CSF. For each stimulus within one experiment 6 probes were taken (time points of 1,2,3,4,6 and 8 hours after initial system stimulation) and further analyzed. Total RNA was isolated, labeled and hybridized to HG-U133-2plus (Affymetrix) according to the manufacturer's protocol. Raw microarray data were processed using the R environment (R Development Core Team, 2007) and the Bioconductor toolbox (Gentleman, 2004). Probe annotation was handled with the Bioconductor package hgu133plus2cdf (Bioconductor Project). Normalization was performed using variance stabilization available in the Bioconductor package vsn (Huber *et al*, 2002). Quality of results was assessed using simpleaffy (Wilson and Miller, 2005). The gene fold expression was calculated with respect to the mean gene expression from two control measurements of an uninduced system at 0 and 8 hours. Measurement errors were calculated from the standard deviation in fold expression of the various probe sets for each gene. A lower error bound of ±0.08 fold expression was deduced from averaging the fold expression over all genes and all time points. Microarray data is available from ArrayExpress under the accession number E-TABM-440

*Gene Ranking*

Gene ranking is based on the peak and mean fold expression of the genes within the experimental time window of 8 hours. This uniquely defines the rank position of each gene in a two dimensional space. The combined rank score s is then defined as

$$s = \sqrt{FE_t^2 + FE_p^2}, \qquad (1)$$

where $FE_i = \langle g_i(t) \rangle_T$ and $FE_p$ denote the time-averaged mean, and peak gene fold expression, respectively, normalized to the maximal peak and maximal mean fold expression of all measured genes.

*Data interpolation*

Experimental gene expression time series are interpolated for model fitting using a cubic spline function with a sampling interval of $\Delta t$=0.1$h$.

*Neural Network Model*

The time-resolved gene expression data were inferred using a Continuous Time Recurrent Neural Network (CTRNN) as a dynamic model for a gene regulatory network. The CTRNN (Beer, 1995) is defined as the following set of N coupled differential equations describing the fold expression kinetics of N genes $g_i$. In brief,

$$\frac{dg_i(t)}{dt} = f_i(g_1,\ldots,g_N) = \frac{1}{\tau_i}\left(-g_i(t) + \sum_{j=1}^{N} W_{ji}\sigma\left[g_j(t-\Delta\tau_j) - \theta_j\right] + I_i(t)\right). \qquad (2)$$

$t_i$ and $I_i(t)$ denote the time constant and external input, respectively, while $q_j$ is an offset term, accounting for basal gene expression. Interactions between genes are incorporated by the weighted input of gene j into gene $i$ via the sigmoid activation function

$$\sigma(x)=\frac{1}{1+\exp(-ax)}. \tag{3}$$

The square matrix $W_{ji}$ describes the directed connection weights between genes . The factor a in Eq. 3 controls the steepness of σ(x) to adjust the transition width between the off and the on state of a gene. The individual summands in the interaction term of Eq. 2 can be positive or negative, depending on the sign of the interaction weights $W_{ji}$, mimicking gene activation or inhibition, respectively. The delay term $\Delta\tau_j$ is a generalization of the original CTRNN definition (Hu *et al*, 2005; Kim *et al*, 2007) and accounts for the time delay between gene induction, transcription, translation and final effect of a gene *j* on any other gene.

Model fitting to the experimental data has been performed twice using two different variants of the input function $I_i(t)$. The first model variant uses an exponentially decaying input function to each gene *i*

$$I_i(t)=I_i^0\exp(-\lambda t) \tag{4}$$

with a fixed decay λ and the initial signaling amplitude $I_i^0$ to be learned from the experimental data. It was chosen $\lambda=\frac{\ln(2)}{2}h^{-1}$, so that the decay of the input amplitude is approximately of the same time scale as the regulation of cellular kinases or phosphatases.

The second model variant has an additional, time-delayed periodic input $I^1$ to each gene *i* is modeled as

$$I_i(t)=I_i^0\left[\exp(-\lambda t)+r\left|\sin\left(2\pi(t-\delta t)p^{-1}\right)\right|\right]\text{with } r=0\mid\forall t\leq\delta t \text{ and } r=0.1\mid\forall t>\delta t \tag{5}$$

where δ*t*, p and r denote, in this order, the time lag, the input period and the amplitude ratio with respect to the corresponding learned input amplitude $I_i^0$. λ is the same parameter as in Eq. 4. In this study, it is set δ*t* = 2.5*h* in all cases and used *p=4h* and *r=0.1* for the inverse modeling. Model simulations use a period of 3 hours and an amplitude ratio of *r=0.08*, if not indicated otherwise. Changing the stimulus period within the above limits does not change system dynamics qualitatively.

Integration of the recurrent neural network is done using a first order Euler method with a step size of Δ*t* = 0.1*h*, constituting a good tradeoff between numerical stability of the integration and computational cost. The independent CTRNN parameters together with their respective ranges considered for model fitting are (I) the interaction weights $-10 \leq W \leq 10$, (II) the offset, $0 \leq \theta \leq 2.5$ , (III) the gene time constants $0.14h \leq \tau \leq 2.5h$, (IV) the delay $0h \leq \Delta\tau \leq 2h$ and (V) the input amplitude $-15 \leq I^0 \leq 15$. All parameters, except the time delay, were varied continuously. The latter is varied in multiples of the integration step size Δ*t* = 0,1*h*. Moreover, the parameter λ was fixed to yield a half-life of 30 minutes for the input decay. The scaling factor a of the sigmoid activation function is set to 4 for all genes throughout all simulations. All parameter ranges were chosen to yield results within biological reasonable bounds.

The maximal possible interaction between genes was calculated from combining the interaction weights $W_{ij}$ and the gene offset $\theta_i$ into a single quantity, the maximal interaction strength

$$W_{ij}^{\max}=W_{ij}\sigma\left(g_i^{\max}-\theta_i\right), \tag{6}$$

where σ, $g_i^{\max}$ and $\theta_i$ denote the sigmoid activation function, the maximal gene fold expression from the experimental data and the learned interaction weights and offset of gene *i*, respectively.

*Parameter estimation*

Model parameters were fitted to the experimentally observed gene fold expression time series with a genetic algorithm (GA) as a global optimization method, using mutation, crossing-over, and selection as well as employing an elitist strategy. The experimental data was interpolated to the integration step size using a cubic spline function (Figs. 1E, 3A). This procedure is based on the assumption that gene expression evolves smoothly in between the measured data points due to the lack of rapid gene expression dynamics.

The GA parameter estimation method used a population of 1000 CTRNN networks evolving over 3000 generations. The parameters in each network were randomly initialized and further on varied within the bounds given above. Networks composing the next generation were selected in pairs of two with a probability according to their fitness (Eqs. 7,8). A crossing-over equally mixed the parental parameter sets into one offspring network. The additional mutation rate of 0.02 randomly changed parameters in the offspring. The elitist strategy ensured the unchanged transfer of the fittest network to the next generation.

The fitness of a CTRNN with a given parameter set is given by

$$Fitness = \frac{1}{1 + \left[MSE + MSE_0\right]}, \qquad (7)$$

where MSE denotes the mean squared error between the spline interpolated experimental gene expression data $g_i^{\exp}$ and the simulated gene expression values $g_i^{\mathrm{mod}\,el}$,

$$MSE = \frac{1}{TN}\sum_{i=1}^{N}\sum_{t=0}^{T}\delta_T^{-2}\left[g_i^{\mathrm{mod}\,el}(t) - g_i^{\exp}(t)\right]^2, \qquad (8)$$

where T and N denote the total integration time and number of genes, respectively. $\delta_T$ is a tolerance parameter that accounts for measurement errors in the experimental data. Throughout this paper it is set $\delta_T = 0.1$. The $MSE_0$ term in Eq. 7 puts a fitness penalty onto system solutions having an unstable homeostasis state, hence calculating the mean difference from zero, when setting all external inputs to zero $(I_i^0 = 0)$.

$$MSE_0 = \frac{1}{TN}\sum_{i=1}^{N}\sum_{t=0}^{T}\delta_T^{-2}\left[g_i^{\mathrm{mod}\,el}(t, I_i^0 = 0)\right]^2 \qquad (9)$$

For the selection of the correct gene network parameter sets, M=5000 independent GA parameter fits were performed for each network described below, each time starting with a random initial parameter set, setting a predefined number of generations as the stopping criterion. Each of these independent runs is ranked according to fitness (Eq. 7) and robustness (see below). The parameter rank score $S_p(i)$ of a fitting run is calculated as

$$S_p(i) = \sqrt{F_N(i)^2 + R_N(i)^2} \qquad (10)$$

where $0 \le F_N \le 1$ and $0 \le R_N \le 1$ denote the normalized model fitness and robustness of a parameter set of a run *i*, respectively. The model parameters are finally computed from the expectation value of the weighted sum of 5% best ranked parameter fits, using the normalized rank scores $S_p(i)$ as weight function. The signal-to-noise ratio (S/N) of each parameter is calculated from the ratio of the expectation value to standard deviation of the above weighted sum.

*Calculation of System Robustness*

Network robustness was determined from a numerical algorithm to calculate the largest Lyapunov exponent (LLE) (Rosenstein *et al*, 1993). Lyapunov exponents quantify the separation of nearby trajectories on an attracting manifold. A negative LLE is the characteristic of dissipative systems, exhibiting asymptotic stability. The more negative the LLE, the more stable the system. A similar Lyapunov exponent-based method has been previously employed to investigate the sensitivity of a signaling pathway to initial conditions (Aldridge *et al*, 2006). Here, the algorithm is used to investigate the local stability of the fixed point of the neural network upon stimulation with the learned input function (cf. Eq. 2). The algorithm calculates the LLE from the simulated time series of a single gene using the method of delays. The LLE $\lambda_L$ is approximated via

$$d_j(i) \approx C_j \exp\left[\lambda_L (i\Delta t)\right] \qquad (11)$$

where $\Delta t$ is the sampling period of the time series, and $d_j(i)$ denotes the distance between the $j^{th}$ pair of simulated expression data at time $i\Delta t$ with a time lag J and an initial separation $C_j$. For the evaluation of the system robustness, the time-averaged divergence of nearby trajectories are used

$$d_{mean} = \left\langle \sum_{j=1}^{M} \ln\left[d_j(i)\right] \right\rangle_T , \qquad (12)$$

where the sum runs over all vectors $M = T-(m-1)J$ from the delay reconstruction of the attractor and the angular brackets $\langle\rangle_T$ denote averaging over the total simulation time.

The more negative the mean divergence, the faster the network settles back into a stable steady state upon stimulation. Hence, systems preferentially return to zero fold expression or take up a stable, activated state within the shortest time.

For sake of computational simplicity, the divergence rather than the Lyapunov exponent is used, which still selects networks according to the desired robustness criteria. Further parameters: embedding dimension $m = 2(N+1)$, where N is the number of network nodes, time lag $J = 1.5h$. Minimal time window for skipping temporally nearest neighbors W=30 hours, which is greater than the longest timescale in the system, as required from the algorithm. Robustness analysis was always performed for the strongest expressed gene *ptgs2*. Changing the time lag, the embedding dimension or the respective gene for analysis did not alter the estimated level of robustness qualitatively.

*Bootstrapping tests*

Time randomization tests were performed to evaluate the correlation of the inverse modeling solution to the gene expression kinetics. 100 independent time randomizations of the gene expression data were performed. Parameters were estimated from the 50 fittest out of 100 independent GA model fits. The correlation between the parameters obtained is computed using the linear Pearson correlation coefficient with respect to the parameter estimates obtained from the full model fitting analysis.

**References**

Aldridge BB, Haller G, Sorger PK, Lauffenburger DA (2006) Direct lyapunov exponent analysis enables parametric study of transient signalling governing cell behaviour. IEE Proc Syst Biol 153: 425-432.

Amit I, Citri A, Shay T, Lu Y, Katz M, Zhang F, Tarcic G, Siwak D, Lahad J, Jacob-Hirsch J, Amariglio N, Vaisman N, Segal E, Rechavi G, Alon U, Mills GB, Domany E, Yarden Y (2007) A module of negative feedback regulators defines growth factor signaling. Nature Genetics 39: 503-512.

Asthagiri AR, Lauffenburger DA (2000) Bioengineering models of cell signaling. Annu Rev Biomed Eng 2: 31-53.

Bansal M, Belcastro V. Ambesi-Impiombato A, di Bernardo D. (2007) How to infer gene networks from expression profiles. Mol. Sys. Biol. 3:78.

Banu N, Buda A, Chell S, Elder D, Moorghen M, Paraskeva C, Qualtrough D, Pignatelli M. (2007) Inhibition of cox-2 with ns-398 decreases colon cancer cell motility through blocking epidermal growth factor receptor transactivation: possibilities for combination therapy. Cell Prolif 40: 768-779.

Baselga J (2006) Targeting Tyrosine Kinases in Cancer: The Second Wave. Science 312:1175-1178.

Beer RD (1995) On the dynamics of small continuous-time recurrent neural networks. Adaptive Behavior 3: 469-509.

Birchmeier C, Birchmeier W, Gherardi E, Woude GFV (2003) Met, metastasis, motility and more. Nat Rev Mol Cell Biol 4: 915-925.

Braunstein S, Kaplan G, Gottlieb AB, Schwartz M, Walsh G, Abalos RM, Fajardo TT, Guido LS, Krueger JG (1994) Gm-csf activates regenerative epidermal growth and stimulates keratinocyte proliferation in human skin in vivo. J Invest Dermatol 103: 601-604.

Burtness B (2007) Her signaling in pancreatic cancer. Expert Opin Biol Ther 7: 823-829

Conesa A, Nueda MJ, Ferrer A, Talón M (2006) maSigPro: a method to identify significantly differential expression profiles in time-course microarray experiments. Bioinformatics 22: 1096-1102.

Davis L (1991) Handbook of Genetic Algorithms. Van Nostrand Reinhold.

Dennis Jr. G, Brad T, Sherman BT, Hosack DA, Yang J, Baseler MW, Lane HC, Lempicki RA (2003) "DAVID: Database for Annotation, Visualization, and Integrated Discovery." Genome Biology 4(5): P3.

D'haeseleer P (2000). Reconstructing gene networks from large scale gene expression data. Ph.D. thesis, University of New Mexico.

Dvorak HF (1986) Tumors: wounds that do not heal. similarities between tumor stroma generation and wound healing. N Engl J Med 315: 1650-1659

Eming SA, Krieg T, Davidson JM (2007) Inflammation in wound repair: molecular and cellular mechanisms. J Invest Dermatol 127: 514-525

Ernst J, Vainas O, Harbison CT, Simon I, Bar-Joseph Z (2007) Reconstructing dynamic regulatory maps. Mol Syst Biol 3: 1

Ferby I, Reschke M, Kudlacek O, Knyazev P, Pantè G, Amann K, Sommergruber W, Kraut N, Ullrich A, Fässler R, Klein R (2006) Mig6 is a negative regulator of egf receptor-mediated skin morphogenesis and tumor formation. Nat Med 12: 568-573

Florin L, Hummerich L, Dittrich BT, Kokocinski F, Wrobel G, Gack S, Schorpp-Kistner M, Werner S, Hahn M, Lichter P, Szabowski A, Angel P (2004) Identification of novel ap-1 target genes in fibroblasts regulated during cutaneous wound healing. Oncogene 23: 7005-7017

Gentleman RC, Carey VJ, Bates DM et al. (2004) Bioconductor: Open software development for computational biology and bioinformatics. Genome Biology 5: R80.

Haken H (2004). Synergetics: Introduction and Advanced Topics. Springer, Berlin.

Hornberg JJ, Bruggeman FJ, Westerhoff HV, Lankelma J (2006). Cancer: a systems biology disease. Biosystems 83: 81-90

Howe AK (2004) Regulation of actin-based cell migration by camp/pka. Biochim Biophys Acta 1692: 159-174

Hu X, Maglia A, Wunsch D (2005). A general recurrent neural network approach to model genetic regulatory networks. In: Engineering in Medicine and Biology Society, 2005. IEEE-EMBS 2005. 27th Annual International Conference of the. 4735-4738

Huber W, von Heydebreck A, Sültmann H, Poustka A, Vingron M (2002) Variance stabilization applied to microarray data calibration and to the quantification of differential expression. Bioinformatics 18 Suppl 1, S96-104.

Janes KA, Gaudet S, Albeck JG, Nielsen UB, Lauffenburger DA Sorger PK (2006) The response of human epithelial cells to TNF involves an inducible autocrine cascade.x

Jette C, Peterson PW, Sandoval IT, Manos EJ, Hadley E, Ireland CM, Jones DA (2004) The tumor suppressor adenomatous polyposis coli and caudal related homeodomain protein regulate expression of retinol dehydrogenase I. J Biol Chem 279: 34397-34405

Kim S, Kim J, Cho KH (2007) Inferring gene regulatory networks from temporal expression profiles under time-delay and noise. Comput Biol Chem 31: 239-245

Krishnan A, Giuliani A, Tomita M (2007). Indeterminacy of reverse engineering of gene regulatory networks: the curse of gene elasticity. PLoS ONE 2: e562

Lauffenburger DA (2000) Cell signaling pathways as control modules: complexity for simplicity? Proc Natl Acad Sci U S A 97: 5031-5033

Li G, Gustafson-Brown C, Hanks SK, Nason K, Arbeit JM, Pogliano K, Wisdom RM, Johnson RS (2003) c-jun is essential for organization of the epidermal leading edge. Dev Cell 4: 865-877

Liao Z, Mason KA, Milas L (2007) Cyclo-oxygenase-2 and its inhibition in cancer: is there a role? Drugs 67: 821-845

Maas-Szabowski N, Szabowski A, Stark HJ, Andrecht S, Kolbus A, Schorpp-Kistner M, Angel P, Fusenig NE (2001) Organotypic cocultures with genetically modified mouse fibroblasts as a tool to dissect molecular mechanisms regulating keratinocyte growth and differentiation. J Invest Dermatol 116: 816-820 Martin P (1997) Wound healing-aiming for perfect skin regeneration. Science 4: 75-81.

McClean MN, Mody A, Broach JR, Ramanathan S (2007) Cross-talk and decision making in MAP kinase pathways. Nature Genetics 39: 409-414

Meinhardt H, Gierer A (2000) Pattern formation by local self-activation and lateral inhibition. Bioessays 22: 753-760

Miller-Jensen K, Janes KA, Brugge JS, Lauffenburger DA (2007) Common effector processing mediates cell-specific responses to stimuli. Nature 448: 1-5

Misteli T (2007) Beyond the Sequence: Cellular Organization of Genome Function. Cell 128: 787-800

Reinitz J, Sharp DH (1995) Mechanism of eve stripe formation. Mech Dev. 49: 133-58

Roeder I, Leonie MK, Braesel K, Dontje B, de Haan G, Loeffler M (2005) Competitive clonal hematopoiesis in mouse chimeras explained by a stochastic model of stem cell organization. Blood 105: 609-616

Nakamura JL (2007) The epidermal growth factor receptor in malignant gliomas: pathogenesis and therapeutic implications. Expert Opin Ther Targets 11: 463-472

Ploplis VA, Tipton H, Menchen H, Castellino FJ (2007) A urokinase-type plasminogen activator deficiency diminishes the frequency of intestinal adenomas in apcmin/+ mice. J Pathol 213: 266-274

R Development Core Team (2007) R: A Language and Environment for Statistical Computing in R Foundation for Statistical Computing, Vienna, Austria.

Rosenstein MT, Collins JJ, Luca CJD (1993) A practical method for calculating largest lyapunov exponents from small data sets. Physica D 65: 117-134

Rüegg C, Dormond O, Mariotti A (2004) Endothelial cell integrins and cox-2: mediators and therapeutic targets of tumor angiogenesis. Biochim Biophys Acta 1654: 51-67

Schreiber M, Kolbus A, Piu F, Szabowski A, Möhle-Steinlein U, Tian J, Karin M, Angel P, Wagner EF. (1999) Control of cell cycle progression by c-Jun is p53 dependent. Genes Dev. 13(5):607-19

Shao J, Evers BM, Sheng H (2004) Prostaglandin e2 synergistically enhances receptor tyrosine kinase-dependent signaling system in colon cancer cells. J Biol Chem 279: 14287-14293.

Sharma SV, Bell DW, Settleman J, Haber DA (2007) Epidermal growth factor receptor mutations in lung cancer. Nat Rev Cancer 7: 169-181.

Stark HJ, Baur M, Breitkreutz D, Fusenig NE (1999) Organotypic keratinocyte cocultures in defined medium with regular epidermal morphogenesis and differentiation. J. Invest. Dermatol. 112: 681-691

Stolovitzky G, Monroe D, Califano A (2007) Dialogue on Reverse-Engineering Assessment and Methods: The DREAM of High-Throughput Pathway Inference. In: Workshop on Dialogue on Reverse Engineering Assessment and Methods (DREAM), Ann. N.Y. Acad. Sci. 1115: 1-23

Stramer B, Martin P (2005) Cell biology: master regulators of sealing and healing. Curr Biol 15: R425-R427

Szabowski A, Maas-Szabowski N, Andrecht S, Kolbus A, Schorpp-Kistner M, Fusenig NE, Angel P (2000) c-jun and junb antagonistically control cytokine-regulated mesenchymal-epidermal interaction in skin. Ce// 103: 745-755

Tunaru S, Kero J, Schaub A, Wufka C, Blaukat A, Pfeffer K, Offermanns S (2003) Puma-g and hm74 are receptors for nicotinic acid and mediate its anti-lipolytic effect. Nat Med 9: 352-355

Vohradsky J (2001) Neural model of the genetic network. J Biol Chem 276: 36168-36173

Werner S, Smola H, Liao X, Longaker MT, Krieg T, Hofschneider PH, Williams LT (1994) The function of kgf in morphogenesis of epithelium and reepithelialization of wounds. Science 266: 819-822

Wick M, Bürger C, Funk M, Müller R (1995) Identification of a novel mitogen-inducible gene (mig-6): regulation during g1 progression and differentiation. Exp Cell Res 219: 527-535

Wilson CL, Miller CJ (2005) Simpleaffy: a BioConductor package for Affymetrix Quality Control and data analysis. Bioinformatics. 21: 3683-3685

Xu, KP, Yu FSX (2007) Cross talk between Met and epidermal growth factor receptor during retinal pigment epithelial wound healing. Invest Ophthalmol Vis Sci 48: 2242-2248.

Yeang CH, Mak HC, McCuine S, Workman C, Jaakkola T, Ideker T (2005) Validation and refinement of gene-regulatory pathways on a network of physical interactions. Genome Biol. 6: R62.

Zenz R, Scheuch H, Martin P, Frank C, Eferl R, Kenner L, Sibilia M, Wagner EF (2003) c-jun regulates eyelid closure and skin tumor development through egfr signaling. Dev Cell 4: 879-889.

Zhou HY, Pon YL, Wong AST (2007) Synergistic effects of epidermal growth factor and hepatocyte growth factor on human ovarian cancer cell invasion and migration: role of extracellular signal-regulated kinase 1/2 and p38 mitogen-activated protein kinase. Endocrinology 148: 5195-5208.

**Example 2: Differentiation between the metastasising potential of two cell lines in vivo**

Two different cell lines derived from colon carcinomas are to be detected. The first cell line will display low mobility in a cell migration assay whereas the second one will display high mobility. The mobility of the cells in the assay will serve as an indicator of their metastasising potential. The expression levels of GPR109B, DHRS9, ERRFI-1 and PTGS-2 will be determined in both cell lines with DNA-microarrays. From the measured gene expression levels the following ratios will be calculated: GPR109B and PTGS-2, DHRS9 and PTGS-2 and of ERRFI-1 and PTGS-2. It will be shown that at least one of the calculated ratios is significantly increased in cells of the cell line that scores high in the migration assay than cells of the cell line that scores low. A significant increase will be determined by applying Student's t-test.

## Claims

**1.** A method of diagnosing whether a solid tumor in a subject has metastasising potential comprising the steps of:

(a) determining in a sample of a subject suspected to suffer from a solid tumor the expression levels of the marker genes GPR109B, DHRS9, and ERRFI-1 and the expression level of a reference gene, and
(b) calculating at least one variable from one or more of said marker genes and the reference gene; and
(c) comparing the variable calculated in step b) to a reference variable, whereby it is to be diagnosed whether the solid tumor has metastasising potential.

**2.** The method of claim 1, wherein the reference gene is PTGS-2.

**3.** The method of claim 1 or 2, wherein

(a) the expression level of the marker gene Ceacam-1 is further determined; and
(b) a variable is calculated from the expression levels of CEACAM-1 and the reference gene.

**4.** The method of claim 1 to 3, wherein

(a) the expression level of the marker gene SPOCD1 is further determined; and
(b) a variable is calculated from the expression levels of SPOCD1 and the reference gene.

**5.** The method of any one of claims 1 to 4, wherein the reference variables are the variables calculated from the expression levels of marker genes and reference gene in a sample of a subject known to not suffer from a solid tumor having metastasising potential.

**6.** The method of claim 5, wherein the variable being different from the reference variable is indicative for a subject suffering from a solid tumor having metastasising potential.

**7.** The method of any one of claims 1 to 4, wherein the reference variable is the variable calculated from the marker genes and the reference gene in a subject known to suffer from a solid tumor having metastasising potential.

**8.** The method of claim 7, wherein the variable being essentially identical to the reference variable is indicative for a subject suffering from a solid tumor having metastasising potential.

**9.** A method of determining whether a therapy against a solid tumor having metastasising potential is successful comprising:

(a) determining in a first sample of a subject suffering from a solid tumor having metastasising potential obtained prior to the onset of the therapy the expression levels of GPR109B, DHRS9, ERRFI-1 and the reference gene,
(b) calculating at least one variable from one or more of said marker genes and the reference gene; and
(c) determining in a second sample of the said subject obtained after the onset of the therapy the variable of b); and
(d) comparing the variable calculated in step b) to the variable calculated in step c), wherein a difference of the variables is indicative for a successful therapy against a solid tumor having metastasising potential.

**10.** The method of claim 9, wherein

(a) the expression level of the marker gene CEACAM-1 is further determined; and
(b) a variable is calculated from the expression levels of CEACAM-1 and the reference gene

**11.** The method of claim 9 or 10, wherein

(a) the expression level of the marker gene SPOCD1 is further determined; and
(b) a variable is calculated from the expression levels of SPOCD1 and the reference gene.

**12.** The method of any one of claims 1 to 11, wherein the expression levels are determined based on the amount of transcribed polynucleotides of the marker genes and the reference gene present in the said sample.

**13.** The method of any one of claims 1 to 11, wherein the expression levels are determined based on the amount of polypeptides encoded by the marker genes and the reference gene present in the said sample.

**14.** The method of any one of claims 1 to 13, wherein said subject is a human.

**15.** The method of any one of claims 1 to 14, wherein said sample is a biopsy sample of the said solid tumor.

**16.** A solid support comprising polynucleotides or polypeptides of the marker genes and the reference gene referred to in anyone of claims 1 to 4 or means for the determination of such polynucleotides or polypeptides.

**17.** A device adapted for carrying out the method of any one of claims 1 to 15 comprising

(a) means for determining the expression levels of the marker genes and the reference gene referred to in any one of claims 1 to 4 in a sample; and
(b) means for calculating at least one variable from one or more of said marker genes and the reference gene; and
(c) means for comparing the variable calculated by the means of b) with a reference variable.

**18.** Use of the marker genes and the reference gene referred to in any one of claims 1 to 4 or means for determining their expression levels in a sample for the manufacture of a diagnostic composition for diagnosing a solid tumor having metastasising potential in a subject.

**19.** Use of the marker genes and the reference gene referred to in any one of claims 1 to 4 or means for determining their expression levels in a sample for the manufacture of a diagnostic composition for determining whether a therapy against a solid tumor having metastasising potential is successful.

**20.** A kit comprising the marker genes and the reference gene referred to in any one of claims 1 to 4 or means for determining their expression levels or the solid support of claim 12.

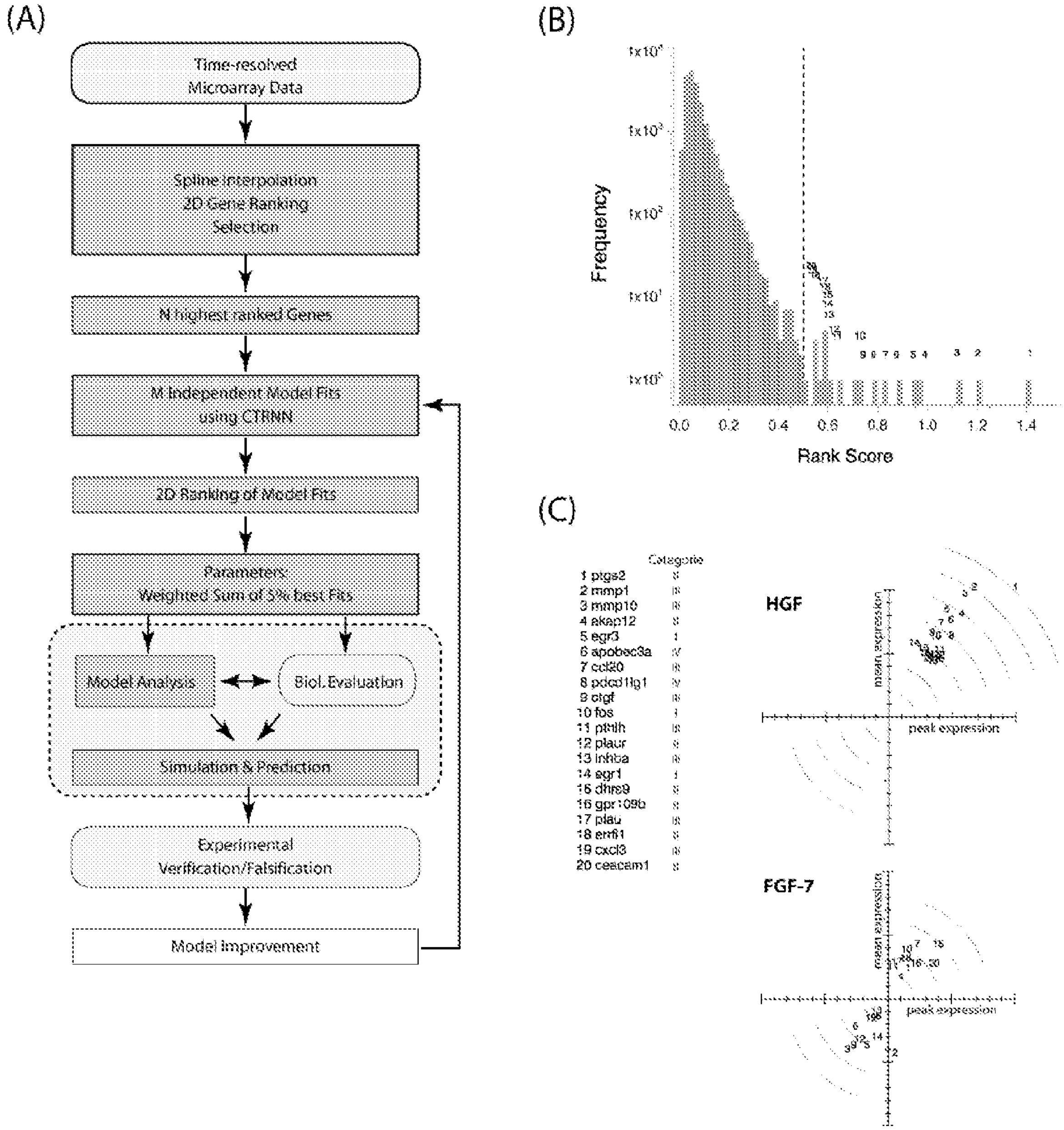
(A)
Time-resolved Microarray Data
Spline interpolation 2D Gene Ranking Selection
N highest ranked Genes
M Independent Model Fits using CTRNN
2D Ranking of Model Fits
Parameters: Weighted Sum of 5% best Fits
Model Analysis
Biol. Evaluation
Simulation & Prediction
Experimental Verification/Falsification
Model Improvement
(B)
Frequency
Rank Score
0.0
0.2
0.4
0.6
0.8
1.0
1.2
1.4
(C)
HGF
FGF-7
mean expression
peak expression

Fig. 1

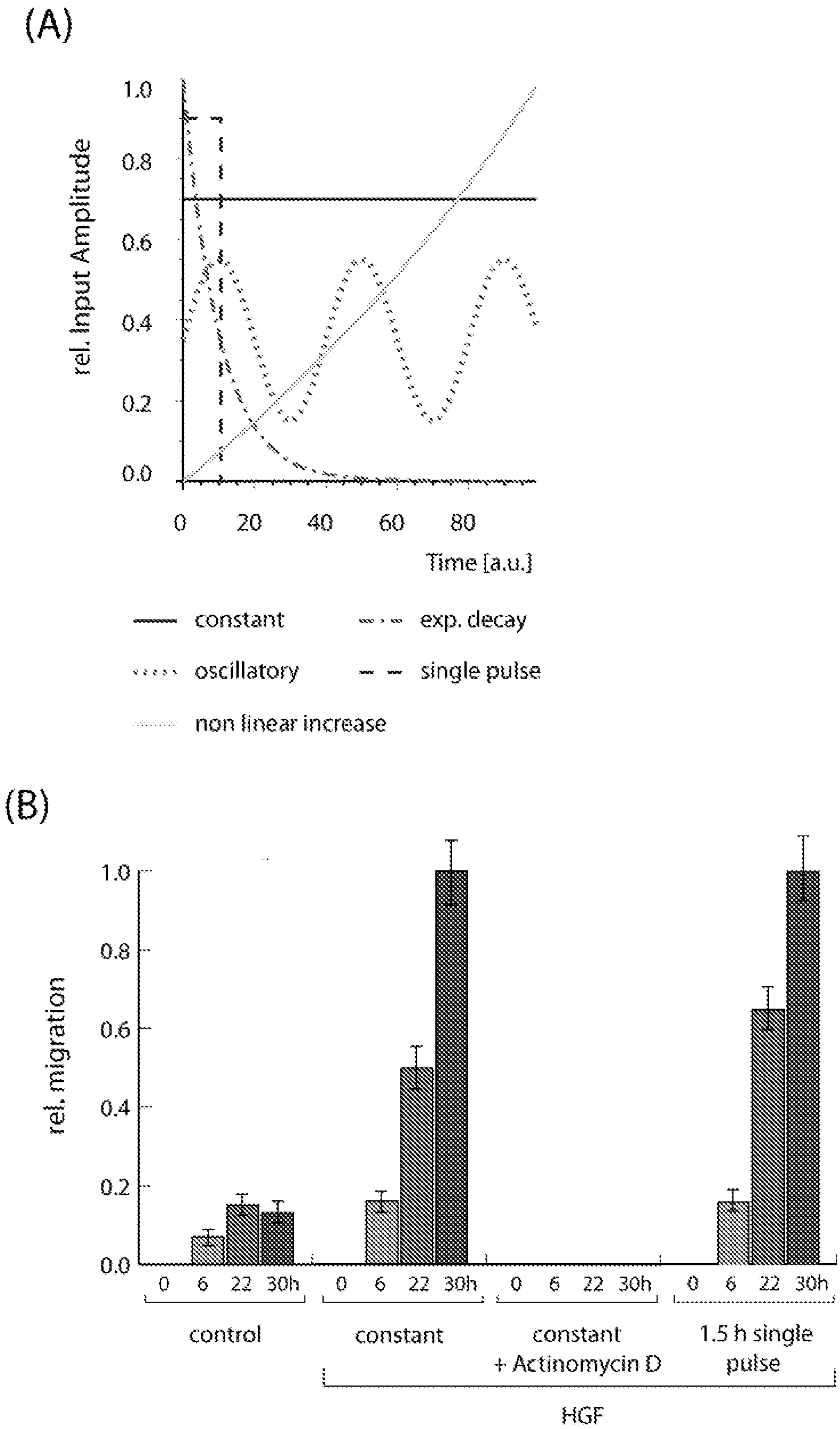
(A)
1.0
0.8
0.6
0.4
0.2
0.0
rel. Input Amplitude
0
20
40
60
80
Time [a.u.]
constant
exp. decay
oscillatory
single pulse
non linear increase
(B)
1.0
0.8
0.6
0.4
0.2
0.0
rel. migration
0 6 22 30h
0 6 22 30h
0 6 22 30h
0 6 22 30h
control
constant
constant + Actinomycin D
1.5 h single pulse
HGF

Fig. 2

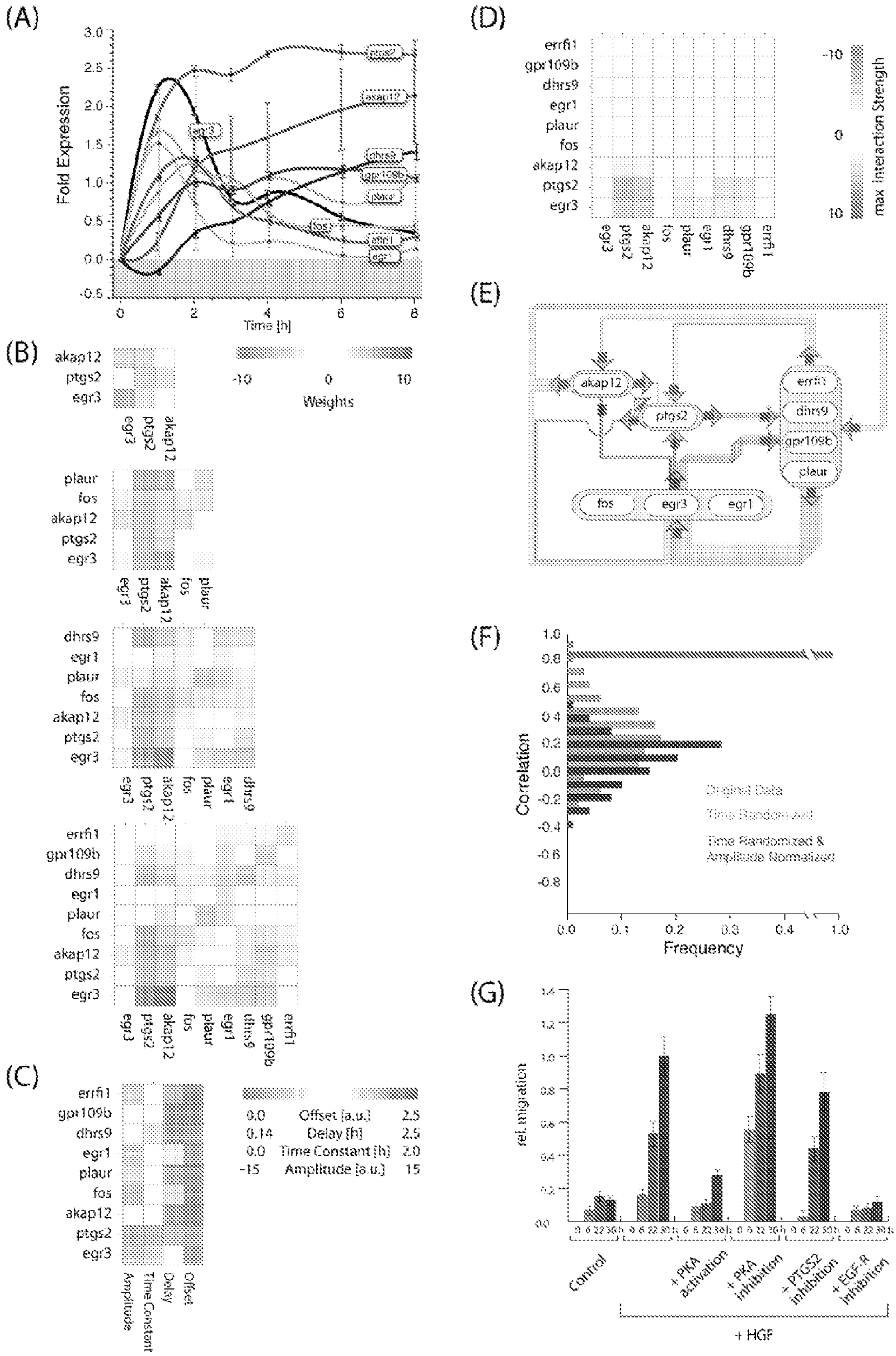
(A)
Fold Expression
Time [h]
(B)
-10
0
10
Weights
(C)
Offset [a.u.]
Delay [h]
Time Constant [h]
Amplitude [a.u.]
(D)
max Interaction Strength
(E)
(F)
Correlation
Frequency
Original Data
Time Randomized
Time Randomized &
Amplitude Normalized
(G)
rel. migration
Control
+ PKA activation
+ PKA inhibition
+ PTGS2 inhibition
+ EGF-R inhibition
+ HGF

Fig. 3

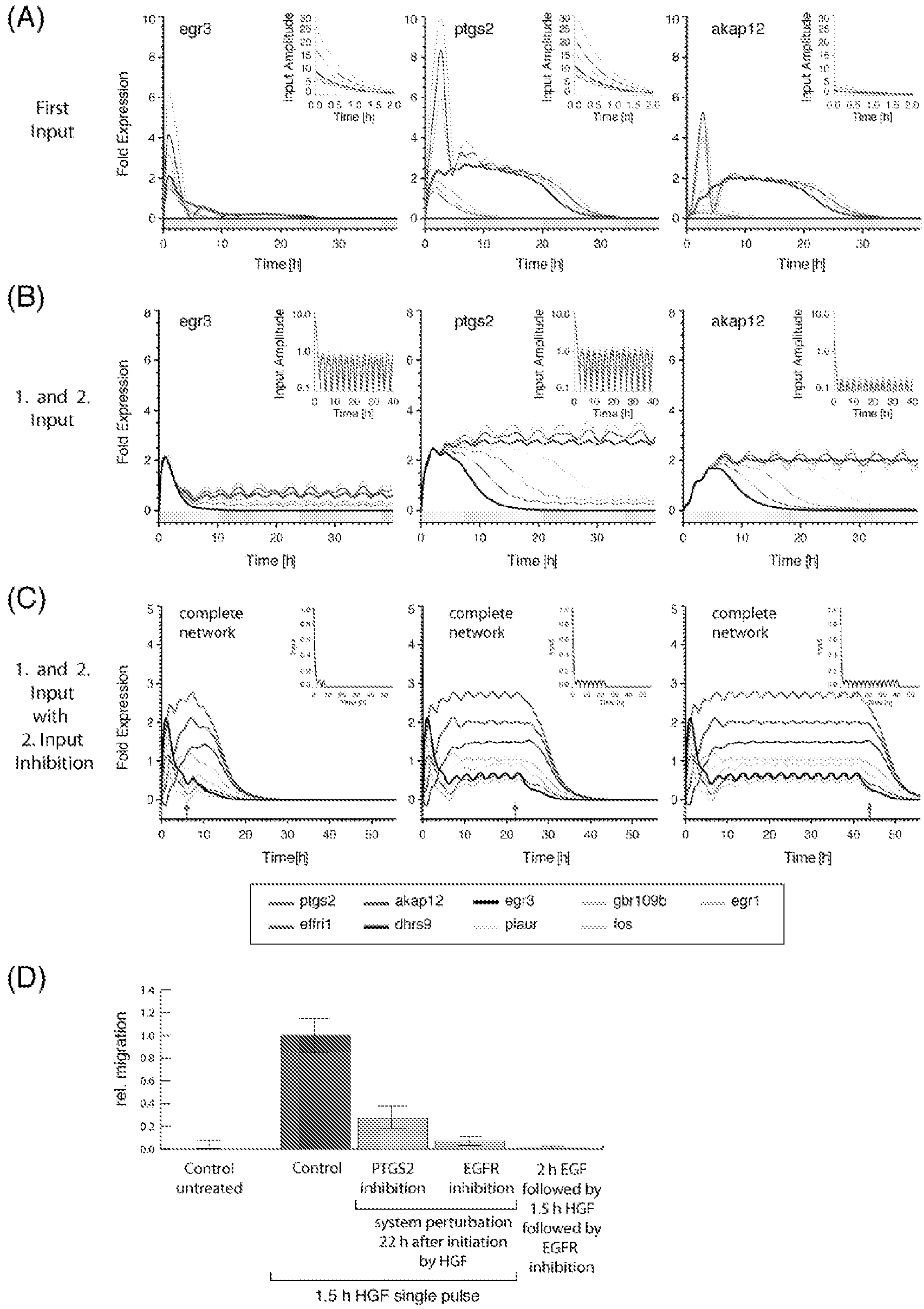
(A)
First Input
egr3
ptgs2
akap12
Fold Expression
Input Amplitude
Time [h]
(B)
1. and 2. Input
egr3
ptgs2
akap12
Fold Expression
Input Amplitude
Time [h]
(C)
1. and 2. Input with 2. Input Inhibition
complete network
Fold Expression
Time[h]
ptgs2
akap12
egr3
gbr109b
egr1
effri1
dhrs9
plaur
fos
(D)
rel. migration
Control untreated
Control
PTGS2 inhibition
EGFR inhibition
system perturbation 22 h after initiation by HGF
2 h EGF followed by 1.5 h HGF followed by EGFR inhibition
1.5 h HGF single pulse

Fig. 4

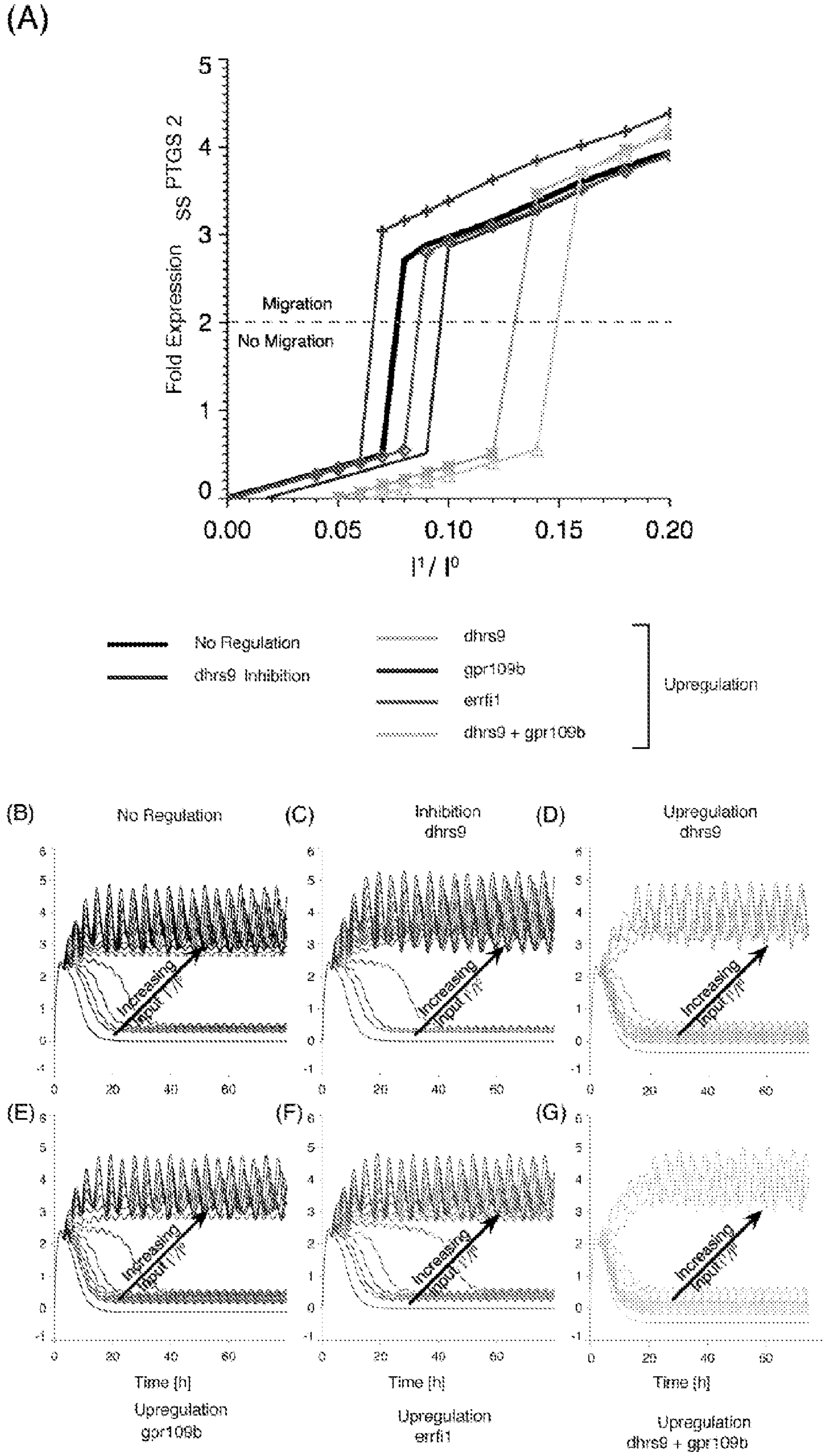
(A)
Fold Expression SS PTGS 2
Migration
No Migration
$I^1 / I^0$
No Regulation
dhrs9 Inhibition
dhrs9
gpr109b
errfi1
dhrs9 + gpr109b
Upregulation
(B)
No Regulation
(C)
Inhibition
dhrs9
(D)
Upregulation
dhrs9
Increasing input $I^1/I^0$
(E)
(F)
(G)
Time [h]
Upregulation
gpr109b
Upregulation
errfi1
Upregulation
dhrs9 + gpr109b

Fig. 5

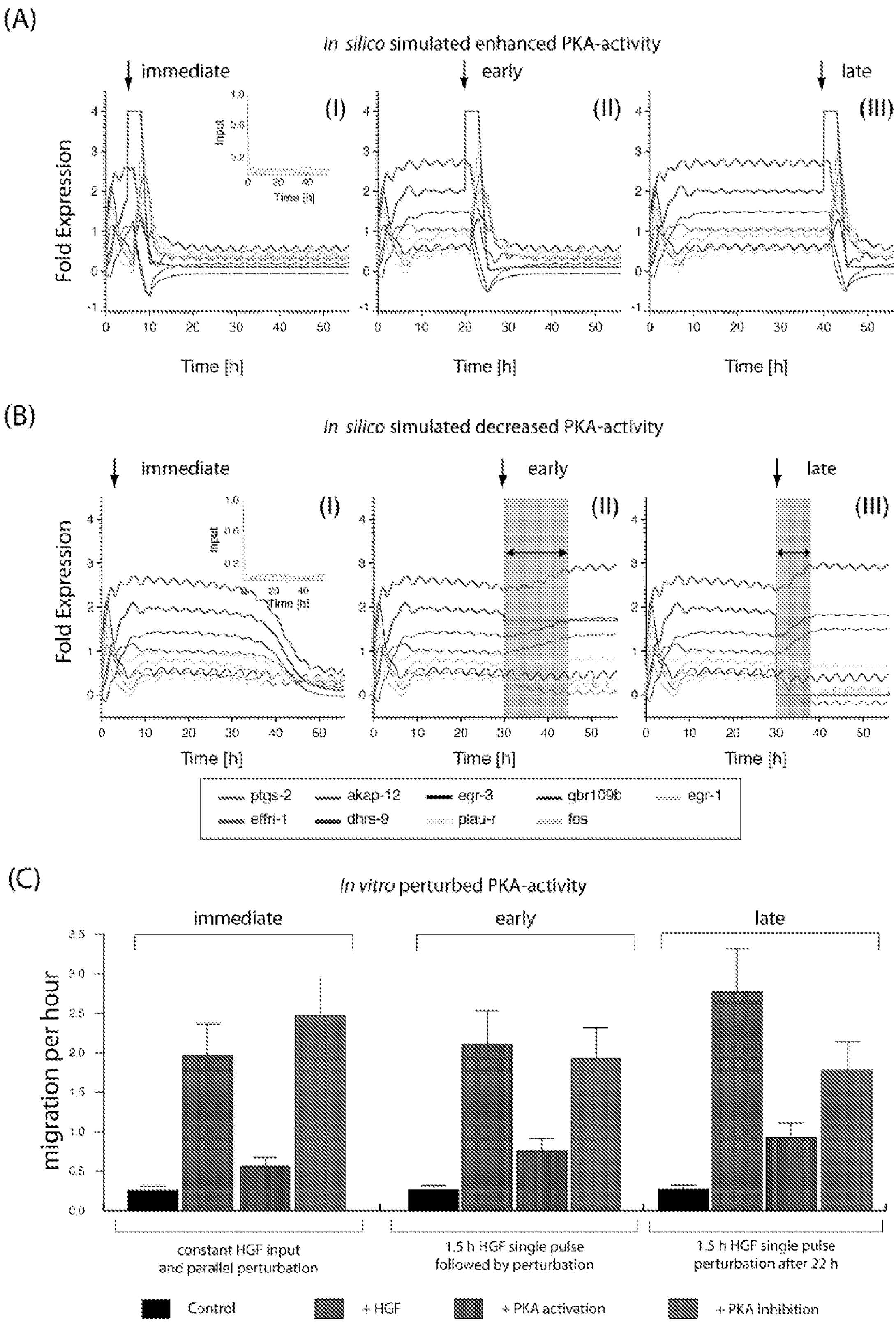
(A)
In silico simulated enhanced PKA-activity
immediate
early
late
(I)
(II)
(III)
Fold Expression
Time [h]
(B)
In silico simulated decreased PKA-activity
immediate
early
late
Fold Expression
Time [h]
ptgs-2
akap-12
egr-3
gbr109b
egr-1
effri-1
dhrs-9
plau-r
fos
(C)
In vitro perturbed PKA-activity
immediate
early
late
migration per hour
constant HGF input and parallel perturbation
1.5 h HGF single pulse followed by perturbation
1.5 h HGF single pulse perturbation after 22 h
Control
+ HGF
+ PKA activation
+ PKA inhibition

Fig. 6

(A)

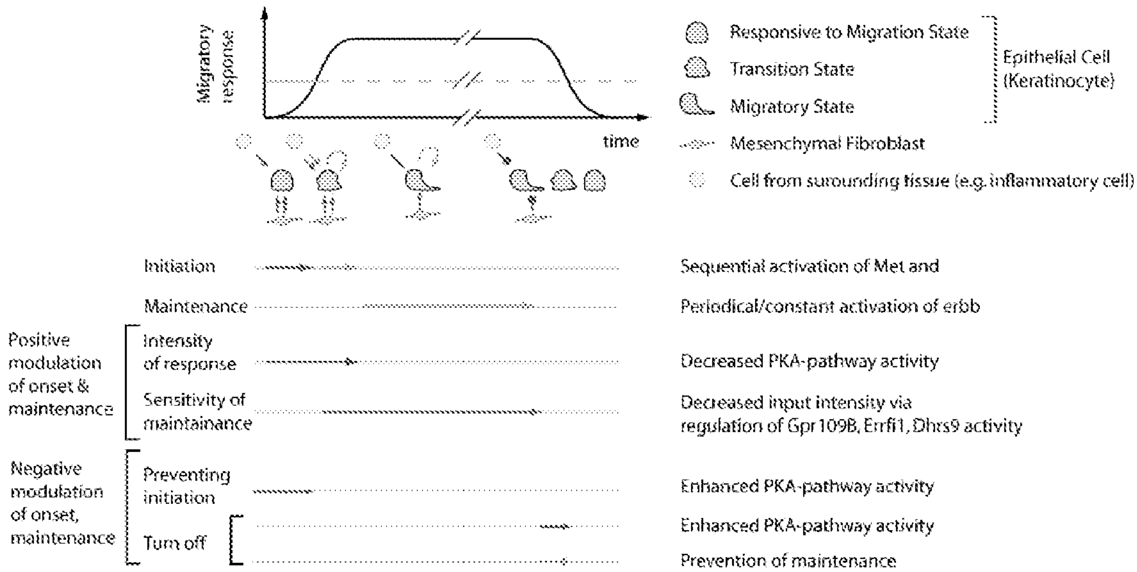
Migratory response
time
Responsive to Migration State
Transition State
Migratory State
Epithelial Cell (Keratinocyte)
Mesenchymal Fibroblast
Cell from surounding tissue (e.g. inflammatory cell)
Initiation
Maintenance
Positive modulation of onset & maintenance
Intensity of response
Sensitivity of maintainance
Negative modulation of onset, maintenance
Preventing initiation
Turn off
Sequential activation of Met and
Periodical/constant activation of erbb
Decreased PKA-pathway activity
Decreased input intensity via regulation of Gpr109B, Errfi1, Dhrs9 activity
Enhanced PKA-pathway activity
Enhanced PKA-pathway activity
Prevention of maintenance

(B)

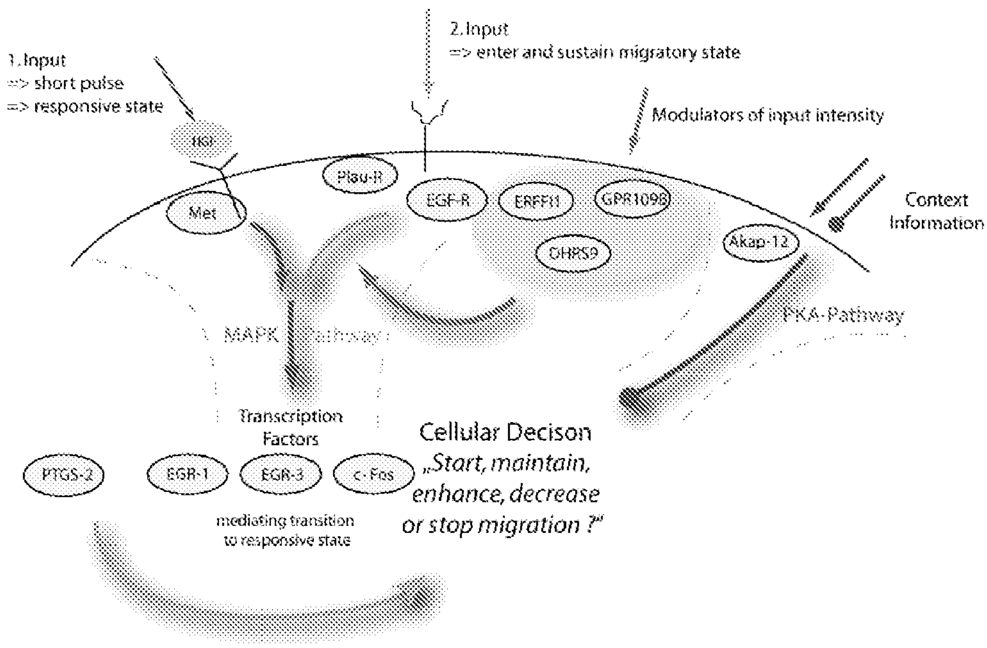
1. Input
=> short pulse
=> responsive state
2. Input
=> enter and sustain migratory state
Modulators of input intensity
HGF
Plau-R
Met
EGF-R
ERFFI1
GPR109B
DHRS9
Akap-12
Context Information
MAPK Pathway
PKA-Pathway
Transcription Factors
PTGS-2
EGR-1
EGR-3
c-Fos
mediating transition to responsive state
Cellular Decison
„Start, maintain, enhance, decrease or stop migration ?"

Fig. 7

Europäisches Patentamt
European Patent Office
Office européen des brevets

# EUROPEAN SEARCH REPORT

Application Number
EP 08 15 7191

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2004/098521 A (TAIGEN BIOTECHNOLOGY [CN]; CHEN HUA-CHIEN; SUN YING [GB]; HUANG YING-H) 18 November 2004 (2004-11-18)<br>* page 1, line 22 - page 1, line 25; claims 1-3; table 1 *<br>----- | 1-20 | INV.<br>G01N33/574<br>C12N15/10 |
| A | US 2007/099209 A1 (CLARKE MICHAEL F [US] ET AL) 3 May 2007 (2007-05-03)<br>* page 1, column 1, paragraph 5 *<br>* page 2, column 2, paragraph 17; tables 9-13,17,26 *<br>* page 3, column 1, paragraph 21 *<br>----- | 1-20 | |
| L | BUSCH HAUKE ET AL: "Gene network dynamics controlling keratinocyte migration."<br>MOLECULAR SYSTEMS BIOLOGY 2008,<br>vol. 4, 2008, page 199, XP002497432<br>ISSN: 1744-4292<br>* page 2, column 2, line 10 - page 2, column 2, line 12; table 3B *<br>* page 5, column 1, line 12 - page 5, column 1, line 17 *<br>----- | 1-20 | TECHNICAL FIELDS SEARCHED (IPC)<br>G01N<br>C12N |

The present search report has been drawn up for all claims

| Place of search | Date of completion of the search | Examiner |
|---|---|---|
| Munich | 26 September 2008 | Landré, Julien |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

4

EPO FORM 1503 03.82 (P04C01)

**ANNEX TO THE EUROPEAN SEARCH REPORT ON EUROPEAN PATENT APPLICATION NO.** EP 08 15 7191

This annex lists the patent family members relating to the patent documents cited in the above-mentioned European search report. The members are as contained in the European Patent Office EDP file on
The European Patent Office is in no way liable for these particulars which are merely given for the purpose of information.

26-09-2008

| Patent document cited in search report | | Publication date | Patent family member(s) | Publication date |
|---|---|---|---|---|
| WO 2004098521 | A | 18-11-2004 | NONE | |
| US 2007099209 | A1 | 03-05-2007 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **Dowdy ; Wearden.** Statistics for Research. John Wiley & Sons, 1983
- **Toulza.** *Genome Biol.,* 2007, vol. 8 (6), R107
- **Soga.** *Biochem Biophys Res Commun,* 2003, vol. 303 (1), 364-369
- **Wise.** *J Biol Chem,* 2003, vol. 278 (11), 9869-9874
- **Tunaru.** *Nat Med,* 2003, vol. 9 (3), 352-355
- **Irobi.** *J Peripher Nerv Syt,* 2002, vol. 7 (2), 87-95
- **Zingoni.** *Genomics,* 1997, vol. 42 (3), 519-523
- **Nomura.** *Int Immunol,* 1993, vol. 5 (10), 1239-1249
- *J. Mol. Evolution.,* 1987, vol. 25, 351-360
- **Higgins et al.** *CABIOS,* 1989, vol. 5, 151-153
- **Needleman ; Wunsch.** *J. Mol. Biol.,* 1970, vol. 48, 443-453
- **Smith ; Waterman.** *Adv. Appl. Math.,* 1981, vol. 2, 482-489
- Genetics Computer Group. *575 Science Drive,* 1991, 53711
- **Jones.** *J Biol Chem,* 2007, vol. 282 (11), 8317-24
- **Jette.** *J Biol Chem,* 2004, vol. 279 (33), 34397-405
- **Clark.** *Genome Res,* 2003, vol. 13 (10), 2265-2270
- **Markova.** *Mol Genet Metab,* 2003, vol. 78 (2), 119-135
- **Soref.** *J Biol Chem,* 2001, vol. 276 (26), 24194-202
- **Chetyrkin.** *J Biol Chem,* 2000, vol. 276 (25), 22278-286
- **Haeseleer.** *Meth Enzymol,* vol. 316, 372-383
- **Zhang.** *Cell Cycle,* 2007, vol. 6 (5), 507-513
- **Gregory.** *Nature,* 2006, vol. 441/7091, 315-321
- **Anastasi.** *Oncogene,* 2005, vol. 24 (28), 4540-4548
- **Hackel.** *Biol Chem,* 2001, vol. 382 (12), 1649-62
- **Wick.** *Exp. Cell Res,* 1995, vol. 219 (2), 527-535
- **Klaile.** *J Biol Chem,* 2007, vol. 282 (36), 26629-26640
- **Lee.** *Infect Immun,* 2007, vol. 75 (9), 4449-4455
- **Hokari.** *Life Sci,* 2007, vol. 81 (4), 336-345
- **N'Guessan.** *J Infect Dis,* 2007, vol. 195 (11), 1651-1660
- **Yu.** *J Biol Chem,* 2006, vol. 281 (51), 39179-39193
- **Thompson.** *Genomics,* 1992, vol. 12 (4), 761-772
- **Kuroki.** *Biochem Biophys Res Commun,* 1991, vol. 176 (2), 578-585
- **Barnett.** *J Cell Biol,* vol. 108 (2), 267-276
- **Hinoda.** *Proc Natl Acad Sci USA,* 1988, vol. 85 (18), 6959-6963
- **Svenberg.** *Clin Exp Immunol,* 1979, vol. 36 (3), 436-441
- **Barnett.** *J Cell Biol,* 1989, vol. 108 (2), 267-276
- *IEE Proc Syst Biol,* vol. 153, 425-432
- **Amit I ; Citri A ; Shay T ; Lu Y ; Katz M ; Zhang F ; Tarcic G ; Siwak D ; Lahad J ; Jacob-Hirsch J.** A module of negative feedback regulators defines growth factor signaling. *Nature Genetics,* vol. 39, 503-512
- **Asthagiri AR ; Lauffenburger DA.** Bioengineering models of cell signaling. *Annu Rev Biomed Eng,* 2000, vol. 2, 31-53
- **Bansal M ; Belcastro V ; Ambesi-Impiombato A ; di Bernardo D.** How to infer gene networks from expression profiles. *Mol. Sys. Biol.,* 2007, vol. 3, 78
- **Banu N ; Buda A ; Chell S ; Elder D ; Moorghen M ; Paraskeva C ; Qualtrough D ; Pignatelli M.** Inhibition of cox-2 with ns-398 decreases colon cancer cell motility through blocking epidermal growth factor receptor transactivation: possibilities for combination therapy. *Cell Prolif,* 2007, vol. 40, 768-779
- **Baselga J.** Targeting Tyrosine Kinases in Cancer: The Second Wave. *Science,* 2006, vol. 312, 1175-1178
- **Beer RD.** On the dynamics of small continuous-time recurrent neural networks. *Adaptive Behavior,* 1995, vol. 3, 469-509
- **Birchmeier C ; Birchmeier W ; Gherardi E ; Woude GFV.** Met, metastasis, motility and more. *Nat Rev Mol Cell Biol,* 2003, vol. 4, 915-925

- **Braunstein S ; Kaplan G ; Gottlieb AB ; Schwartz M ; Walsh G ; Abalos RM ; Fajardo TT ; Guido LS ; Krueger JG.** Gm-csf activates regenerative epidermal growth and stimulates keratinocyte proliferation in human skin in vivo. *J Invest Dermatol,* 1994, vol. 103, 601-604
- **Burtness B.** Her signaling in pancreatic cancer. *Expert Opin Biol Ther,* 2007, vol. 7, 823-829
- **Conesa A ; Nueda MJ ; Ferrer A ; Talón M.** maSigPro: a method to identify significantly differential expression profiles in time-course microarray experiments. *Bioinformatics,* 2006, vol. 22, 1096-1102
- **Davis L.** Handbook of Genetic Algorithms. Van Nostrand Reinhold. 1991
- **Dennis Jr. G ; Brad T ; Sherman BT ; Hosack DA ; Yang J ; Baseler MW ; Lane HC ; Lempicki RA.** DAVID: Database for Annotation, Visualization, and Integrated Discovery. *Genome Biology,* vol. 4 (5), 3
- **D'haeseleer P.** Reconstructing gene networks from large scale gene expression data. *Ph.D. thesis,* 2000
- **Dvorak HF.** Tumors: wounds that do not heal. similarities between tumor stroma generation and wound healing. *N Engl J Med,* 1986, vol. 315, 1650-1659
- **Eming SA ; Krieg T ; Davidson JM.** Inflammation in wound repair: molecular and cellular mechanisms. *J Invest Dermatol,* 2007, vol. 127, 514-525
- **Ernst J ; Vainas O ; Harbison CT ; Simon I ; Bar-Joseph Z.** Reconstructing dynamic regulatory maps. *Mol Syst Biol,* 2007, vol. 3, 1
- **Ferby I ; Reschke M ; Kudlacek O ; Knyazev P ; Pantè G ; Amann K ; Sommergruber W ; Kraut N ; Ullrich A ; Fässler R.** Mig6 is a negative regulator of egf receptor-mediated skin morphogenesis and tumor formation. *Nat Med,* 2006, vol. 12, 568-573
- **Florin L ; Hummerich L ; Dittrich BT ; Kokocinski F ; Wrobel G ; Gack S ; Schorpp-Kistner M ; Werner S ; Hahn M ; Lichter P.** Identification of novel ap-1 target genes in fibroblasts regulated during cutaneous wound healing. *Oncogene,* 2004, vol. 23, 7005-7017
- **Gentleman RC ; Carey VJ ; Bates DM et al.** Bioconductor: Open software development for computational biology and bioinformatics. *Genome Biology,* 2004, vol. 5, R80
- **Haken H.** Synergetics: Introduction and Advanced Topics. Springer, 2004
- **Hornberg JJ ; Bruggeman FJ ; Westerhoff HV ; Lankelma J.** Cancer: a systems biology disease. *Biosystems,* 2006, vol. 83, 81-90
- **Howe AK.** Regulation of actin-based cell migration by camp/pka. *Biochim Biophys Acta,* 2004, vol. 1692, 159-174
- **Hu X ; Maglia A ; Wunsch D.** A general recurrent neural network approach to model genetic regulatory networks. *Engineering in Medicine and Biology Society, 2005. IEEE-EMBS 2005. 27th Annual International Conference,* 2005, 4735-4738
- **Huber W ; von Heydebreck A ; Sültmann H ; Poustka A ; Vingron M.** Variance stabilization applied to microarray data calibration and to the quantification of differential expression. *Bioinformatics,* 2002, vol. 18 (1), 96-104
- **Janes KA ; Gaudet S ; Albeck JG ; Nielsen UB ; Lauffenburger DA ; Sorger PK.** *The response of human epithelial cells to TNF involves an inducible autocrine cascade.x,* 2006
- **Jette C ; Peterson PW ; Sandoval IT ; Manos EJ ; Hadley E ; Ireland CM ; Jones DA.** The tumor suppressor adenomatous polyposis coli and caudal related homeodomain protein regulate expression of retinol dehydrogenase I. *J Biol Chem,* 2004, vol. 279, 34397-34405
- **Kim S ; Kim J ; Cho KH.** Inferring gene regulatory networks from temporal expression profiles under time-delay and noise. *Comput Biol Chem,* 2007, vol. 31, 239-245
- **Krishnan A ; Giuliani A ; Tomita M.** Indeterminacy of reverse engineering of gene regulatory networks: the curse of gene elasticity. *PLoS ONE,* 2007, vol. 2, e562
- **Lauffenburger DA.** Cell signaling pathways as control modules: complexity for simplicity?. *Proc Natl Acad Sci U S A,* 2000, vol. 97, 5031-5033
- **Li G ; Gustafson-Brown C ; Hanks SK ; Nason K ; Arbeit JM ; Pogliano K ; Wisdom RM ; Johnson RS.** c-jun is essential for organization of the epidermal leading edge. *Dev Cell,* 2003, vol. 4, 865-877
- **Liao Z ; Mason KA ; Milas L.** Cyclo-oxygenase-2 and its inhibition in cancer: is there a role?. *Drugs,* 2007, vol. 67, 821-845
- **Maas-Szabowski N ; Szabowski A ; Stark HJ ; Andrecht S ; Kolbus A ; Schorpp-Kistner M ; Angel P ; Fusenig NE.** Organotypic cocultures with genetically modified mouse fibroblasts as a tool to dissect molecular mechanisms regulating keratinocyte growth and differentiation. *J Invest Dermatol,* 2001, vol. 116, 816-820
- **Martin P.** Wound healing- aiming for perfect skin regeneration. *Science,* 1997, vol. 4, 75-81
- **McClean MN ; Mody A ; Broach JR ; Ramanathan S.** Cross-talk and decision making in MAP kinase pathways. *Nature Genetics,* 2007, vol. 39, 409-414
- **Meinhardt H ; Gierer A.** Pattern formation by local self-activation and lateral inhibition. *Bioessays,* 2000, vol. 22, 753-760
- **Miller-Jensen K ; Janes KA ; Brugge JS ; Lauffenburger DA.** Common effector processing mediates cell-specific responses to stimuli. *Nature,* 2007, vol. 448, 1-5

- **Misteli T.** Beyond the Sequence: Cellular Organization of Genome Function. *Cell,* 2007, vol. 128, 787-800
- **Reinitz J ; Sharp DH.** Mechanism of eve stripe formation. *Mech Dev.,* 1995, vol. 49, 133-58
- **Roeder I ; Leonie MK ; Braesel K ; Dontje B ; de Haan G ; Loeffler M.** Competitive clonal hematopoiesis in mouse chimeras explained by a stochastic model of stem cell organization. *Blood,* 2005, vol. 105, 609-616
- **Nakamura JL.** The epidermal growth factor receptor in malignant gliomas: pathogenesis and therapeutic implications. *Expert Opin Ther Targets,* 2007, vol. 11, 463-472
- **Ploplis VA ; Tipton H ; Menchen H ; Castellino FJ.** A urokinase-type plasminogen activator deficiency diminishes the frequency of intestinal adenomas in apcmin/+ mice. *J Pathol,* 2007, vol. 213, 266-274
- R Development Core Team (2007) R: A Language and Environment for Statistical Computing. *R Foundation for Statistical Computing,* 2007
- **Rosenstein MT ; Collins JJ ; Luca CJD.** A practical method for calculating largest lyapunov exponents from small data sets. *Physica D,* 1993, vol. 65, 117-134
- **Rüegg C ; Dormond O ; Mariotti A.** Endothelial cell integrins and cox-2: mediators and therapeutic targets of tumor angiogenesis. *Biochim Biophys Acta,* 2004, vol. 1654, 51-67
- **Schreiber M ; Kolbus A ; Piu F, Szabowski A ; Möhle-Steinlein U ; Tian J ; Karin M ; Angel P ; Wagner EF.** Control of cell cycle progression by c-Jun is p53 dependent. *Genes Dev.,* 1999, vol. 13 (5), 607-19
- **Shao J ; Evers BM ; Sheng H.** Prostaglandin e2 synergistically enhances receptor tyrosine kinase-dependent signaling system in colon cancer cells. *J Biol Chem,* 2004, vol. 279, 14287-14293
- **Sharma SV ; Bell DW ; Settleman J ; Haber DA.** Epidermal growth factor receptor mutations in lung cancer. *Nat Rev Cancer,* 2007, vol. 7, 169-181
- **Stark HJ ; Baur M ; Breitkreutz D ; Fusenig NE.** Organotypic keratinocyte cocultures in defined medium with regular epidermal morphogenesis and differentiation. *J. Invest. Dermatol.,* 1999, vol. 112, 681-691
- **Stolovitzky G ; Monroe D ; Califano A.** Dialogue on Reverse-Engineering Assessment and Methods: The DREAM of High-Throughput Pathway Inference. In: Workshop on Dialogue on Reverse Engineering Assessment and Methods (DREAM). *Ann. N.Y. Acad. Sci.,* 2007, vol. 1115, 1-23
- **Stramer B ; Martin P.** Cell biology: master regulators of sealing and healing. *Curr Biol,* 2005, vol. 15, R425-R427
- **Szabowski A ; Maas-Szabowski N ; Andrecht S ; Kolbus A ; Schorpp-Kistner M ; Fusenig NE ; Angel P.** c-jun and junb antagonistically control cytokine-regulated mesenchymal-epidermal interaction in skin. *Ce,* 2000, vol. 103, 745-755
- **Tunaru S ; Kero J ; Schaub A ; Wufka C ; Blaukat A ; Pfeffer K ; Offermanns S.** Puma-g and hm74 are receptors for nicotinic acid and mediate its anti-lipolytic effect. *Nat Med,* 2003, vol. 9, 352-355
- **Vohradsky J.** Neural model of the genetic network. *J Biol Chem,* 2001, vol. 276, 36168-36173
- **Werner S ; Smola H ; Liao X ; Longaker MT ; Krieg T ; Hofschneider PH ; Williams LT.** The function of kgf in morphogenesis of epithelium and reepithelialization of wounds. *Science,* 1994, vol. 266, 819-822
- **Wick M ; Bürger C ; Funk M ; Müller R.** Identification of a novel mitogen-inducible gene (mig-6): regulation during g1 progression and differentiation. *Exp Cell Res,* 1995, vol. 219, 527-535
- **Wilson CL ; Miller CJ.** Simpleaffy: a BioConductor package for Affymetrix Quality Control and data analysis. *Bioinformatics,* 2005, vol. 21, 3683-3685
- **Xu, KP ; Yu FSX.** Cross talk between Met and epidermal growth factor receptor during retinal pigment epithelial wound healing. *Invest Ophthalmol Vis Sci,* 2007, vol. 48, 2242-2248
- **Yeang CH ; Mak HC ; McCuine S ; Workman C ; Jaakkola T ; Ideker T.** Validation and refinement of gene-regulatory pathways on a network of physical interactions. *Genome Biol.,* 2005, vol. 6, R62
- **Zenz R ; Scheuch H ; Martin P ; Frank C ; Eferl R ; Kenner L ; Sibilia M ; Wagner EF.** c-jun regulates eyelid closure and skin tumor development through egfr signaling. *Dev Cell,* 2003, vol. 4, 879-889
- **Zhou HY ; Pon YL ; Wong AST.** Synergistic effects of epidermal growth factor and hepatocyte growth factor on human ovarian cancer cell invasion and migration: role of extracellular signal-regulated kinase 1/2 and p38 mitogen-activated protein kinase. *Endocrinology,* 2007, vol. 148, 5195-5208